# EUROPEAN PATENT APPLICATION

(11) **EP 4 818 317 A1**
(43) Date of publication of application: **07.10.2026**
(21) Application number: 24895847.2
(22) Date of filing: 03.09.2024
(51) Int. Cl.: C12N 9/90, C12N 15/61

(54) **POLYPEPTIDE FOR BIOSYNTHESIS OF 1,2-PROPANEDIOL AND USE THEREOF**

(30) Priority: 29.11.2023 CN 202311617289
(71) Applicant: Tianjin University, Tianjin 300072 (CN)
(72) Inventor: ZHANG, Yan, Tianjin 300072 (CN); MA, Kailiang, Tianjin 300072 (CN)
(74) Representative: Huang, Liwei
(86) International application number: PCT/CN2024/116467
(87) International publication number: WO 2025/112748

(57) **Abstract**

Provided are a 1,5-anhydroalditol-6-phosphate isomerase and a use thereof, in particular the use thereof in the synthesis of 1,2-propanediol.

## Description

### Cross-Reference to Related Application

This application claims priority to Chinese Patent Application No. 202311617289.7 filed on November 29, 2023, and the contents of which are incorporated herein in its entirety by reference.

### Technical Field

The present disclosure relates generally to the fields of genetic engineering, enzyme engineering, and bioinformatics. Specifically, the present disclosure provides a novel glycolytic pathway, a 1,5-anhydroalditol-6-phosphate isomerase involved in the pathway, and uses thereof, particularly the use in a synthesis of 1,2-propanediol.

### Background Art

1,2-Propanediol is mainly used in the production of coatings and unsaturated polyester resins, and also in the production of plasticizers and hydraulic brake fluids. In addition, 1,2-propanediol can be used as a good solvent in the preparation of inks and epoxy resins; be used in a non-ionic detergent serving as an enzyme stabilizer and solvent; be used as an antifreeze agent; and be used as a humectant in the pharmaceutical, cosmetic, animal feed, and tobacco industries.

Common techniques for producing 1,2-propanediol include direct and indirect hydration of epoxypropane, direct catalytic oxidation of propylene, and a dimethyl carbonate (DMC)/propylene glycol co-production method. However, these methods often require complex chemical apparatus, consume large amounts of raw materials and energy, and produce by-products, resulting in high production costs and poor atom economy.

Given that 1,2-propanediol is an important industrial raw material with significant value and uses, research on its synthetic pathways holds considerable scientific and industrial disclosure value.

### Summary of the Invention

In a first aspect, the present disclosure provides a polypeptide including an amino acid sequence shown in SEQ ID NO: 1 or 97, or a functional variant thereof, where the functional variant possesses 1,5-anhydroalditol-6-phosphate isomerase activity.

In some embodiments of the first aspect, the polypeptide includes an amino acid sequence shown in SEQ ID NO: 1 or a functional variant thereof, where the functional variant possesses 1,5-anhydroglucitol-6-phosphate isomerase activity. In some embodiments, the polypeptide has an active site in its three-dimensional conformation defined such that, with reference to SEQ ID NO: 1, the active site includes amino acid residues H165, H282, S283, H334, C441, E443, R453, T455, L562, S662, I664, and G786, which are spatially close to one another.

In some embodiments of the first aspect, the functional variant is a natural isozyme of the amino acid sequence shown in SEQ ID NO: 1.

In some embodiments of the first aspect, the polypeptide includes an amino acid sequence shown in SEQ ID NO: 97 or a functional variant thereof, where the functional variant possesses 1,5-anhydromannitol-6-phosphate isomerase activity. In some embodiments, the polypeptide has an active site in its three-dimensional conformation defined such that, with reference to SEQ ID NO: 97, the active site includes amino acid residues Q162, H169, S277, S278, R323, F331, P335, C431, E433, D445, Y628, V630, and G752.

In some embodiments of the first aspect, the functional variant is a natural isozyme of the amino acid sequence shown in SEQ ID NO: 97.

In some embodiments of the first aspect, an substrate of the polypeptide is 1,5-anhydroalditol-6-phosphate.

In some embodiments of the first aspect, the functional variant is generated by one or more amino acid insertions, substitutions, and/or deletions in the amino acid sequence shown in SEQ ID NO: 1 or 97, or a natural isozyme thereof.

In a second aspect, the present disclosure provides a nucleic acid molecule encoding the polypeptide of the first aspect.

In a third aspect, the present disclosure provides an expression cassette including the nucleic acid molecule of the second aspect.

In a fourth aspect, the present disclosure provides an expression vector including the nucleic acid molecule of the second aspect or the expression cassette of the third aspect.

In a fifth aspect, the present disclosure provides a host cell including the nucleic acid molecule of the second aspect, the expression cassette of the third aspect, or the expression vector of the fourth aspect.

In some embodiments of the fifth aspect, the host cell is capable of expressing and producing a polypeptide including an amino acid sequence shown in SEQ ID NO: 1 or 97, or a functional variant thereof, where the functional variant possesses 1,5-anhydroalditol-6-phosphate isomerase activity.

In some embodiments of the fifth aspect, the host cell further expresses an activating enzyme, an aldolase, a hydroxyacetone reductase, and/or a transport complex.

In some embodiments of the fifth aspect, the transport complex possesses phosphoenolpyruvate-phosphotransferase system transport activity.

In some embodiments of the fifth aspect, the host cell is a eukaryotic cell or a prokaryotic cell.

In some embodiments of the fifth aspect, the eukaryotic cell is a yeast cell.

In some embodiments of the fifth aspect, the prokaryotic cell is selected from *Escherichia spp.*, *Klebsiella spp.*, *Streptococcus spp.*, *Lactobacillus spp.*, *Bifidobacterium spp.*, *Bacteroidetes*, or *Firmicutes*, etc.

In a sixth aspect, the present disclosure provides a composition including the polypeptide of the first aspect.

In some embodiments of the sixth aspect, the composition is used for catalyzing a conversion of 1,5-anhydroalditol-6-phosphate to 1,2-propanediol.

In a seventh aspect, the present disclosure provides use of the polypeptide of the first aspect, the nucleic acid molecule of the second aspect, the expression cassette of the third aspect, the expression vector of the fourth aspect, or the host cell of the fifth aspect in the preparation of a composition for catalyzing a conversion of 1,5-anhydroalditol-6-phosphate to 1,2-propanediol.

In an eighth aspect, the present disclosure provides a method for producing 1,2-propanediol, including culturing the host cell of the fifth aspect in a medium containing 1,5-anhydroglycitol to obtain 1,2-propanediol.

In a ninth aspect, the present disclosure provides a method for producing 1,2-propanediol, including contacting the composition of the sixth aspect with 1,5-anhydroalditol-6-phosphate to obtain 1,2-propanediol.

### Brief Description of the Drawings

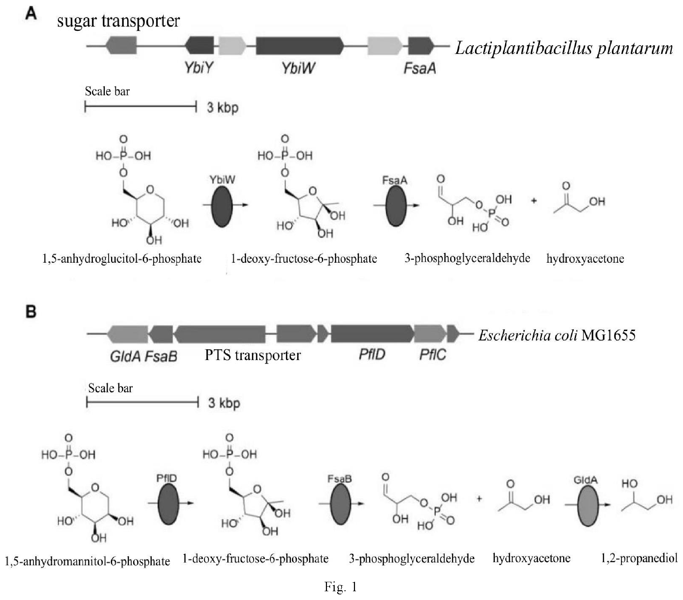

Fig. 1 is a schematic diagram of radical-dependent anhydroglycolysis. A. Gene cluster in *Lactiplantibacillus plantarum* (including the gene encoding the 1,5-anhydroglucitol-6-phosphate isomerase shown in SEQ ID NO: 2) and a YbiW-dependent anhydroglycolysis pathway. B. Gene cluster in *Escherichia coli* (including the gene encoding the 1,5-anhydromannitol-6-phosphate isomerase shown in SEQ ID NO: 98) anda PflD-dependent anhydroglycolysis pathway.

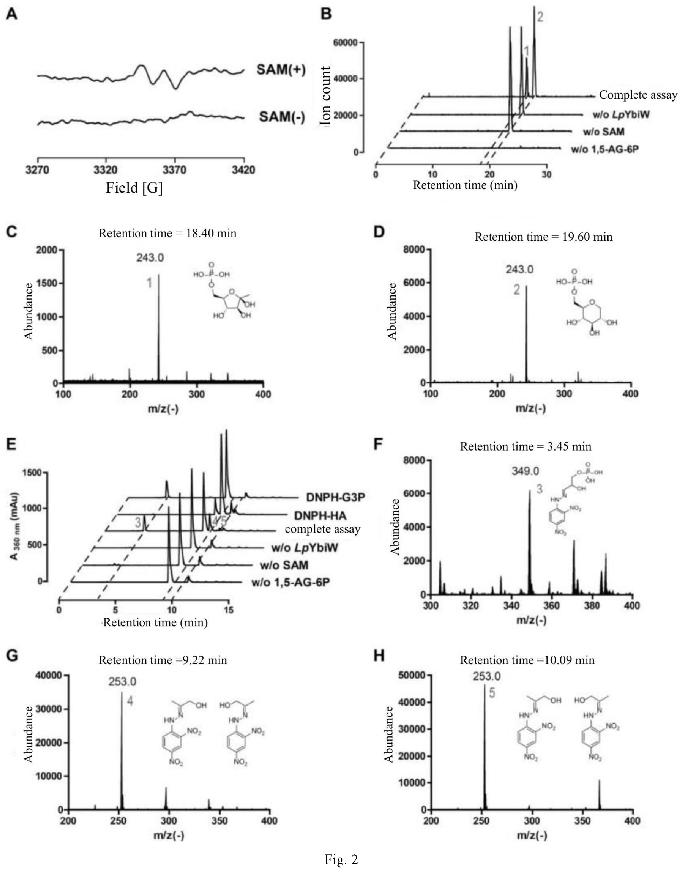

Fig. 2 shows electron paramagnetic resonance (EPR) spectra and LC-MS enzyme reaction analysis of YbiW. A. EPR spectra of LpYbiW and LpYbiY with titanium(III) citrate in the presence or absence of S-adenosylmethionine (SAM). B. LC-MS detection of 1-deoxyfructose-6-phosphate (1-deoxy-F6P) formation in the isomerization of 1,5-anhydroglucitol-6-phosphate (1,5-AG-6P) catalyzed by LpYbiW. Extracted ion chromatogram (EIC) monitoring for m/z=243 (in a negative ion mode) of 1-deoxy-F6P formation (t_{R}=18.40 min). C-D. Mass spectra (in a negative ion mode) of compound 1 (1-deoxy-F6P) and compound 2 (1,5-AG-6P) corresponding to the EIC peaks in B. E. HPLC elution profiles of DNPH derivatives of reaction products and standards in the LpFsaA-coupled LpYbiW reaction analysis. F-H. ESI(-) m/z mass spectra of DNPH-3-phosphoglyceraldehyde (peak 3) and DNPH-hydroxyacetone (peaks 4 and 5) in E. "w/o" indicates "not containing".

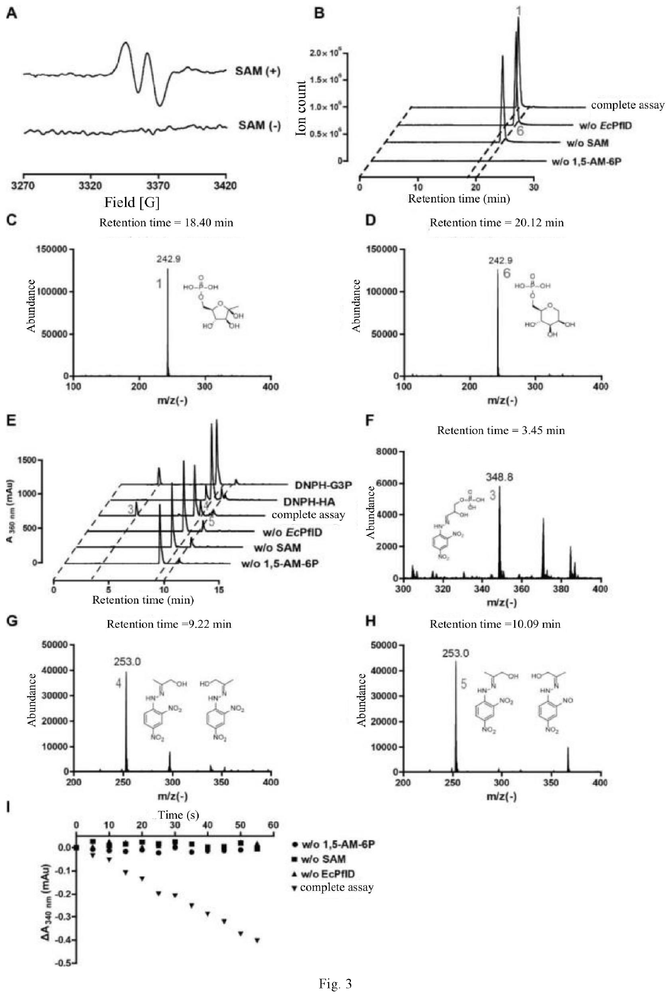

Fig. 3 shows EPR spectra and LC-MS enzyme reaction analysis of PflD. A. EPR spectra of EcPflD and EcPflC with titanium(III) citrate in the presence or absence of SAM. B. LC-MS detection of 1-deoxy-F6P formation in the isomerization of 1,5-anhydromannitol-6-phosphate (1,5-AM-6P) catalyzed by EcPflD. EIC monitoring for m/z=243 (in a negative ion mode) of 1-deoxy-F6P formation (t_{R}=18.40 min). C-D. Mass spectra (in a negative ion mode) of compound 1 (1-deoxy-F6P) and compound 6 (1,5-AM-6P) corresponding to the EIC peaks in B. E. HPLC elution profiles of DNPH derivatives of reaction products and standards in the EcFsaB-coupled EcPflD reaction analysis. F-H. Mass spectra of DNPH-3-phosphoglyceraldehyde (peak 3) and DNPH-hydroxyacetone (peaks 4 and 5) in E. I. Spectrophotometric determination of EcGldA-EcFsaB coupled with EcPflD, monitoring NADH consumption as hydroxyacetone is reduced. "w/o" indicates "not containing".

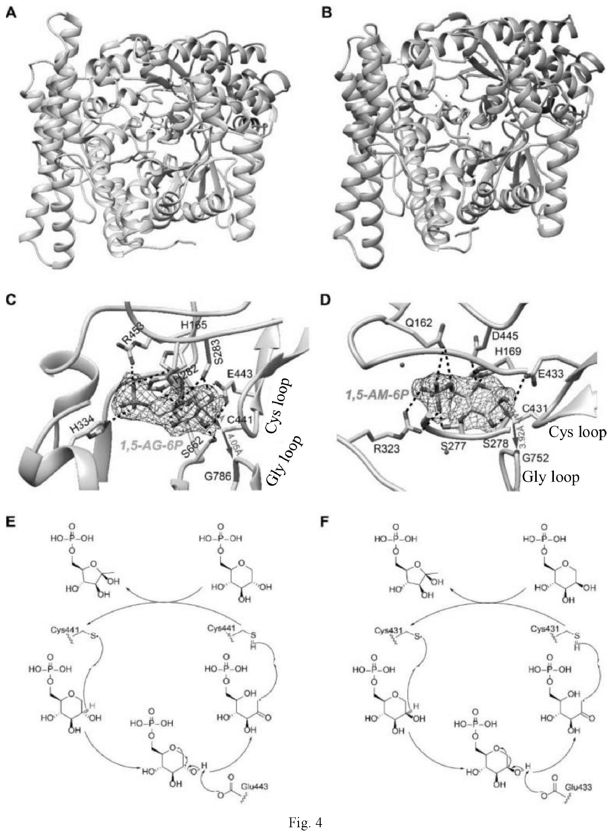

Fig. 4 shows X-ray diffraction crystal structures of EcYbiW and SdPflD. A-B. Structures of monomers of EcYbiW and SdPflD in each asymmetric unit. C-D. Active site structures of complexes of EcYbiW with 1,5-AG-6P and SdPflD with 1,5-AM-6P, respectively. Hydrogen atom transfer pathways are indicated by arrows, hydrogen bonds are represented by black dashed lines, and distances between key atoms are shown. The 2Fo-Fc electron density of the substrate is at 1.0σ. E-F. Catalytic mechanisms of EcYbiW and SdPflD.

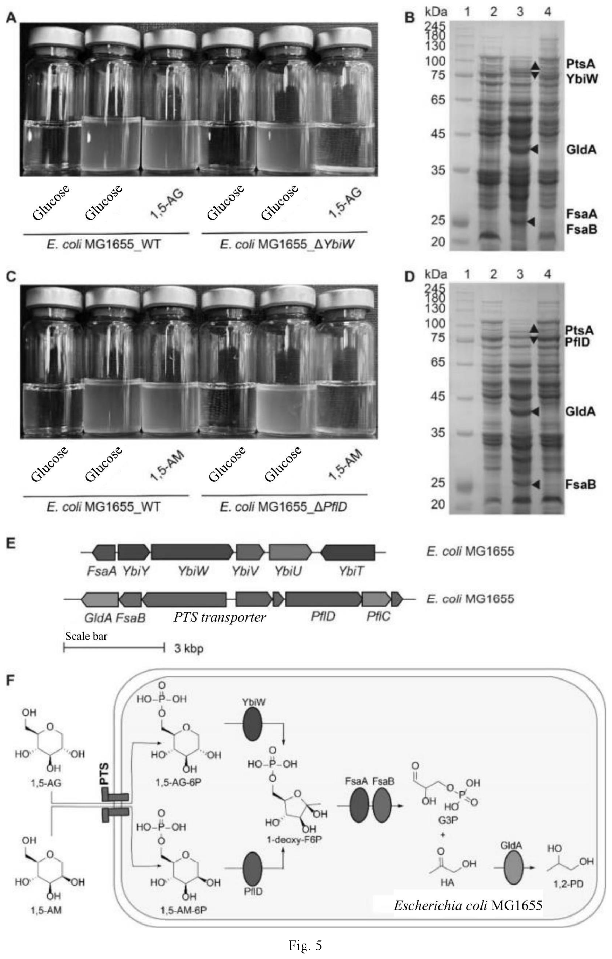

Fig. 5 shows YbiW-dependent glycolytic pathway induced by 1,5-anhydroglucitol (1,5-AG) and PflD-dependent glycolytic pathway induced by 1,5-anhydromannitol (1,5-AM) in *E. coli*. A. Growth of *E. coli* MG1655 wild-type and ΔybiW strains using 1,5-AG as the sole carbon source. Positive control: glucose is used as the sole carbon source. Negative control (w/o glucose): no carbon source is added. B. SDS-PAGE analysis of *E. coli* MG1655 wild-type using glucose (lane 2) or 1,5-AG (lane 3) as a sole carbon source, and of ΔybiW using glucose (lane 4) as a sole carbon source. Arrows indicate two ~ 95 kDa bands containing PtsA and YbiW, a ~ 42 kDa band identified as GldA, and a ~ 27 kDa band identified containing FsaB and FsaA. C. Growth of *E. coli* MG1655 wild-type and ΔpflD strains using 1,5-AM as a sole carbon source. Positive control: glucose is used as a sole carbon source. Negative control: no carbon source is added. D. SDS-PAGE analysis of *E. coli* MG1655 wild-type using glucose (lane 2) or 1,5-AM (lane 3) as a sole carbon source, and ΔpflD using glucose (lane 4) as a sole carbon source. Arrows indicate a ~ 95 kDa band identified as PtsA, a ~ 90 kDa band identified as PflD, a ~ 42 kDa band identified as GldA, and a ~ 27 kDa band identified as FsaB. E. Adjacent genome of YbiW (SEQ ID NO: 1) and PflD (SEQ ID NO: 98) in *E. coli* MG1655. F. 1,5-AG anhydroglycolysis pathway and 1,5-AM anhydroglycolysis pathway in *E. coli*. "w/o" indicates"not containing".

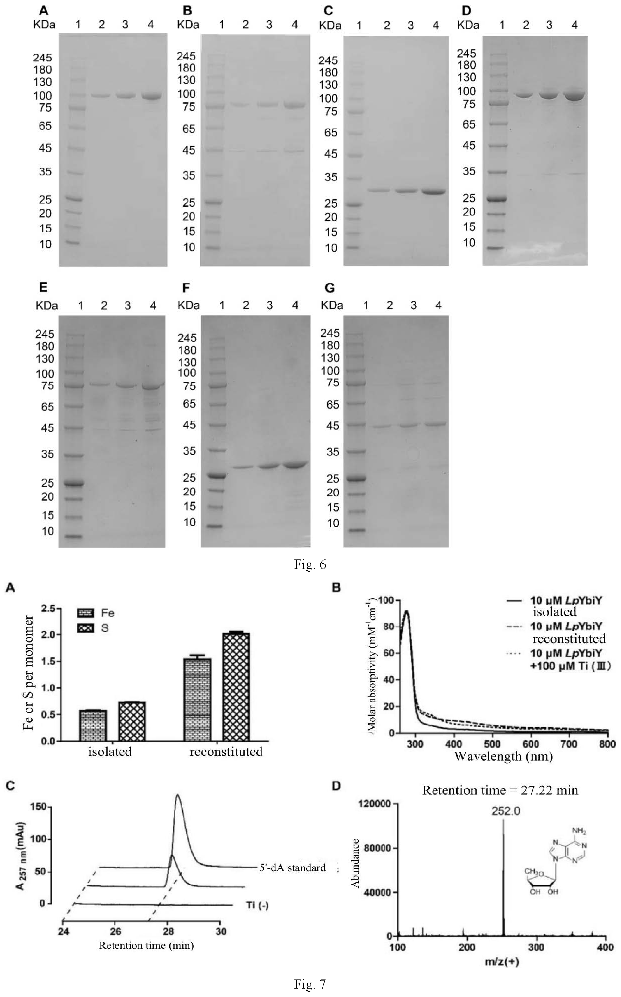

Fig. 6 shows SDS-PAGE analysis of purified proteins used for enzyme assays and biochemical characterization. A. LpYbiW; B. MBP-LpYbiY; C. LpFsaA; D. EcPflD; E. MBP-EcPflC; F. EcFsaB; G. EcGldA. Lanes 1 to 4 in each 4-20% gradient gel (Bis-Tris) contain protein molecular weight marker and 1 µg, 2 µg, and 4 µg of recombinant protein, respectively.
Fig. 7 shows characterization of recombinant LpYbiY. A. [Fe-S] cluster quantification in LpYbiY, assays performed in triplicate and shown with standard deviation. B. UV-Vis absorption spectra of LpYbiY is isolated and reconstituted, corresponding that the characteristic absorbance at 410 nm for the [4Fe-4S]²⁺ cluster in reconstituted LpYbiY disappears upon reduction with the strong reducing agent titanium(III) citrate. C. LC-MS elution profiles of SAM cleavage reaction mixtures catalyzed by LpYbiY in the presence and absence of Ti(III); commercially available 5'-deoxyadenosine (5'-dA) used as a standard. D. Positive ionization mass spectrum of the 5'-dA peak eluted at 27.22 minutes.

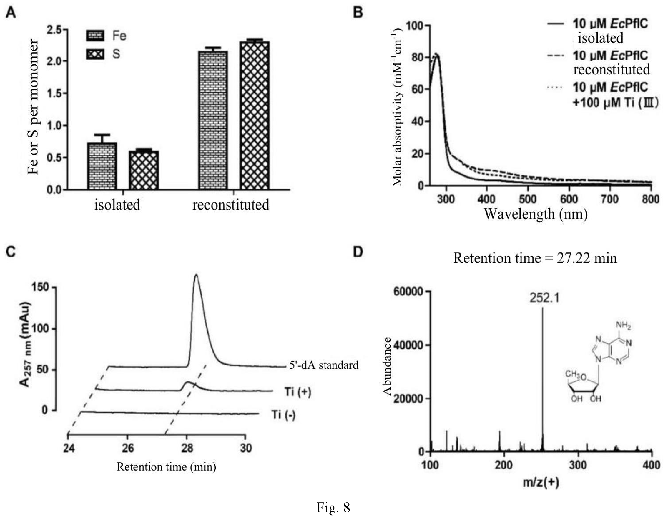

Fig. 8 shows characterization of recombinant EcPflC. A. [Fe-S] cluster quantification in EcPflC, assays performed in triplicate and shown with standard deviation. B. UV-Vis absorption spectra of isolated and reconstituted EcPflC, the characteristic absorbance at 410 nm corresponding to the [4Fe-4S]²⁺ cluster in reconstituted EcPflC disappears upon reduction with the strong reducing agent titanium(III) citrate. C. LC-MS elution profiles of SAM cleavage reaction mixtures catalyzed by EcPflC in the presence and absence of Ti(III); commercially available 5'-dA used as a standard. D. Positive ionization mass spectrum of the 5'-dA peak eluted at 27.22 minutes.

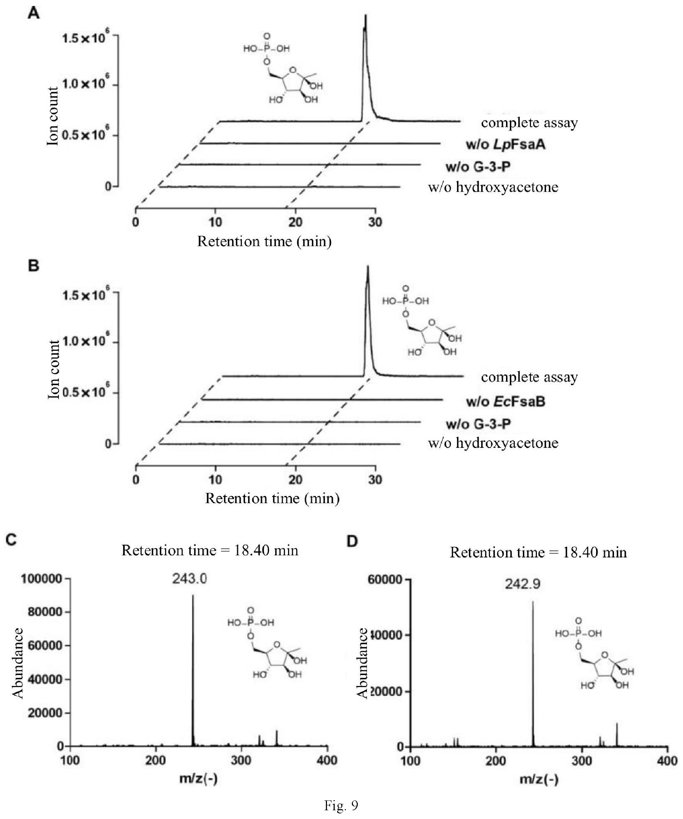

Fig. 9 shows LC-MS enzyme activity assays of LpFsaA and EcFsaB catalyzing the aldol addition of hydroxyacetone and 3-phosphoglyceraldehyde. A-B. Extracted ion chromatograms (m/z=243, in a negative ion mode) of LpFsaA- and EcFsaB-catalyzed reaction mixtures, monitoring the formation of 1-deoxy-F6P. C-D. Negative ionization mass spectra of 1-deoxy-F6P formed by LpFsaA and EcFsaB. "w/o" indicates "not containing".

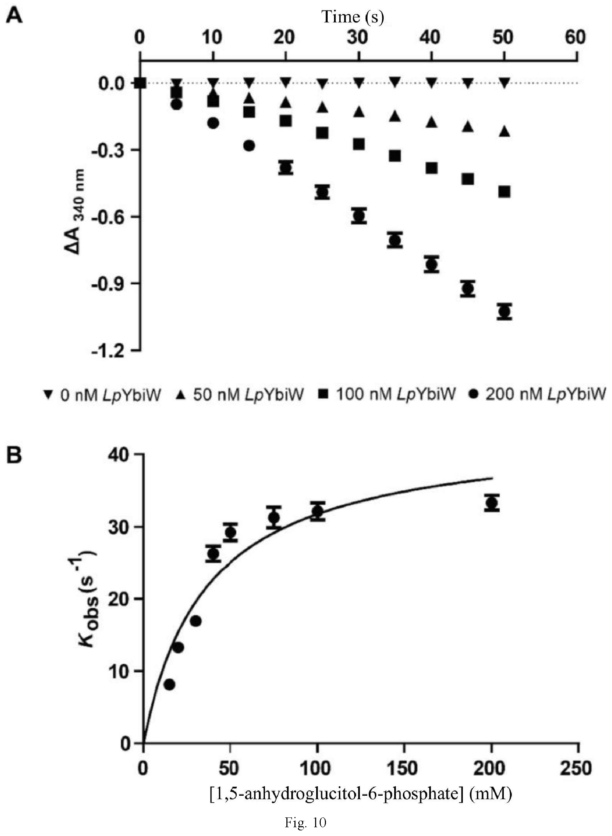

Fig. 10 shows enzyme kinetics data for LpYbiW. A. Dose-dependent LpFsaA-EcGldA coupled enzyme activity assay for LpYbiW; the amount of LpYbiW used in the assay is indicated. B. Michaelis-Menten kinetics of LpYbiW. Error bars represent the standard deviation of three independent experiments.

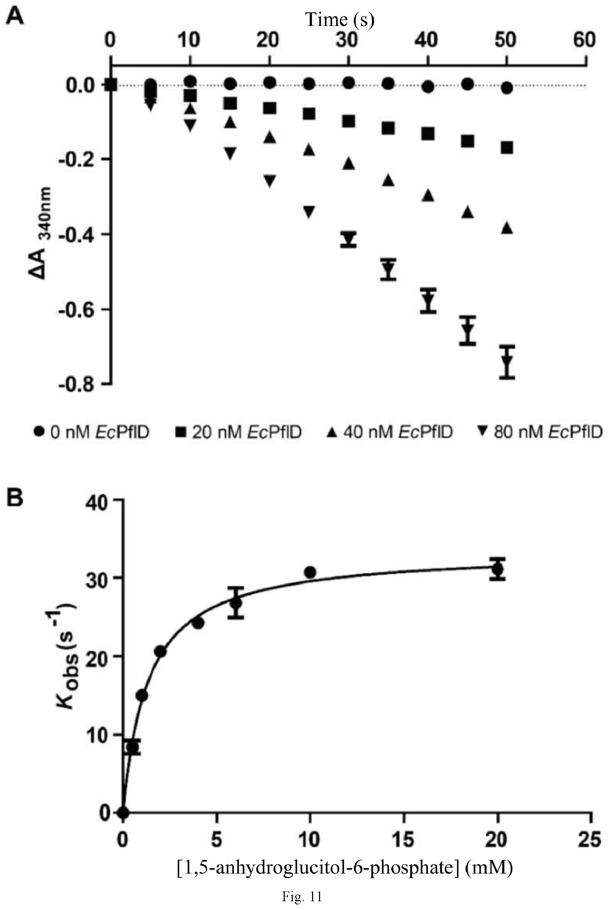

Fig. 11 shows enzyme kinetics data for EcPflD. A. Dose-dependent EcFsaB-EcGldA coupled enzyme activity assay for EcPflD; the amount of EcPflD used in the assay is indicated. B. Michaelis-Menten kinetics of EcPflD. Error bars represent the standard deviation of three independent experiments.

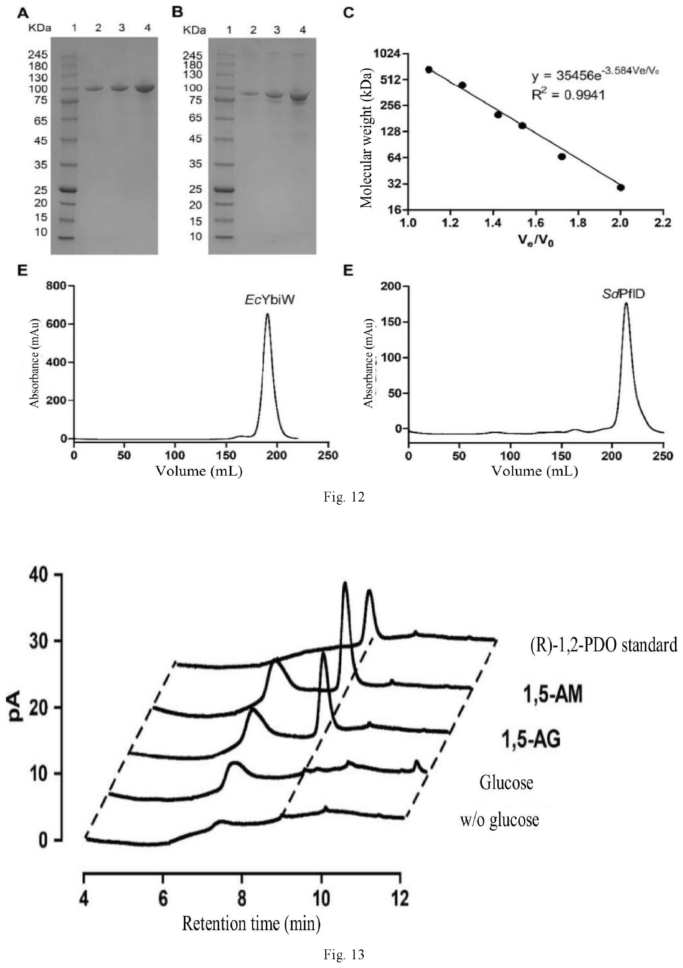

Fig. 12 shows SDS-PAGE and SEC analysis of purified EcYbiW (E114A, E115A, and K117A) and SdPflD used for protein crystallization. A. 4-20% gradient gel (Bis-Tris): lane 1, protein molecular weight marker; lanes 2-4, 1 µg, 2 µg, and 4 µg of purified EcYbiW (E114A, E115A, and K117A). B. 4-20% gradient gel (Bis-Tris): lane 1, protein molecular weight marker; lanes 2-4, 1 µg, 2 µg, and 4 µg of purified SdPflD. C. SEC standard curve established using standard proteins: bovine thyroglobulin (669 kDa), horse spleen ferritin (443 kDa), sweet potato β-amylase (200 kDa), yeast alcohol dehydrogenase (150 kDa), BSA (66 kDa), and bovine carbonic anhydrase (29 kDa) (Sigma MWGF 1000-1KT). D-E. Elution profiles of EcYbiW (E114A, E115A, and K117A) and SdPflD using Superdex 200 gel filtration chromatography to determine molecular weights of EcYbiW and SdPflD; estimated molecular weights are 154.9 kDa and 77.9 kDa, respectively.
Fig. 13 shows gas chromatography (GC) analysis of fermentation broth of wild-type *E. coli* MG1655 grown on glucose, 1,5-AG, or 1,5-AM. "w/o glucose" indicates not containing glucose (negative control); glucose is the positive control.

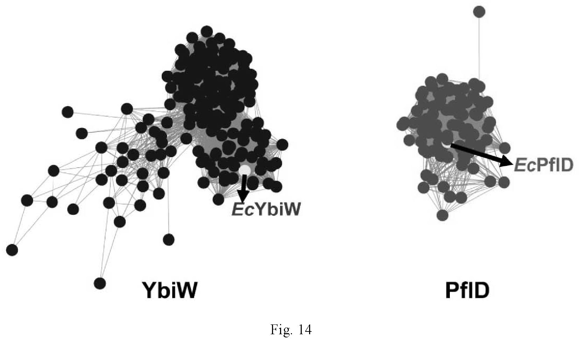

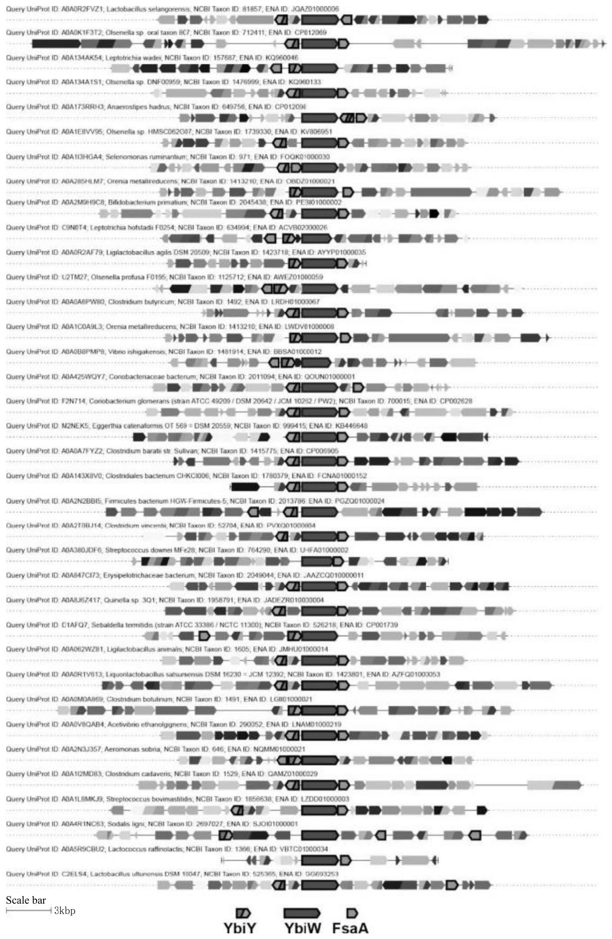

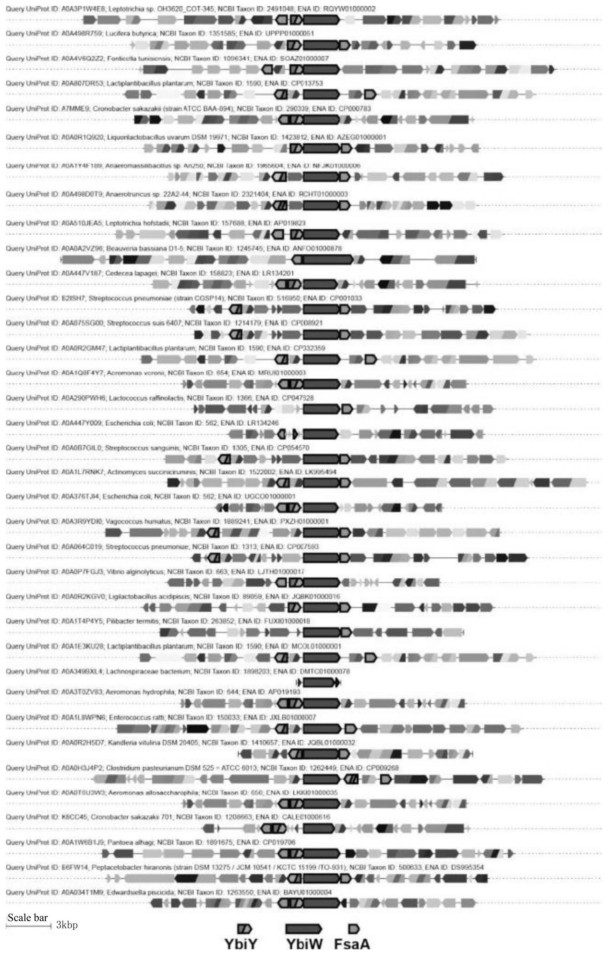

Fig. 14 shows the YbiW cluster and PflD cluster constructed using the Sequence Similarity Network (SSN) tool. Each node represents sequences with 80% or more similarity. The nodes containing EcYbiW and EcPflD are indicated with arrows in the two clusters.

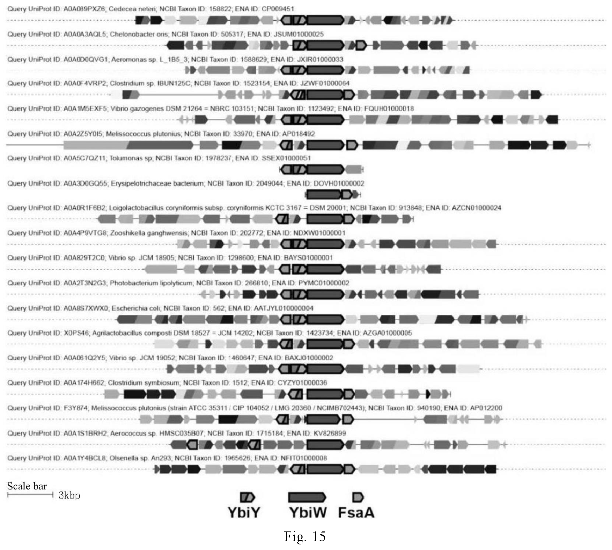

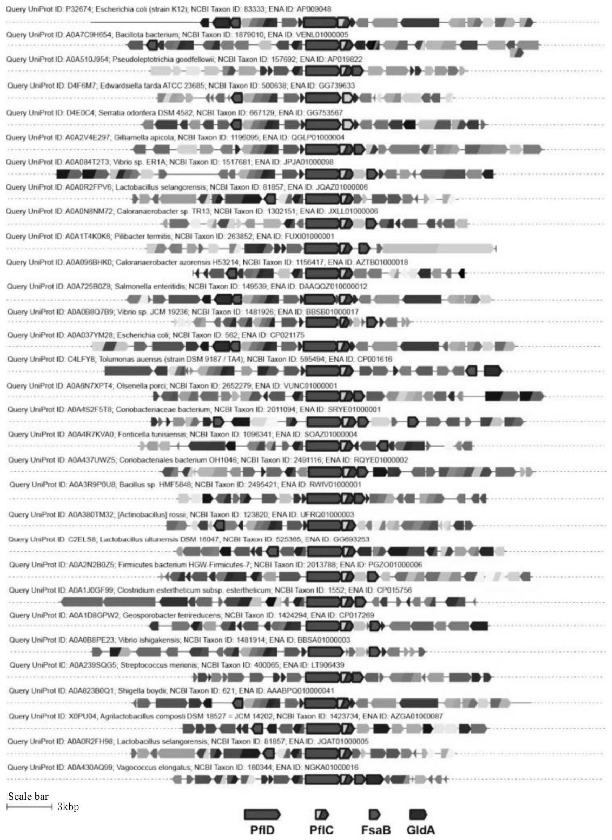

Fig. 15 shows exemplary gene cluster distributions including the natural isozyme of 1,5-anhydroglucitol-6-phosphate isomerase shown in SEQ ID NO: 1, an S-adenosylmethionine radical enzyme family glycine radical enzyme activating enzyme, and a 1-deoxyfructose-6-phosphate aldolase (gene directions indicate the coding strand direction, 5' to 3').

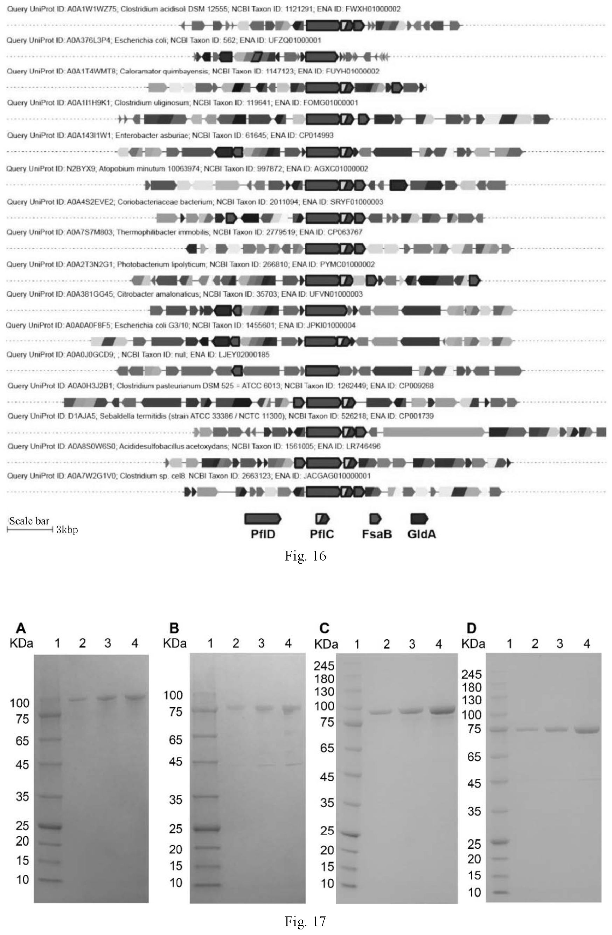

Fig. 16 shows exemplary gene cluster distributions including the natural isozyme of 1,5-anhydromannitol-6-phosphate isomerase shown in SEQ ID NO: 97, an S-adenosylmethionine radical enzyme family glycine radical enzyme activating enzyme, a 1-deoxyfructose-6-phosphate aldolase, and a hydroxyacetone reductase.
Fig. 17 shows SDS-PAGE analysis of purified proteins used for biochemical characterization. A. EcYbiW; B. MBP-EcYbiY; C. SdPflD; D. MBP-SdPflC. Lanes 1 to 4 in each 4-20% gradient gel (Bis-Tris) contain a protein molecular weight marker and 1 µg, 2 µg, and 4 µg of recombinant protein, respectively.

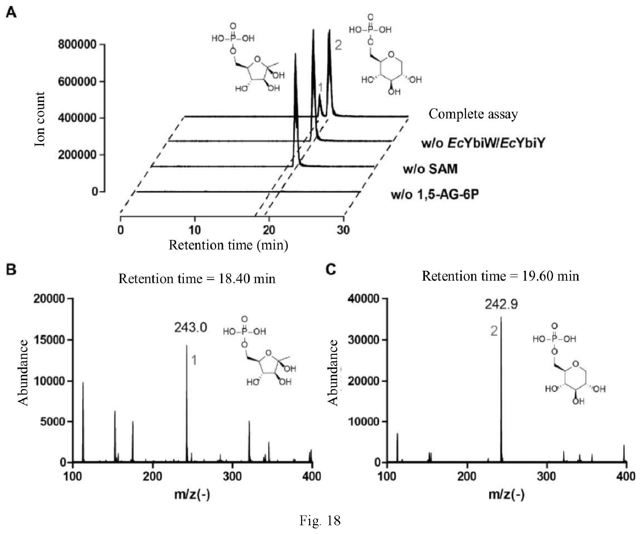

Fig. 18 shows LC-MS enzyme reaction analysis of *E. coli* YbiW. A. LC-MS detection of 1-deoxy-F6P formation in the isomerization of 1,5-AG-6P catalyzed by EcYbiW. EIC monitoring for m/z=243 (in a negative ion mode) of 1-deoxy-F6P formation (t_{R}=18.40 min). B-C. Mass spectra (in a negative ion mode) of compound 1 (1-deoxy-F6P) and compound 2 (1,5-AG-6P) corresponding to the EIC peaks in A. "w/o" indicates "not containing".

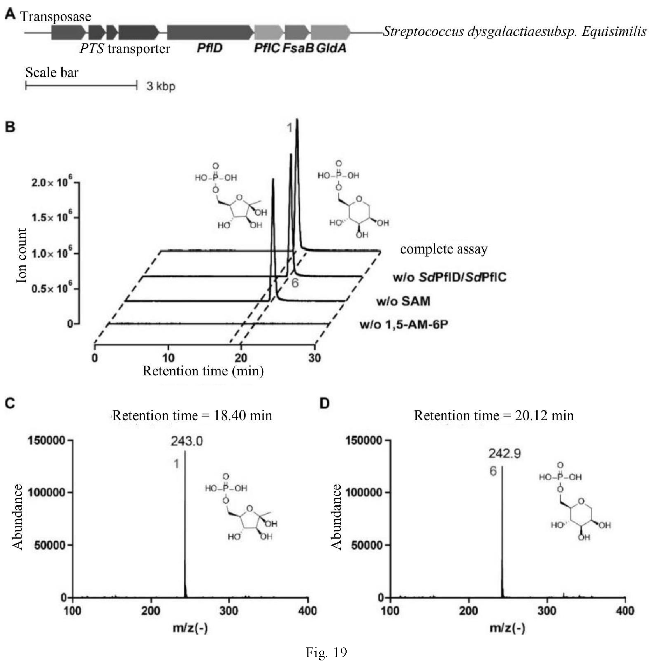

Fig. 19 shows the gene cluster of PflD from *Streptococcus dysgalactiae subsp. Equisimilis* and LC-MS enzyme reaction analysis. A. Gene cluster in *Streptococcus dysgalactiae subsp. equisimilis* (including the gene encoding the 1,5-anhydromannitol-6-phosphate isomerase shown in SEQ ID NO: 97). B. LC-MS detection of 1-deoxy-F6P formation in the isomerization of 1,5-AM-6P catalyzed by SdPflD. EIC monitoring for m/z=243 (in a negative ion mode) of 1-deoxy-F6P formation (t_{R}=18.40 min). C-D. Mass spectra (in a negative ion mode) of compound 1 (1-deoxy-F6P) and compound 6 (1,5-AM-6P) corresponding to the EIC peaks in B. "w/o" indicates "not containing".

### Sequence Descriptions

SEQ ID NO: 1 shows the amino acid sequence of 1,5-anhydroglucitol-6-phosphate isomerase with the Uniprot accession No. P75793, expressed by *Escherichia coli*.
SEQ ID NO: 2 shows the amino acid sequence of 1,5-anhydroglucitol-6-phosphate isomerase with the Uniprot accession No. A0A807DR53, expressed by *Lactiplantibacillus plantarum*.
SEQ ID NO: 3 shows the amino acid sequence of 1,5-anhydroglucitol-6-phosphate isomerase with the Uniprot accession No. A0A0R2FVZ1, expressed by *Lactobacillus selangorensis*.
SEQ ID NO: 4 shows the amino acid sequence of 1,5-anhydroglucitol-6-phosphate isomerase with the Uniprot accession No. A0A0A6S4M4, expressed by *Streptococcus uberis*.
SEQ ID NO: 5 shows the amino acid sequence of 1,5-anhydroglucitol-6-phosphate isomerase with the Uniprot accession No. A0A0K1F3T2, expressed by *Olsenella sp. oral taxon 807*.
SEQ ID NO: 6 shows the amino acid sequence of 1,5-anhydroglucitol-6-phosphate isomerase with the Uniprot accession No. A0A134AK54, expressed by *Leptotrichia wadei*.
SEQ ID NO: 7 shows the amino acid sequence of 1,5-anhydroglucitol-6-phosphate isomerase with the Uniprot accession No. A0A134A1S1, expressed by *Senella sp.* DNF00959.
SEQ ID NO: 8 shows the amino acid sequence of 1,5-anhydroglucitol-6-phosphate isomerase with the Uniprot accession No. A0A0E2UQR7, expressed by *Streptococcus parauberis*.
SEQ ID NO: 9 shows the amino acid sequence of 1,5-anhydroglucitol-6-phosphate isomerase with the Uniprot accession No. A0A173RRH3, expressed by *Anaerostipes hadrus*.
SEQ ID NO: 10 shows the amino acid sequence of 1,5-anhydroglucitol-6-phosphate isomerase with the Uniprot accession No. A0A1E8VV95, expressed by *Senella sp.* HMSC062G07.
SEQ ID NO: 11 shows the amino acid sequence of 1,5-anhydroglucitol-6-phosphate isomerase with the Uniprot accession No. A0A1I3HGA4, expressed by *Selenomonas ruminantium*.
SEQ ID NO: 12 shows the amino acid sequence of 1,5-anhydroglucitol-6-phosphate isomerase with the Uniprot accession No. A0A285HLM7, expressed by *Orenia metallireducens*.
SEQ ID NO: 13 shows the amino acid sequence of 1,5-anhydroglucitol-6-phosphate isomerase with the Uniprot accession No. A0A2M9H9C8, expressed by *Bifidobacterium primatium*.
SEQ ID NO: 14 shows the amino acid sequence of 1,5-anhydroglucitol-6-phosphate isomerase with the Uniprot accession No. C9N0T4, expressed by *Leptotrichia hofstadii* F0254.
SEQ ID NO: 15 shows the amino acid sequence of 1,5-anhydroglucitol-6-phosphate isomerase with the Uniprot accession No. A0A0R2AF79, expressed by *Ligilactobacillus agilis* DSM 20509.
SEQ ID NO: 16 shows the amino acid sequence of 1,5-anhydroglucitol-6-phosphate isomerase with the Uniprot accession No. U2TM27, expressed by *Senella profusa* F0195.
SEQ ID NO: 17 shows the amino acid sequence of 1,5-anhydroglucitol-6-phosphate isomerase with the Uniprot accession No. A0A0A6PW80, expressed by *Clostridium butyricum*.
SEQ ID NO: 18 shows the amino acid sequence of 1,5-anhydroglucitol-6-phosphate isomerase with the Uniprot accession No. A0A1C0A9L3, expressed by *Orenia metallireducens*.
SEQ ID NO: 19 shows the amino acid sequence of 1,5-anhydroglucitol-6-phosphate isomerase with the Uniprot accession No. A0A0B8PMP8, expressed by *Vibrio ishigakensis*.
SEQ ID NO: 20 shows the amino acid sequence of 1,5-anhydroglucitol-6-phosphate isomerase with the Uniprot accession No. A0A425WQY7, expressed by *Coriobacteriaceae*.
SEQ ID NO: 21 shows the amino acid sequence of 1,5-anhydroglucitol-6-phosphate isomerase with the Uniprot accession No. F2N714, expressed by *Coriobacterium glomerans* strain ATCC 49209/DSM 20642/JCM 10262/PW2.
SEQ ID NO: 22 shows the amino acid sequence of 1,5-anhydroglucitol-6-phosphate isomerase with the Uniprot accession No. M2NEK5, expressed by *Eggerthia cateniformis* OT 569.
SEQ ID NO: 23 shows the amino acid sequence of 1,5-anhydroglucitol-6-phosphate isomerase with the Uniprot accession No. A0A0A7FYZ2, expressed by *Clostridium baratii str. Sullivan*.
SEQ ID NO: 24 shows the amino acid sequence of 1,5-anhydroglucitol-6-phosphate isomerase with the Uniprot accession No. A0A143X8V0, expressed by *Clostridiales* bacterium CHKC1006.
SEQ ID NO: 25 shows the amino acid sequence of 1,5-anhydroglucitol-6-phosphate isomerase with the Uniprot accession No. A0A2N2BBI5, expressed by *Firmicutes* bacterium HGW-Firmicutes-5.
SEQ ID NO: 26 shows the amino acid sequence of 1,5-anhydroglucitol-6-phosphate isomerase with the Uniprot accession No. A0A2T0BJ14, expressed by *Clostridium vincentii*.
SEQ ID NO: 27 shows the amino acid sequence of 1,5-anhydroglucitol-6-phosphate isomerase with the Uniprot accession No. A0A380JDF6, expressed by *Streptococcus downei* MFe28.
SEQ ID NO: 28 shows the amino acid sequence of 1,5-anhydroglucitol-6-phosphate isomerase with the Uniprot accession No. A0A847CI73, expressed by *Erysipelotrichaceae* bacterium.
SEQ ID NO: 29 shows the amino acid sequence of 1,5-anhydroglucitol-6-phosphate isomerase with the Uniprot accession No. A0A8J6Z417, expressed by *Quinella sp. 3Q1*.
SEQ ID NO: 30 shows the amino acid sequence of 1,5-anhydroglucitol-6-phosphate isomerase with the Uniprot accession No. D1AFQ7, expressed by *Sebaldella termitidis* (strain ATCC 33386/NCTC 11300).
SEQ ID NO: 31 shows the amino acid sequence of 1,5-anhydroglucitol-6-phosphate isomerase with the Uniprot accession No. A0A062WZ81, expressed by *Ligilactobacillus animalis*.
SEQ ID NO: 32 shows the amino acid sequence of 1,5-anhydroglucitol-6-phosphate isomerase with the Uniprot accession No. A0A0R1V613, expressed by *Liquorilactobacillus satsumensis* DSM 16230.
SEQ ID NO: 33 shows the amino acid sequence of 1,5-anhydroglucitol-6-phosphate isomerase with the Uniprot accession No. A0A0M0A869, expressed by *Clostridium botulinum*.
SEQ ID NO: 34 shows the amino acid sequence of 1,5-anhydroglucitol-6-phosphate isomerase with the Uniprot accession No. A0A0V8QAB4, expressed by *Acetivibrio ethanolgignens*.
SEQ ID NO: 35 shows the amino acid sequence of 1,5-anhydroglucitol-6-phosphate isomerase with the Uniprot accession No. A0A2N3J357, expressed by *Aeromonas sobria*.
SEQ ID NO: 36 shows the amino acid sequence of 1,5-anhydroglucitol-6-phosphate isomerase with the Uniprot accession No. A0A1I2MD83, expressed by *Clostridium cadaveris*.
SEQ ID NO: 37 shows the amino acid sequence of 1,5-anhydroglucitol-6-phosphate isomerase with the Uniprot accession No. A0A1L8MKJ9, expressed by *Streptococcus bovimastitidis*.
SEQ ID NO: 38 shows the amino acid sequence of 1,5-anhydroglucitol-6-phosphate isomerase with the Uniprot accession No. A0A4R1NC63, expressed by *Sodalis ligni*.
SEQ ID NO: 39 shows the amino acid sequence of 1,5-anhydroglucitol-6-phosphate isomerase with the Uniprot accession No. A0A5R9CBU2, expressed by *Lactococcus raffinolactis*.
SEQ ID NO: 40 shows the amino acid sequence of 1,5-anhydroglucitol-6-phosphate isomerase with the Uniprot accession No. C2ELS4, expressed by *Lactobacillus ultunensis* DSM 16047.
SEQ ID NO: 41 shows the amino acid sequence of 1,5-anhydroglucitol-6-phosphate isomerase with the Uniprot accession No. A0A3P1W4E8, expressed by *Leptotrichia sp.* OH3620.
SEQ ID NO: 42 shows the amino acid sequence of 1,5-anhydroglucitol-6-phosphate isomerase with the Uniprot accession No. A0A498R759, expressed by *Lucifera butyrica*.
SEQ ID NO: 43 shows the amino acid sequence of 1,5-anhydroglucitol-6-phosphate isomerase with the Uniprot accession No. A0A4V6Q2Z2, expressed by *Fonticella tunisiensis*.
SEQ ID NO: 44 shows the amino acid sequence of 1,5-anhydroglucitol-6-phosphate isomerase with the Uniprot accession No. A7MME9, expressed by *Cronobacter sakazakii* strain ATCC BAA-894.
SEQ ID NO: 45 shows the amino acid sequence of 1,5-anhydroglucitol-6-phosphate isomerase with the Uniprot accession No. A0A0R1Q920, expressed by *Liquorilactobacillus uvarum* DSM 19971.
SEQ ID NO: 46 shows the amino acid sequence of 1,5-anhydroglucitol-6-phosphate isomerase with the Uniprot accession No. A0A1Y4F189, expressed by *Anaeromassilibacillus sp.* An250.
SEQ ID NO: 47 shows the amino acid sequence of 1,5-anhydroglucitol-6-phosphate isomerase with the Uniprot accession No. A0A498D0T9, expressed by *Anaerotruncus sp.* 22A2-44.
SEQ ID NO: 48 shows the amino acid sequence of 1,5-anhydroglucitol-6-phosphate isomerase with the Uniprot accession No. A0A510JEA5, expressed by *Leptotrichia hofstadii*.
SEQ ID NO: 49 shows the amino acid sequence of 1,5-anhydroglucitol-6-phosphate isomerase with the Uniprot accession No. A0A0A2VZ96, expressed by *Beauveria bassiana* D1-5.
SEQ ID NO: 50 shows the amino acid sequence of 1,5-anhydroglucitol-6-phosphate isomerase with the Uniprot accession No. A0A447V187, expressed by *Cedecea lapagei*.
SEQ ID NO: 51 shows the amino acid sequence of 1,5-anhydroglucitol-6-phosphate isomerase with the Uniprot accession No. B2ISH7, expressed by *Streptococcus pneumoniae* strain CGSP14.
SEQ ID NO: 52 shows the amino acid sequence of 1,5-anhydroglucitol-6-phosphate isomerase with the Uniprot accession No. A0A075SG00, expressed by *Streptococcus suis* 6407.
SEQ ID NO: 53 shows the amino acid sequence of 1,5-anhydroglucitol-6-phosphate isomerase with the Uniprot accession No. A0A0R2GM47, expressed by *Lactiplantibacillus plantarum*.
SEQ ID NO: 54 shows the amino acid sequence of 1,5-anhydroglucitol-6-phosphate isomerase with the Uniprot accession No. A0A1Q8F4Y7, expressed by *Aeromonas veronii*.
SEQ ID NO: 55 shows the amino acid sequence of 1,5-anhydroglucitol-6-phosphate isomerase with the Uniprot accession No. A0A290PWH6, expressed by *Lactococcus raffinolactis*.
SEQ ID NO: 56 shows the amino acid sequence of 1,5-anhydroglucitol-6-phosphate isomerase with the Uniprot accession No. A0A447Y009, expressed by *Escherichia coli*.
SEQ ID NO: 57 shows the amino acid sequence of 1,5-anhydroglucitol-6-phosphate isomerase with the Uniprot accession No. A0A0B7GIL0, expressed by *Streptococcus sanguinis*.
SEQ ID NO: 58 shows the amino acid sequence of 1,5-anhydroglucitol-6-phosphate isomerase with the Uniprot accession No. A0A1L7RNK7, expressed by *Actinomyces succiniciruminis*.
SEQ ID NO: 59 shows the amino acid sequence of 1,5-anhydroglucitol-6-phosphate isomerase with the Uniprot accession No. A0A376TJI4, expressed by *Escherichia coli*.
SEQ ID NO: 60 shows the amino acid sequence of 1,5-anhydroglucitol-6-phosphate isomerase with the Uniprot accession No. A0A3R9YDI0, expressed by *Vagococcus humatus*.
SEQ ID NO: 61 shows the amino acid sequence of 1,5-anhydroglucitol-6-phosphate isomerase with the Uniprot accession No. A0A064C019, expressed by *Streptococcus pneumoniae*.
SEQ ID NO: 62 shows the amino acid sequence of 1,5-anhydroglucitol-6-phosphate isomerase with the Uniprot accession No. A0A0P7FGJ3, expressed by *Vibrio alginolyticus*.
SEQ ID NO: 63 shows the amino acid sequence of 1,5-anhydroglucitol-6-phosphate isomerase with the Uniprot accession No. A0A0R2KGV0, expressed by *Ligilactobacillus acidipiscis*.
SEQ ID NO: 64 shows the amino acid sequence of 1,5-anhydroglucitol-6-phosphate isomerase with the Uniprot accession No. A0A1T4P4Y5, expressed by *Pilibacter termitis*.
SEQ ID NO: 65 shows the amino acid sequence of 1,5-anhydroglucitol-6-phosphate isomerase with the Uniprot accession No. A0A1E3KU28, expressed by *Lactiplantibacillus plantarum*.
SEQ ID NO: 66 shows the amino acid sequence of 1,5-anhydroglucitol-6-phosphate isomerase with the Uniprot accession No. A0A349BXL4, expressed by *Lachnospiraceae bacterium*.
SEQ ID NO: 67 shows the amino acid sequence of 1,5-anhydroglucitol-6-phosphate isomerase with the Uniprot accession No. A0A3T0ZV83, expressed by *Aeromonas hydrophila*.
SEQ ID NO: 68 shows the amino acid sequence of 1,5-anhydroglucitol-6-phosphate isomerase with the Uniprot accession No. A0A1L8WPN6, expressed by *Enterococcus ratti*.
SEQ ID NO: 69 shows the amino acid sequence of 1,5-anhydroglucitol-6-phosphate isomerase with the Uniprot accession No. A0A0R2H5D7, expressed by *Kandleria vitulina*.
SEQ ID NO: 70 shows the amino acid sequence of 1,5-anhydroglucitol-6-phosphate isomerase with the Uniprot accession No. A0A0H3J4P2, expressed by *Clostridium pasteurianum* DSM 525.
SEQ ID NO: 71 shows the amino acid sequence of 1,5-anhydroglucitol-6-phosphate isomerase with the Uniprot accession No. A0A0T6U3W3, expressed by *Aeromonas allosaccharophila*.
SEQ ID NO: 72 shows the amino acid sequence of 1,5-anhydroglucitol-6-phosphate isomerase with the Uniprot accession No. K8CC45, expressed by *Cronobacter sakazakii*.
SEQ ID NO: 73 shows the amino acid sequence of 1,5-anhydroglucitol-6-phosphate isomerase with the Uniprot accession No. A0A1W6B1J9, expressed by *Pantoea alhagi*.
SEQ ID NO: 74 shows the amino acid sequence of 1,5-anhydroglucitol-6-phosphate isomerase with the Uniprot accession No. B6FW14, expressed by *Peptacetobacter hiranonis* strain DSM 13275/JCM 10541/KCTC 15199/TO-931.
SEQ ID NO: 75 shows the amino acid sequence of 1,5-anhydroglucitol-6-phosphate isomerase with the Uniprot accession No. A0A034T1M9, expressed by *Edwardsiella piscicida*.
SEQ ID NO: 76 shows the amino acid sequence of 1,5-anhydroglucitol-6-phosphate isomerase with the Uniprot accession No. A0A089PXZ6, expressed by *Cedecea neteri*.
SEQ ID NO: 77 shows the amino acid sequence of 1,5-anhydroglucitol-6-phosphate isomerase with the Uniprot accession No. A0A0A3AQL5, expressed by *Chelonobacter oris*.
SEQ ID NO: 78 shows the amino acid sequence of 1,5-anhydroglucitol-6-phosphate isomerase with the Uniprot accession No. A0A0D0QVG1, expressed by *Aeromonas sp.* L_1B5_3.
SEQ ID NO: 79 shows the amino acid sequence of 1,5-anhydroglucitol-6-phosphate isomerase with the Uniprot accession No. A0A0F4VRP2, expressed by *Clostridium sp.* IBUN125C.
SEQ ID NO: 80 shows the amino acid sequence of 1,5-anhydroglucitol-6-phosphate isomerase with the Uniprot accession No. A0A1M5EXF5, expressed by *Vibrio gazogenes* DSM 21264.
SEQ ID NO: 81 shows the amino acid sequence of 1,5-anhydroglucitol-6-phosphate isomerase with the Uniprot accession No. A0A2Z5Y015, expressed by *Melissococcus plutonius*.
SEQ ID NO: 82 shows the amino acid sequence of 1,5-anhydroglucitol-6-phosphate isomerase with the Uniprot accession No. A0A5C7QZ11, expressed by *Tolumonas sp..*
SEQ ID NO: 83 shows the amino acid sequence of 1,5-anhydroglucitol-6-phosphate isomerase with the Uniprot accession No. A0A3D0GQ55, expressed by *Erysipelotrichaceae bacterium*.
SEQ ID NO: 84 shows the amino acid sequence of 1,5-anhydroglucitol-6-phosphate isomerase with the Uniprot accession No. A0A0R1F6B2, expressed by *Loigolactobacillus coryniformis subsp. coryniformis* KCTC 3167.
SEQ ID NO: 85 shows the amino acid sequence of 1,5-anhydroglucitol-6-phosphate isomerase with the Uniprot accession No. A0A4P9VTG8, expressed by *Zooshikella ganghwensis*.
SEQ ID NO: 86 shows the amino acid sequence of 1,5-anhydroglucitol-6-phosphate isomerase with the Uniprot accession No. A0A829T2C0, expressed by *Vibrio sp.*.
SEQ ID NO: 87 shows the amino acid sequence of 1,5-anhydroglucitol-6-phosphate isomerase with the Uniprot accession No. A0A2T3N2G3, expressed by *Photobacterium lipolyticum*.
SEQ ID NO: 88 shows the amino acid sequence of 1,5-anhydroglucitol-6-phosphate isomerase with the Uniprot accession No. A0A8S7XWX0, expressed by *Escherichia coli*.
SEQ ID NO: 89 shows the amino acid sequence of 1,5-anhydroglucitol-6-phosphate isomerase with the Uniprot accession No. X0PS46, expressed by *Agrilactobacillus composti* DSM 18527.
SEQ ID NO: 90 shows the amino acid sequence of 1,5-anhydroglucitol-6-phosphate isomerase with the Uniprot accession No. A0A061Q2Y5, expressed by *Vibrio sp.* JCM 19052.
SEQ ID NO: 91 shows the amino acid sequence of 1,5-anhydroglucitol-6-phosphate isomerase with the Uniprot accession No. A0A174H662, expressed by *Clostridium symbiosum*.
SEQ ID NO: 92 shows the amino acid sequence of 1,5-anhydroglucitol-6-phosphate isomerase with the Uniprot accession No. F3Y874, expressed by *Melissococcus plutonius* strain ATCC 35311/CIP 104052/LMG 20360/NCIMB 702443.
SEQ ID NO: 93 shows the amino acid sequence of 1,5-anhydroglucitol-6-phosphate isomerase with the Uniprot accession No. A0A1S1BRH2, expressed by *Aerococcus sp.* HMSC035B07.
SEQ ID NO: 94 shows the amino acid sequence of 1,5-anhydroglucitol-6-phosphate isomerase with the Uniprot accession No. A0A1Y4BCL8, expressed by *Senella sp.* An293.
SEQ ID NO: 95 shows the amino acid sequence of 1,5-anhydroglucitol-6-phosphate isomerase with the Uniprot accession No. A0A7Z0VH52.
SEQ ID NO: 96 shows the amino acid sequence of 1,5-anhydroglucitol-6-phosphate isomerase with the Uniprot accession No. A0A0I9WIZ4.
SEQ ID NO: 97 shows the amino acid sequence of 1,5-anhydromannitol-6-phosphate isomerase with the NCBI accession No. OCX05109.1, expressed by *Streptococcus dysgalactiae subsp. equisimilis*.
SEQ ID NO: 98 shows the amino acid sequence of 1,5-anhydromannitol-6-phosphate isomerase with the Uniprot accession No. P32674, expressed by *Escherichia coli* strain K12.
SEQ ID NO: 99 shows the amino acid sequence of 1,5-anhydromannitol-6-phosphate isomerase with the Uniprot accession No. A0A7C9H654, expressed by *Firmicutes bacterium*.
SEQ ID NO: 100 shows the amino acid sequence of 1,5-anhydromannitol-6-phosphate isomerase with the Uniprot accession No. A0A510J954, expressed by *Pseudoleptotrichia goodfellowii*.
SEQ ID NO: 101 shows the amino acid sequence of 1,5-anhydromannitol-6-phosphate isomerase with the Uniprot accession No. D4F6M7, expressed by *Edwardsiella tarda*.
SEQ ID NO: 102 shows the amino acid sequence of 1,5-anhydromannitol-6-phosphate isomerase with the Uniprot accession No. D4E0C4, expressed by *Serratia odorifera* DSM 4582.
SEQ ID NO: 103 shows the amino acid sequence of 1,5-anhydromannitol-6-phosphate isomerase with the Uniprot accession No. A0A2V4E297, expressed by *Gilliamella apicola*.
SEQ ID NO: 104 shows the amino acid sequence of 1,5-anhydromannitol-6-phosphate isomerase with the Uniprot accession No. A0A084T2T3, expressed by *Vibrio sp.* ER1A.
SEQ ID NO: 105 shows the amino acid sequence of 1,5-anhydromannitol-6-phosphate isomerase with the Uniprot accession No. A0A0R2FPV6, expressed by *Lactobacillus selangorensis*.
SEQ ID NO: 106 shows the amino acid sequence of 1,5-anhydromannitol-6-phosphate isomerase with the Uniprot accession No. A0A0N8NM72, expressed by *Caloranaerobacter sp.*.
SEQ ID NO: 107 shows the amino acid sequence of 1,5-anhydromannitol-6-phosphate isomerase with the Uniprot accession No. A0A1T4K0K6, expressed by *Pilibacter termitis*.
SEQ ID NO: 108 shows the amino acid sequence of 1,5-anhydromannitol-6-phosphate isomerase with the Uniprot accession No. A0A096BHK0, expressed by *Caloranaerobacter azorensis* H53214.
SEQ ID NO: 109 shows the amino acid sequence of 1,5-anhydromannitol-6-phosphate isomerase with the Uniprot accession No. A0A725B0Z8, expressed by *Salmonella enteritidis*.
SEQ ID NO: 110 shows the amino acid sequence of 1,5-anhydromannitol-6-phosphate isomerase with the Uniprot accession No. A0A0B8Q7B9, expressed by *Vibrio sp.* JCM 19236.
SEQ ID NO: 111 shows the amino acid sequence of 1,5-anhydromannitol-6-phosphate isomerase with the Uniprot accession No. A0A037YM28, expressed by *Escherichia coli*.
SEQ ID NO: 112 shows the amino acid sequence of 1,5-anhydromannitol-6-phosphate isomerase with the Uniprot accession No. C4LFY8, expressed by *Tolumonas auensis* strain DSM 9187/TA4.
SEQ ID NO: 113 shows the amino acid sequence of 1,5-anhydromannitol-6-phosphate isomerase with the Uniprot accession No. A0A6N7XPT4, expressed by *Senella porci*.
SEQ ID NO: 114 shows the amino acid sequence of 1,5-anhydromannitol-6-phosphate isomerase with the Uniprot accession No. A0A4S2F5T8, expressed by *Coriobacteriaceae* bacterium.
SEQ ID NO: 115 shows the amino acid sequence of 1,5-anhydromannitol-6-phosphate isomerase with the Uniprot accession No. A0A4R7KVA0, expressed by *Fonticella tunisiensis*.
SEQ ID NO: 116 shows the amino acid sequence of 1,5-anhydromannitol-6-phosphate isomerase with the Uniprot accession No. A0A437UWZ5, expressed by *Coriobacteriales* bacterium OH1046.
SEQ ID NO: 117 shows the amino acid sequence of 1,5-anhydromannitol-6-phosphate isomerase with the Uniprot accession No. A0A3R9P0U8, expressed by *Bacillus sp.* HMF5848.
SEQ ID NO: 118 shows the amino acid sequence of 1,5-anhydromannitol-6-phosphate isomerase with the Uniprot accession No. A0A380TM32, expressed by *Actinobacillus rossii*.
SEQ ID NO: 119 shows the amino acid sequence of 1,5-anhydromannitol-6-phosphate isomerase with the Uniprot accession No. C2ELS8, expressed by *Lactobacillus ultunensis* DSM 16047.
SEQ ID NO: 120 shows the amino acid sequence of 1,5-anhydromannitol-6-phosphate isomerase with the Uniprot accession No. A0A2N2B0Z5, expressed by *Firmicutes* bacterium HGW-Firmicutes-7.
SEQ ID NO: 121 shows the amino acid sequence of 1,5-anhydromannitol-6-phosphate isomerase with the Uniprot accession No. A0A1J0GF99, expressed by *Clostridium estertheticum subsp.* estertheticum.
SEQ ID NO: 122 shows the amino acid sequence of 1,5-anhydromannitol-6-phosphate isomerase with the Uniprot accession No. A0A1D8GPW2, expressed by *Geosporobacter ferrireducens*.
SEQ ID NO: 123 shows the amino acid sequence of 1,5-anhydromannitol-6-phosphate isomerase with the Uniprot accession No. A0A0B8PE23, expressed by *Vibrio ishigakensis*.
SEQ ID NO: 124 shows the amino acid sequence of 1,5-anhydromannitol-6-phosphate isomerase with the Uniprot accession No. A0A239SQG5, expressed by *Streptococcus merionis*.
SEQ ID NO: 125 shows the amino acid sequence of 1,5-anhydromannitol-6-phosphate isomerase with the Uniprot accession No. A0A823B0Q1, expressed by *Shigella boydii*.
SEQ ID NO: 126 shows the amino acid sequence of 1,5-anhydromannitol-6-phosphate isomerase with the Uniprot accession No. X0PU04, expressed by *Agrilactobacillus composti* DSM 18527.
SEQ ID NO: 127 shows the amino acid sequence of 1,5-anhydromannitol-6-phosphate isomerase with the Uniprot accession No. A0A0R2FH98, expressed by *Lactobacillus selangorensis*.
SEQ ID NO: 128 shows the amino acid sequence of 1,5-anhydromannitol-6-phosphate isomerase with the Uniprot accession No. A0A430AQ99, expressed by *Vagococcus elongatus*.
SEQ ID NO: 129 shows the amino acid sequence of 1,5-anhydromannitol-6-phosphate isomerase with the Uniprot accession No. A0A1W1WZ75, expressed by *Clostridium acidisoli* DSM 12555.
SEQ ID NO: 130 shows the amino acid sequence of 1,5-anhydromannitol-6-phosphate isomerase with the Uniprot accession No. A0A376L3P4, expressed by *Escherichia coli*.
SEQ ID NO: 131 shows the amino acid sequence of 1,5-anhydromannitol-6-phosphate isomerase with the Uniprot accession No. A0A1T4WMT8, expressed by *Caloramator quimbayensis*.
SEQ ID NO: 132 shows the amino acid sequence of 1,5-anhydromannitol-6-phosphate isomerase with the Uniprot accession No. A0A1I1H9K1, expressed by *Clostridium uliginosum*.
SEQ ID NO: 133 shows the amino acid sequence of 1,5-anhydromannitol-6-phosphate isomerase with the Uniprot accession No. A0A143I1W1, expressed by *Enterobacter asburiae*.
SEQ ID NO: 134 shows the amino acid sequence of 1,5-anhydromannitol-6-phosphate isomerase with the Uniprot accession No. N2BYX9, expressed by *Atopobium minutum*.
SEQ ID NO: 135 shows the amino acid sequence of 1,5-anhydromannitol-6-phosphate isomerase with the Uniprot accession No. A0A4S2EVE2, expressed by *Coriobacteriaceae* bacterium.
SEQ ID NO: 136 shows the amino acid sequence of 1,5-anhydromannitol-6-phosphate isomerase with the Uniprot accession No. A0A7S7M803, expressed by *Thermophilobacter immobilis*.
SEQ ID NO: 137 shows the amino acid sequence of 1,5-anhydromannitol-6-phosphate isomerase with the Uniprot accession No. A0A2T3N2G1, expressed by *Photobacterium lipolyticum*.
SEQ ID NO: 138 shows the amino acid sequence of 1,5-anhydromannitol-6-phosphate isomerase with the Uniprot accession No. A0A381GG45, expressed by *Citrobacter amalonaticus*.
SEQ ID NO: 139 shows the amino acid sequence of 1,5-anhydromannitol-6-phosphate isomerase with the Uniprot accession No. A0A0A0F8F5, expressed by *Escherichia coli* G3.
SEQ ID NO: 140 shows the amino acid sequence of 1,5-anhydromannitol-6-phosphate isomerase with the Uniprot accession No. A0A0J0GCD9, expressed by *Enterobacter asburiae*.
SEQ ID NO: 141 shows the amino acid sequence of 1,5-anhydromannitol-6-phosphate isomerase with the Uniprot accession No. A0A0H3J2B1, expressed by *Clostridium pasteurianum*.
SEQ ID NO: 142 shows the amino acid sequence of 1,5-anhydromannitol-6-phosphate isomerase with the Uniprot accession No. D1AJA5, expressed by *Sebaldella termitidis* strain ATCC 33386/NCTC 11300.
SEQ ID NO: 143 shows the amino acid sequence of 1,5-anhydromannitol-6-phosphate isomerase with the Uniprot accession No. A0A8S0W6S0, expressed by *Acididesulfobacillus acetoxydans*.
SEQ ID NO: 144 shows the amino acid sequence of 1,5-anhydromannitol-6-phosphate isomerase with the Uniprot accession No. A0A7W2G1V0, expressed by *Clostridium sp*..
SEQ ID NO: 145 shows the amino acid sequence of LpYbiY with the Uniprot accession No. A0A162FGG2, expressed by *Lactiplantibacillus plantarum*.
SEQ ID NO: 146 shows the amino acid sequence of LpFsaA with the Uniprot accession No. A0A0M4CJJ6, expressed by *Lactiplantibacillus plantarum*.
SEQ ID NO: 147 shows the amino acid sequence of EcPflC with the Uniprot accession No. P32675, expressed by *Escherichia coli* strain K12.
SEQ ID NO: 148 shows the amino acid sequence of EcFsaB with the Uniprot accession No. P32669, expressed by *Escherichia coli* strain K12.
SEQ ID NO: 149 shows the amino acid sequence of EcGldA with the Uniprot accession No. P0A9S5, expressed by *Escherichia coli* strain K12.
SEQ ID NO: 150 shows the amino acid sequence of PtsA, a subunit of the PTS with the Uniprot accession No. P32670, expressed by *Escherichia coli* strain K12.
SEQ ID NO: 151 shows the amino acid sequence of FrwB, a subunit of the PTS with the Uniprot accession No. P69816, expressed by *Escherichia coli* strain K12.
SEQ ID NO: 152 shows the amino acid sequence of FrwC, a subunit of the PTS with the Uniprot accession No. P32672, expressed by *Escherichia coli* strain K12.
SEQ ID NO: 153 shows the amino acid sequence of FrwD, a subunit of the PTS with the Uniprot accession No. P32676, expressed by *Escherichia coli* strain K12.
SEQ ID NO: 154 shows the nucleotide sequence of Primer 1F.
SEQ ID NO: 155 shows the nucleotide sequence of Primer 1R.
SEQ ID NO: 156 shows the nucleotide sequence of Primer 2F.
SEQ ID NO: 157 shows the nucleotide sequence of Primer 2R.
SEQ ID NO: 158 shows the nucleotide sequence of Primer 3F.
SEQ ID NO: 159 shows the nucleotide sequence of Primer 3R.
SEQ ID NO: 160 shows the nucleotide sequence of Primer 4F.
SEQ ID NO: 161 shows the nucleotide sequence of Primer 4R.
SEQ ID NO: 162 shows the nucleotide sequence of Primer 5F.
SEQ ID NO: 163 shows the nucleotide sequence of Primer 5R.
SEQ ID NO: 164 shows the nucleotide sequence of Primer 6F.
SEQ ID NO: 165 shows the nucleotide sequence of Primer 6R.
SEQ ID NO: 166 shows the nucleotide sequence of Primer 7F.
SEQ ID NO: 167 shows the nucleotide sequence of Primer 7R.
SEQ ID NO: 168 shows the nucleotide sequence of Primer 8F.
SEQ ID NO: 169 shows the nucleotide sequence of Primer 8R.
SEQ ID NO: 170 shows the nucleotide sequence of Primer 9F.
SEQ ID NO: 171 shows the nucleotide sequence of Primer 9R.
SEQ ID NO: 172 shows the nucleotide sequence of Primer 10F.
SEQ ID NO: 173 shows the nucleotide sequence of Primer 10R.
SEQ ID NO: 174 shows the nucleotide sequence of Primer 11F.
SEQ ID NO: 175 shows the nucleotide sequence of Primer 11R.
SEQ ID NO: 176 shows the nucleotide sequence of Primer 12F.
SEQ ID NO: 177 shows the nucleotide sequence of Primer 12R.
SEQ ID NO: 178 shows the nucleotide sequence of Primer 13F.
SEQ ID NO: 179 shows the nucleotide sequence of Primer 13R.
SEQ ID NO: 180 shows the nucleotide sequence of Primer 14F.
SEQ ID NO: 181 shows the nucleotide sequence of Primer 14R.
SEQ ID NO: 182 shows the nucleotide sequence of Primer 15F.
SEQ ID NO: 183 shows the nucleotide sequence of Primer 16F.
SEQ ID NO: 184 shows the nucleotide sequence of Primer 16R.
SEQ ID NO: 185 shows the nucleotide sequence of Primer 17F.
SEQ ID NO: 186 shows the nucleotide sequence of Primer 17R.
SEQ ID NO: 187 shows the nucleotide sequence of the guide RNA contained in the pRed_Cas9_recA_ΔybiW plasmid.
SEQ ID NO: 188 shows the nucleotide sequence of the guide RNA contained in the pRed_Cas9_recA_ΔpflD plasmid.
SEQ ID NO: 189 shows the nucleotide sequence of Primer 18F.
SEQ ID NO: 190 shows the nucleotide sequence of Primer 18R.
SEQ ID NO: 191 shows the nucleotide sequence of Primer 19F.
SEQ ID NO: 192 shows the nucleotide sequence of Primer 19R.

### Detailed Description of the Invention

Through bioinformatics analysis, the inventors of the present disclosure discovered for the first time two new radical-dependent glycolytic pathways. Through heterologous expression and purification of related enzymes in these two new pathways, enzyme activity was verified by LC-MS detection, and enzyme kinetic parameters were characterized, and the catalytic mechanism of key enzymes (e.g., 1,5-anhydroglucitol-6-phosphate isomerase or 1,5-anhydromannitol-6-phosphate isomerase) was explored by combining with protein crystallography analysis. When using 1,5-anhydroglucitol and 1,5-anhydromannitol each as a sole carbon source, related pathway proteins were highly expressed in *E. coli* cells. Knockout of key genes (e.g., genes encoding 1,5-anhydroglucitol-6-phosphate isomerase or 1,5-anhydromannitol-6-phosphate isomerase) resulted in the inability of *E. coli* to grow when using 1,5-anhydroglucitol or 1,5-anhydromannitol as a sole carbon source, indicating that these two new glycolytic pathways discovered by the inventors can be utilized by *E. coli* for energy production and growth. These are new glycolytic pathways discovered since the 1920s to 1950s when EMP, ED and the pentose phosphate pathway were identified. Both of these new pathways can produce 1,2-propanediol.

Unless otherwise indicated, terms used in the present disclosure have the meanings commonly understood by those skilled in the art.

### Definitions

Unless otherwise indicated, nucleic acids are written from left to right in a 5' to 3' direction, and amino acid sequences are written from left to right in an amino to carboxy direction, respectively. Numerical ranges include the numbers defining the range. Amino acids may be referred to herein by either their commonly known three-letter symbols or by the single-letter symbols recommended by the IUPAC-IUB Biochemical Nomenclature classification. Likewise, nucleotides may be referred to herein by commonly accepted single-letter codes. The terms defined above are more fully defined by reference to the specification as a whole. As used herein, amino acid residues are abbreviated as follows: Alanine is shown in Ala or A; Arginine is shown in Arg or R; Asparagine is shown in Asn or N; Aspartic acid is shown in Asp or D; Cysteine is shown in Cys or C; Glutamic acid is shown in Glu or E; Glutamine is shown in Gln or Q; Glycine is shown in Gly or G; Histidine is shown in His or H; Isoleucine is shown in Ile or I; Leucine is shown in Leu or L; Lysine is shown in Lys or K; Methionine is shown in Met or M; Phenylalanine is shown in Phe or F; Proline is shown in Pro or P; Serine is shown in Ser or S; Threonine is shown in Thr or T; Tryptophan is shown in Trp or W; Tyrosine is shown in Tyr or Y; and Valine is shown in Val or V.

The terms "polypeptide" and "protein" are used interchangeably herein and refer to polymers of amino acid residues and variants thereof, as well as synthetic and natural analogs thereof. Thus, these terms apply to natural amino acid polymers and natural chemical derivatives theirof, as well as amino acid polymers in which one or more amino acid residues are synthetic, unnatural amino acids (such as chemical analogs of the corresponding natural amino acids). Such derivatives include, for example, post-translational modifications and degradation products, including phosphorylated, glycosylated, oxidized, isomerized, carboxylated, and deamidated variants of polypeptide fragments.

As used herein, the term "active centor of an enzyme" refers to a region of an enzyme molecule that directly binds to a substrate molecule and catalyzes a chemical reaction of the substrate, and this region constitutes the active centor of the enzyme.

As used herein, the term "amino acid" refers to a compound in which a hydrogen atom on the carboxyl carbon atom is replaced by an amino group, and an amino acid molecule contains both an amino group and a carboxyl group. The amino acid includes natural and unnatural amino acids, as well as amino acid analogs and mimetics. natural amino acids include 20 types of (*L*)-amino acids used in protein biosynthesis, as well as other amino acids, such as 4-hydroxyproline, hydroxylysine, carboxylated lysine, desmosine, isodesmosine, homocysteine, citrulline, and ornithine. Unnatural amino acids include, for example, (*D*)-amino acids, norleucine, norvaline, p-fluorophenylalanine, ethionine, etc., as are known to those skilled in the art. Amino acid analogs include modified forms of natural and unnatural amino acids. Such modifications may include, for example, the substitution of chemical groups or moieties on the amino acid, or derivatization of the amino acid. Amino acid mimetics include, for example, organic structures that exhibit functionally similar properties, such as the charge and charge spatial characteristics of an amino acid. For example, an organic structure that mimics arginine (Arg or R) has a positively charged moiety located in a similar molecular space and having a degree of mobility similar to the ε-amino group of the side chain of a natural Arg amino acid. Mimetics also include constrained structures to maintain optimal spatial and charge interactions of amino acids or amino acid functional groups. A person skilled in the art is able to determining what structure constitutes a functionally equivalent amino acid analog or amino acid mimetic.

As used herein, the term "isozyme" refers to an enzyme that catalyzes the same reaction in an organism but has a different molecular structure.

As used herein, the term "nucleic acid" refers to mRNA, RNA, cRNA, cDNA, or DNA, including single-stranded and double-stranded forms of DNA. The term "nucleic acid" generally refers to a polymeric form of nucleotides of at least 10 bases in length, and said nucleotides are ribonucleotides or deoxynucleotides or modified forms of either type of nucleotide.

As used herein, the term "encoding" when used in the context of a specific nucleic acid means that the nucleic acid includes necessary information to direct the translation of the nucleotide sequence into a specific protein. The information encoding the protein is indicated by codons. A nucleic acid encoding a protein may include untranslated sequences (e.g., introns) within a translation region of the nucleic acid, or may lack such intervening untranslated sequences (e.g., as in cDNA).

As used herein, the term "full-length sequence" with respect to a particular polynucleotide or the protein it encodes means the entire nucleic acid sequence or the entire amino acid sequence of the natural (non-synthetic) endogenous sequence. A full-length polynucleotide encodes the full-length, catalytically active form of the particular protein. As used herein, the term "isolated" refers to a polypeptide or nucleic acid or a biologically active portion thereof that is substantially or essentially free from components that normally accompany or interact with the protein or nucleic acid as found in its natural environment. Thus, when an isolated polypeptide or nucleic acid is produced by recombinant techniques, the isolated polypeptide or nucleic acid is substantially free of other cellular material or culture medium, or when an isolated polypeptide or nucleic acid is chemically synthesized, the isolated polypeptide or nucleic acid is substantially free of chemical precursors or other chemicals.

As used herein, the term "expression vector" is a recombinantly or synthetically produced nucleic acid construct having a series of specific nucleic acid elements that permit transcription of a particular nucleic acid in a host cell. As used herein, the term "host cell" refers to a cell that receives an exogenous gene in transformation or transduction (infection). A host cell can be a eukaryotic cell, such as a yeast cell, or a prokaryotic cell, such as *Escherichia coli*. As used herein, the term "phosphoenolpyruvate-phosphotransferase system (PTS)" refers to an enzyme complex widely present in bacteria, fungi, and some archaea. PTS consists of cytoplasmic enzyme I (EI) or histidine phosphocarrier protein (HPr or NPr) and phosphotransferases, such as sugar-specific enzyme II complexes, and has both catalytic transport functions and a wide range of regulatory functions. The bacterial phosphoenolpyruvate-phosphotransferase system primarily phosphorylates various sugars and their derivatives through a phosphorylation cascade reaction, followed by transport them into a cell. All PTSs depend on cytoplasmic enzyme I (EI) and the histidine phosphocarrier protein (HPr), HPr phosphorylates the sugar via a sugar-specific EII complex, which is composed of two cytoplasmic domains (EIIA and EIIB) and one or two membrane domains (EIIC or EIID). The EIIC or EIID domain is associated with the membrane and transfers the sugar into the cytoplasm, where the sugar undergoes a multi-stage phosphorylation process involving the EIIA and EIIB domains. The phosphate group of phosphoenolpyruvate is transferred from EI to HPr, then from HPr to EIIA, then from EIIA to EIIB, and then to the sugar transported by EIIC or EIID. Most bacteria use the phosphoenolpyruvate-phosphotransferase system (PTS) to transport sugars such as glucose. EI catalyzes the phosphotransfer reaction from the glycolytic intermediate phosphoenolpyruvate (PEP) to HPr. HPr then transfers the phosphate group to various EIIA proteins, and then to EIIB. Finally, the carbohydrate is transported across the membrane through EIIC and EIID while being phosphorylated by EIIB.

### Specific Embodiments

In a first aspect, the present disclosure provides a polypeptide, including an amino acid sequence shown in SEQ ID NO: 1 or 97, or a functional variant thereof, where the functional variant possesses 1,5-anhydroalditol-6-phosphate isomerase activity.

In some embodiments of the first aspect, the present disclosure provides a polypeptide, including an amino acid sequence shown in SEQ ID NO: 1 or a functional variant thereof, where the functional variant possesses 1,5-anhydroglucitol-6-phosphate isomerase activity. In some embodiments, the polypeptide has an active site in its three-dimensional conformation defined such that, with reference to SEQ ID NO: 1, the active site includes amino acid residues H165, H282, S283, H334, C441, E443, R453, T455, L562, S662, I664, and G786, which are spatially close to one another.

In some embodiments of the first aspect, the functional variant is a natural isozyme of the amino acid sequence shown in SEQ ID NO: 1.

In some embodiments of the first aspect, the natural isozyme of the amino acid sequence shown in SEQ ID NO: 1 is derived from: *Lactiplantibacillus plantarum*, *Escherichia coli*, *Lactobacillus selangorensis*, *Streptococcus uberis*, *Olsenella sp.*, *Leptotrichia wadei*, *Streptococcus parauberis*, *Anaerostipes hadrus*, *Senella sp.*, *Selenomonas ruminantium*, *Orenia metallireducens*, *Bifidobacterium primatium*, *Leptotrichia hofstadii*, *Ligilactobacillus agilis*, *Senella profusa*, *Clostridium butyricum*, *Vibrio ishigakensis*, *Coriobacteriaceae*, *Coriobacterium glomerans*, *Eggerthia cateniformis*, *Clostridium baratii str. Sullivan*, *Clostridiales, Firmicutes, Clostridium vincentii*, *Streptococcus downei, Erysipelotrichaceae, Quinella sp., Sebaldella termitidis, Ligilactobacillus animalis, Liquorilactobacillus satsumensis, Clostridium botulinum, Acetivibrio ethanolgignens, Aeromonas sobria, Clostridium cadaveris, Streptococcus bovimastitidis, Sodalis ligni, Lactococcus raffinolactis, Lactobacillus ultunensis, Leptotrichia sp., Lucifera butyrica, Fonticella tunisiensis, Cronobacter sakazakii, Liquorilactobacillus uvarum, Anaeromassilibacillus sp., Anaerotruncus sp., Beauveria bassiana, Cedecea lapagei, Streptococcus pneumoniae, Streptococcus suis, Aeromonas veronii, Lactococcus raffinolactis, Streptococcus sanguinis, Actinomyces succiniciruminis, Vagococcus humatus, Vibrio alginolyticus, Ligilactobacillus acidipiscis, Pilibacter termitis, Lachnospiraceae, Aeromonas hydrophila, Enterococcus ratti, Kandleria vitulina, Clostridium pasteurianum, Aeromonas allosaccharophila, Pantoea alhagi, Peptacetobacter hiranonis, Edwardsiella piscicida, Cedecea neteri, Chelonobacter oris, Aeromonas sp., Clostridium sp., Vibrio gazogenes, Melissococcus plutonius, Tolumonas sp., Loigolactobacillus coryniformis, Zooshikella ganghwensis, Vibrio sp., Photobacterium lipolyticum, Agrilactobacillus composti, Clostridium symbiosum*, or *Aerococcus sp..*

In some specific embodiments of the first aspect, the natural isozyme of the amino acid sequence shown in SEQ ID NO: 1 includes an amino acid sequence shown in any one of SEQ ID NOs: 2-96.

In some embodiments of the first aspect, the present disclosure provides a polypeptide, including an amino acid sequence shown in SEQ ID NO: 97 or a functional variant thereof, where the functional variant possesses 1,5-anhydromannitol-6-phosphate isomerase activity. In some embodiments, the polypeptide has an active site in its three-dimensional conformation defined such that, with reference to SEQ ID NO: 97, the active site includes amino acid residues Q162, H169, S277, S278, R323, F331, P335, C431, E433, D445, Y628, V630, and G752.

In some embodiments of the first aspect, the functional variant is a natural isozyme of the amino acid sequence shown in SEQ ID NO: 97.

In some embodiments of the first aspect, the natural isozyme of the amino acid sequence shown in SEQ ID NO: 97 is derived from: *Escherichia coli, Streptococcus dysgalactiae subsp. equisimilis, Firmicutes, Pseudoleptotrichia goodfellowii, Edwardsiella tarda, Serratia odorifera, Gilliamella apicola, Vibrio sp., Lactobacillus selangorensis, Caloranaerobacter sp., Pilibacter termitis, Caloranaerobacter azorensis, Salmonella enteritidis, Tolumonas auensis, Senella porci, Coriobacteriaceae, Fonticella tunisiensis, Coriobacteriales, Bacillus sp., Actinobacillus rossii, Lactobacillus ultunensis, Clostridium estertheticum, Geosporobacter ferrireducens, Vibrio ishigakensis, Streptococcus merionis, Shigella boydii, Agrilactobacillus composti, Lactobacillus selangorensis, Vagococcus elongatus, Clostridium acidisoli, Caloramator quimbayensis, Clostridium uliginosum, Enterobacter asburiae, Atopobium minutum, Thermophilobacter immobilis, Photobacterium lipolyticum, Citrobacter amalonaticus, Clostridium pasteurianum, Sebaldella termitidis, Acididesulfobacillus acetoxydans,* or *Clostridium sp..*

In some specific embodiments of the first aspect, the natural isozyme of the amino acid sequence shown in SEQ ID NO: 97 includes an amino acid sequence shown in any one of SEQ ID NOs: 98-144.

In some specific embodiments of the first aspect, an substrate of the polypeptide is 1,5-anhydroalditol-6-phosphate. In some specific embodiments of the first aspect, the 1,5-anhydroalditol-6-phosphate is 1,5-anhydroglucitol-6-phosphate or 1,5-anhydromannitol-6-phosphate.

In some specific embodiments of the first aspect, when the polypeptide possesses 1,5-anhydroglucitol-6-phosphate isomerase activity, the polypeptide isomerizes 1,5-anhydroglucitol-6-phosphate to produce 1-deoxyfructose-6-phosphate.

In some specific embodiments of the first aspect, when the polypeptide possesses 1,5-anhydromannitol-6-phosphate isomerase activity, the polypeptide isomerizes 1,5-anhydromannitol-6-phosphate to produce 1-deoxyfructose-6-phosphate.

In some embodiments of the first aspect, the functional variant is generated by one or more amino acid insertions, substitutions, and/or deletions in the amino acid sequence shown in SEQ ID NO: 1 or 97, or a natural isozyme thereof.

In some embodiments of the first aspect, the insertions, substitutions, and/or deletions do not occur in the active site. In some embodiments of the first aspect, the number of amino acid insertions, substitutions and/or deletions is 1-30, preferably 1-20, more preferably 1-10, where an obtained functional variant is basically remaining the 1,5-anhydroalditol-6-phosphate isomerase (e.g., 1,5-anhydroglucitol-6-phosphate isomerase or 1,5-anhydromannitol-6-phosphate isomerase) activity unchanged.

In some embodiments of the first aspect, the functional variant differs from the amino acid sequence shown in SEQ ID NO: 1 or 97 by about 1, 2, 3, 4, 5, 6, 7, 8, 9, or 10 amino acid insertions, substitutions, and/or deletions.

In some embodiments of the first aspect, the functional variant differs from the amino acid sequence shown in SEQ ID NO: 1 or 97 by about 1, 2, 3, 4, or 5 amino acid insertions, substitutions, and/or deletions.

In some embodiments of the first aspect, the polypeptide is an isolated polypeptide.

In some embodiments of the first aspect, the polypeptide belongs to the glycine radical enzyme family. When the polypeptide possesses 1,5-anhydroglucitol-6-phosphate isomerase or 1,5-anhydromannitol-6-phosphate isomerase activity, its catalysis involves glycine and cysteine radicals.

In a second aspect, the present disclosure provides a nucleic acid molecule encoding the polypeptide of the first aspect.

In some embodiments of the second aspect, the nucleic acid molecule of the present disclosure includes a nucleic acid that hybridizes to a nucleotide sequence encoding the polypeptide shown in any one of SEQ ID NOs: 1-96 under stringent conditions, or the nucleic acid molecule of the present disclosure is composed of nucleic acids that specifically hybridize to a nucleotide sequence encoding the polypeptide shown in any one of SEQ ID NOs: 1-96 and encodes a polypeptide that is functionally equivalent to the polypeptide shown in any one of SEQ ID NOs: 1-96. In some embodiments of the second aspect, the nucleic acid molecule of the present disclosure includes a nucleic acid that hybridizes to a nucleotide sequence encoding the polypeptide shown in any one of SEQ ID NOs: 97-144 under stringent conditions, or the nucleic acid molecule of the present disclosure is composed of nucleic acids that specifically hybridize to a nucleotide sequence encoding the polypeptide shown in any one of SEQ ID NOs: 97-144 and encodes a polypeptide that is functionally equivalent to the polypeptide shown in any one of SEQ ID NOs: 97-144.

Those skilled in the art can routinely select stringent conditions for DNA hybridization. Generally longer probes require higher temperatures for proper annealing, while shorter probes require lower temperatures. Hybridization generally depends on the ability of denatured DNA to reanneal when the complementary strand is in an environment below its melting temperature. The higher the degree of homology between the probe and the hybridizable sequence, the higher the relative temperature that can be used. Therefore, a higher relative temperature tends to make the reaction conditions more stringent, while at a lower temperature, the stringency is lower. For a detailed description of the stringent conditions of the hybridization reaction, Ausubel et al., Current Protocols in Molecular Biology, Wiley Interscience Publishers, (1995) can be referred.

In some embodiments of the second aspect, stringent conditions used for DNA hybridization include: 1) Low ionic strength and high temperature are used for washing, for example, 0.015M sodium chloride/0.0015M sodium citrate/0.1% sodium alkyl sulfate at 50 °C.; 2) Formamide and other denaturants are used for hybridization at 42° C, such as 50% (v/v) formamide plus 0.1% bovine serum albumin/0.1% Ficoll/0.1% polydiene pyrrolidone/50 mM sodium phosphate buffer (pH 6.5), 750 mM sodium chloride, 75 mM sodium citrate; or (3) hybridization is performed overnight at 42 °C, where the hybridization solution contains 50% formamide, 5×SSC (0.75M sodium chloride, 0.075M citric acid sodium), 50 mM sodium phosphate (pH 6.8), 0.1% sodium pyrophosphate, 5× Denhardt's solution, sonicated salmon sperm DNA (50 mg/mL), 0.1% SDS and 10% dextran sulfate, then DNA is washed in 0.2×SSC (sodium chloride/sodium citrate) at 42°C for 10 minutes, and then washed with high stringency at 55° C with 0.1×SSC containing EDTA. The moderately stringent conditions can be determined as described in Sambrook et al., Molecular Cloning: A Laboratory Manual, New York: Cold Spring Harbor Press, 1989. Moderately stringent conditions include the use of washing solutions and hybridization conditions (such as temperature, ionic strength, and SDS percentage) that are less stringent than those described above. For example, moderately stringent conditions include hybridization with at least about 16% v/v to at least about 30% v/v formamide and at least about 0.5 M to at least about 0.9 M salt at 42 °C, and at least about 0.1 M to at least about 0.2M salt is washed at 55 °C. Moderately stringent conditions can also include hybridization with 1% bovine serum albumin (BSA), 1 mM EDTA, 0.5M NaHPO4 (pH 7.2), 7% SDS at 65 °C, and (i) 2×SSC, 0.1% SDS; or (ii) 0.5% BSA, 1 mM EDTA, 40 mM NaHPO4 (pH 7.2), 5% SDS wash at 60-65 °C. Professionals will adjust the temperature and ionic strength according to the length of the probe and other factors. The stringency of hybridizing nucleic acids depends on the length and degree of complementarity of the nucleic acid molecules, as well as other variables well known in the art. The greater the similarity or homology between two nucleotide sequences, the greater the Tm of nucleic acid hybrids containing these sequences. The relative stability of nucleic acid hybridization (corresponding to higher Tm) decreases in the following order: RNA:RNA, DNA:RNA, DNA:DNA. Preferably, the minimum length of a hybridizable nucleic acid is at least about 12 nucleotides, preferably at least about 16, more preferably at least about 24, and most preferably at least about 36 nucleotides.

The nucleic acid molecules of the present disclosure can be combined with other DNA sequences, such as promoters, polyadenylation signals, other restriction sites, multiple cloning sites, other coding segments, etc., so that their total length can vary significantly. It is therefore considered that polynucleotide fragments of almost any length can be utilized; the total length is preferably limited by the ease of preparation and use in the intended recombinant DNA protocol.

Any one of a variety of mature technologies known and available in the art can be used to prepare, manipulate, and/or express polynucleotides and their fusions. For example, nucleic acid molecules encoding the polypeptides of the present disclosure or functional variants thereof can be used in recombinant DNA molecules to direct the expression of the polypeptides in appropriate host cells. Due to the inherent degeneracy of the genetic code, other DNA sequences encoding substantially the same or functionally equivalent amino acid sequences can also be used in this disclosure, and these sequences can be used to clone and express a given polypeptide.

In addition, methods known in the art can be used to modify the nucleic acid molecules of the present disclosure, including but not limited to the cloning, processing, expression and/or activity modification of gene products.

In some embodiments of the second aspect, the nucleic acid molecule is produced by artificial synthesis, such as direct chemical synthesis or enzymatic synthesis.

In some embodiments of the second aspect, the nucleic acid molecule is produced by recombinant technology.

In some embodiments of the second aspect, the nucleic acid molecule is an isolated nucleic acid molecule.

In a third aspect, the present disclosure provides an expression cassette, including the nucleic acid molecule of the second aspect.

In some specific embodiments of the third aspect, the expression cassette may additionally include a 5'leader sequence, which can play a role in enhancing translation.

When preparing the expression cassette, various DNA fragments can be manipulated to provide the DNA sequence in the proper orientation and in the proper reading frame when appropriate. To achieve this goal, adaptors or linkers can be used to connect DNA fragments, or other operations can be involved to provide convenient restriction sites, remove excess DNA, remove restriction sites, and so on. For this purpose, it may involve in vitro mutagenesis, primer repair, restriction, annealing, and replacement, such as transition and transversion.

In a fourth aspect, the present disclosure provides an expression vector, including the nucleic acid molecule of the second aspect or the expression cassette of the third aspect.

In some embodiments of the fourth aspect, any suitable expression vector can be used in the present disclosure. For example, the expression vector can be a vector suitable for a system of *Escherichia coli*. in the present disclosure. In some embodiments, the expression vector can be any one of the vectors such as HT or pACYC.

In some embodiments of the fourth aspect, the nucleic acid molecule encoding the polypeptide shown in any one of SEQ ID NOs: 1-96 or the nucleic acid molecule encoding the polypeptide shown in any one of SEQ ID NOs: 97-144 is cloned into a vector to form a recombinant vector containing the nucleic acid molecules of the present disclosure. In some embodiments of the fourth aspect, the expression vector used to clone the polynucleotide is a plasmid vector.

In some embodiments of the fourth aspect, the above-mentioned expression vector further includes a control sequence for regulating the expression of the nucleic acid molecule, where the nucleic acid molecule is operably linked to the control sequence.

The term "regulatory sequence" as used herein refers to a polynucleotide sequence required to achieve expression of a coding sequence linked to it. The nature of such regulatory sequences varies with the host organism. In prokaryotes, such regulatory sequences generally include promoters, ribosome binding sites, and terminator; in eukaryotes, such regulatory sequences generally include promoters, terminators, and in some cases enhancers. Therefore, the term "regulatory sequence" includes all sequences whose existence is the minimum necessary for the expression of the target gene, and may also include other sequences whose existence is advantageous for the expression of the target gene, such as leader sequences.

The term "operably linked" as used herein refers to a situation where the involved sequences are in a relationship that allows them to function in a desired manner. Thus, for example, a regulatory sequence "operably linked" to a coding sequence allows the expression of the coding sequence to be achieved under conditions compatible with the regulatory sequence.

In some embodiments of the fourth aspect, methods well known to those skilled in the art are used to construct a nucleotide sequence that encodes the polypeptide shown in any one of SEQ ID NOs: 1-96 or a nucleotide sequence that encodes the polypeptide shown in any one of SEQ ID NOs: 97-144 and suitable expression vector for transcription/translation regulatory elements. These methods include in vitro recombinant DNA technology, DNA synthesis technology, in vivo recombination technology, etc. (Sambroook, et al. Molecular Cloning, a Laboratory Manual, cold Spring Harbor Laboratory. New York, 1989). The nucleotide sequence is operably linked to an appropriate promoter in the expression vector to direct mRNA synthesis. Representative examples of these promoters include: *Escherichia coli* lac or trp promoter; λ phage PL promoter; eukaryotic promoters include CMV immediate early promoter, HSV thymidine kinase promoter, early and late SV40 promoter, Retrovirus LTRs and some other known promoters that can control gene expression in prokaryotic cells or eukaryotic cells or their viruses. The expression vector also includes a ribosome binding site for translation initiation and a transcription terminator. Inserting an enhancer sequence into the vector will enhance its transcription in higher eukaryotic cells. Enhancers are cis-acting factors of DNA expression, usually about 10 to 300 base pairs, acting on promoters to enhance gene transcription. Examples include SV40 enhancers of 100 to 270 base pairs on the late side of the replication initiation point, polyoma enhancers and adenovirus enhancers on the late side of the replication initiation point, and etc.

In addition, the expression vector preferably contains one or more selectable marker genes to provide phenotypic traits for selection of transformed host cells, such as those encoding kanamycin sulfate resistance, ampicillin resistance, and etc.

In a fifth aspect, the present disclosure provides a host cell, including the nucleic acid molecule of the second aspect or the expression cassette of the third aspect or the expression vector of the fourth aspect.

In some embodiments of the fifth aspect, the host cell is capable of expressing and producing a polypeptide including an amino acid sequence shown in SEQ ID NO: 1 or 97, or a functional variant thereof, where the functional variant possesses 1,5-anhydroalditol-6-phosphate isomerase activity.

In some embodiments of the fifth aspect, the host cell is capable of expressing and producing a polypeptide including the amino acid sequence shown in SEQ ID NO: 1 or a functional variant thereof, where the functional variant possesses 1,5-anhydroglucitol-6-phosphate isomerase activity. In some embodiments, the polypeptide has an active site in its three-dimensional conformation defined such that, with reference to SEQ ID NO: 1, the active site includes amino acid residues H165, H282, S283, H334, C441, E443, R453, T455, L562, S662, I664, and G786, which are spatially close to one another.

In some embodiments of the fifth aspect, the host cell is capable of expressing and producing a polypeptide including the amino acid sequence shown in SEQ ID NO: 97 or a functional variant thereof, where the functional variant possesses 1,5-anhydromannitol-6-phosphate isomerase activity. In some embodiments, the polypeptide has an active site in its three-dimensional conformation defined such that, with reference to SEQ ID NO: 97, the active site includes amino acid residues Q162, H169, S277, S278, R323, F331, P335, C431, E433, D445, Y628, V630, and G752.

In some embodiments of the fifth aspect, the host cell further expresses an activating enzyme, an aldolase, a hydroxyacetone reductase, and/or a transport complex.

In some embodiments of the fifth aspect, the host cell further expresses an S-adenosylmethionine radical enzyme family glycine radical enzyme activating enzyme (Enzyme classification No. EC:1.97.1.4) of the polypeptide of the first aspect, a 1-deoxyfructose-6-phosphate aldolase (also known as fructose-6-phosphate aldolase, Enzyme classification No. EC:4.1.2.-), a hydroxyacetone reductase (also known as glycerol dehydrogenase, Enzyme classification No. EC:1.1.1.6), and/or the transport complex.

In some embodiments of the fifth aspect, the transport complex possesses phosphoenolpyruvate-phosphotransferase system transport activity.

In some embodiments of the fifth aspect, the transport complex is a phosphoenolpyruvate-phosphotransferase system.

In some embodiments of the fifth aspect, the transport complex includes at least one of SEQ ID NOs: 150-153 or a functional variant thereof.

In some embodiments of the fifth aspect, the transport complex includes at least two of SEQ ID NOs: 150-153 or functional variants thereof.

In some embodiments of the fifth aspect, the transport complex includes at least three of SEQ ID NOs: 150-153 or functional variants thereof.

In some embodiments of the fifth aspect, the transport complex includes SEQ ID NOs: 150-153 or functional variants thereof.

In some embodiments of the fifth aspect, SEQ ID NOs: 150-153 represent the amino acid sequences of Multiphosphoryl transfer protein 2 (PtsA), PTS system fructose-like EIIB component 2 (FrvB), PTS system fructose-like EIIC component 2 (FrvC), and PTS system fructose-like EIIB component 3 (FrvD), respectively.

In some specific embodiments of the fifth aspect, the four sequences shown in SEQ ID NOs: 150-153 constitute a phosphoenolpyruvate-phosphotransferase system, where PtsA contains EI and EIIA domains, FrvC is EIIC, and FrvB and FrvD are EIIB.

In some specific embodiments of the fifth aspect, PtsA, FrvB, FrvC, and FrvD interact to form a transport complex that exerts phosphoenolpyruvate-phosphotransferase system transport activity. In some embodiments, the transport complex transports 1,5-anhydroglycitol (e.g., 1,5-anhydroglucitol or 1,5-anhydromannitol) into the host cell for subsequent glycolysis.

In some embodiments of the fifth aspect, a functional variant of any one of SEQ ID NOs: 150-153 differs from the corresponding amino acid sequence shown in any one of SEQ ID NOs: 150-153 by about 1, 2, 3, 4, 5, 6, 7, 8, 9, or 10 amino acid insertions, substitutions, and/or deletions.

In some embodiments of the fifth aspect, a functional variant of any one of SEQ ID NOs: 150-153 differs from the corresponding amino acid sequence shown in any one of SEQ ID NOs: 150-153 by about 1, 2, 3, 4, or 5 amino acid insertions, substitutions, and/or deletions.

In some embodiments of the fifth aspect, a usable host cell is a cell containing the above expression vector, which can be a eukaryotic cell, for example, a yeast cell culture system can be used for expression of the polypeptide of the present disclosure. The host cell can also be a prokaryotic cell containing the above expression vector, for example, selected from *Escherichia spp.* (e.g., *E. coli*), *Klebsiella spp.*, *Streptococcus spp.*, *Lactobacillus spp.*, *Bifidobacterium spp.*, *Bacteroidetes*, and *Firmicutes*, etc.

In some specific embodiments of the fifth aspect, the host cell is a yeast cell or *E. coli*.

In some specific embodiments of the fifth aspect, the nucleic acid molecule encoding one or more enzymes of the present disclosure may exist in the host cell in the form of a free vector, or may be integrated into the genome of the host cell.

In some embodiments of any of the above aspects, the isolated nucleic acid is operably linked to a regulatory sequence, which can be recognized by a host cell transformed with the expression vector.

The expression vector can be introduced into the host cell using any technique known in the art, including transformation, transduction, transfection, viral infection, particle bombardment or Ti-mediated gene transfer. Specific methods include calcium phosphate transfection, DEAE-dextran-mediated transfection, lipofection or electroporation (Davis, L., Dibner, M., Battey, I., Basic Methods in Molecular Biology, (1986)). As an example, when the host is a prokaryotic organism such as Escherichia coli, competent cells can be harvested after the exponential growth phase and transformed by the CaCl₂ method well known in the art.

In a sixth aspect, the present disclosure provides a composition including the polypeptide of the first aspect.

In some embodiments of the sixth aspect, the composition further includes: an activating enzyme, an aldolase, and/or a hydroxyacetone reductase.

In some specific embodiments of the sixth aspect, the composition includes: a 1,5-anhydroalditol-6-phosphate isomerase, an S-adenosylmethionine radical enzyme family glycine radical enzyme activating enzyme, a 1-deoxyfructose-6-phosphate aldolase, and a hydroxyacetone reductase.

In some embodiments of the sixth aspect, the S-adenosylmethionine radical enzyme family glycine radical enzyme activating enzyme is an activating enzyme for the 1,5-anhydroalditol-6-phosphate isomerase. In some embodiments, the S-adenosylmethionine radical enzyme family glycine radical enzyme activating enzyme includes an [Fe-S] cluster. In some embodiments, the S-adenosylmethionine radical enzyme family glycine radical enzyme activating enzyme includes a [4Fe-4S] cluster, i.e., includes 4 Fe and 4 S. In some embodiments, the S-adenosylmethionine radical enzyme family glycine radical enzyme activating enzyme catalyzes the cleavage of S-adenosylmethionine (SAM).

In some embodiments of the sixth aspect, the aldolase is a 1-deoxyfructose-6-phosphate aldolase.

In some embodiments of the sixth aspect, the composition is used for catalyzing a conversion of 1,5-anhydroalditol-6-phosphate to 1,2-propanediol.

In some embodiments of the sixth aspect, the 1,5-anhydroalditol-6-phosphate is 1,5-anhydroglucitol-6-phosphate or 1,5-anhydromannitol-6-phosphate.

In a seventh aspect, the present disclosure provides use of the polypeptide of the first aspect, the nucleic acid molecule of the second aspect, the expression cassette of the third aspect, the expression vector of the fourth aspect, or the host cell of the fifth aspect in the preparation of a composition for catalyzing a conversion of 1,5-anhydroalditol-6-phosphate to 1,2-propanediol.

In some embodiments of the seventh aspect, the 1,5-anhydroalditol-6-phosphate is 1,5-anhydroglucitol-6-phosphate or 1,5-anhydromannitol-6-phosphate.

In some specific embodiments, 1,5-anhydroglucitol is transported into the host cell by a transport complex (e.g., a transport complex having phosphoenolpyruvate-phosphotransferase system transport activity) and phosphorylated (e.g., at C-6 position) to yield 1,5-anhydroglucitol-6-phosphate, 1,5-anhydroglucitol-6-phosphate is catalyzed by 1,5-anhydroglucitol-6-phosphate isomerase to yield 1-deoxyfructose-6-phosphate, 1-deoxyfructose-6-phosphate is cleaved by an aldolase (e.g., 1-deoxyfructose-6-phosphate aldolase) to yield hydroxyacetone and 3-phosphoglyceraldehyde, and hydroxyacetone is reduced by hydroxyacetone reductase to yield 1,2-propanediol.

In some specific embodiments, 1,5-anhydromannitol is transported into the host cell by a transport complex (e.g., a transport complex having phosphoenolpyruvate-phosphotransferase system transport activity) and phosphorylated (e.g., at C-6 position) to yield 1,5-anhydromannitol-6-phosphate, 1,5-anhydromannitol-6-phosphate is catalyzed by 1,5-anhydromannitol-6-phosphate isomerase to yield 1-deoxyfructose-6-phosphate, 1-deoxyfructose-6-phosphate is cleaved by an aldolase (e.g., 1-deoxyfructose-6-phosphate aldolase) to yield hydroxyacetone and 3-phosphoglyceraldehyde, and hydroxyacetone is reduced by hydroxyacetone reductase to yield 1,2-propanediol.

In an eighth aspect, the present disclosure provides a method for producing 1,2-propanediol, including culturing the host cell of the fifth aspect in a medium containing 1,5-anhydroglycitol to obtain 1,2-propanediol.

In some embodiments of the eighth aspect, the 1,5-anhydroglycitol is 1,5-anhydroglucitol or 1,5-anhydromannitol. In some embodiments of the eighth aspect, the method further includes steps of collecting a culture and purifying 1,2-propanediol.

In a ninth aspect, the present disclosure provides a method for producing 1,2-propanediol, including contacting the composition of the sixth aspect with 1,5-anhydroalditol-6-phosphate to obtain 1,2-propanediol.

In some embodiments of the ninth aspect, the 1,5-anhydroalditol-6-phosphate is 1,5-anhydroglucitol-6-phosphate or 1,5-anhydromannitol-6-phosphate.

### Examples

The following examples are illustrative only and are not intended to limit the scope of the embodiments of the present disclosure or the scope of the claims.

### Example 1. Identification, characterization, and testing of the YbiW-related pathway

### Materials and Methods

### Experimental Materials

Tryptone and yeast extract used to prepare LB medium were purchased from Oxoid Limited (Hampshire, UK). Ultrapure deionized water from a Millipore Direct-Q system was used. TALON resin was purchased from Clontech Laboratories Inc. (California, USA). 1,5-Anhydroglucitol (1,5-AG) was purchased from Shanghai Yuanye Bio-Technology Co., Ltd., and 1,5-anhydroglucitol-6-phosphate (1,5-AG-6P) was chemically synthesized by Tianjin Heowns Biochemical Technology Co., Ltd. Oligonucleotide primers were synthesized by Beijing Tsingke Biotech Co., Ltd. All protein purification chromatography experiments were performed on an ÄKTA FPLC system (GE Healthcare, USA). Anaerobic experiments were carried out in a Lab2000 glove box (Etelux) protected by N₂ (with an oxygen concentration of less than 5 ppm).

### Gene synthesis and cloning

Gene fragments of LpYbiW (Uniprot accession No.: A0A807DR53), LpYbiY (Uniprot accession No.: A0A162FGG2), and *E. coli* codon-optimized LpFsaA (Uniprot accession No.: A0A0M4CJJ6) were synthesized by Beijing Tsingke Biotech Co., Ltd. LpYbiW and LpFsaA were inserted into the SspI site of the HT plasmid (an optimized pET28 vector) to express proteins with an N-terminal His₆ tag. LpYbiY was inserted into the NdeI site of the pACYC-MBP vector to express a protein with an N-terminal His₆ tag and maltose-binding protein (MBP).

For biochemical characterization purposes, EcYbiW (Uniprot accession No.: P75793) and EcYbiY (Uniprot accession No.: P75794) were obtained by amplifying the *E. coli* MG1655 genome using primers 18F/18R and 19F/19R (Table 6). EcYbiW was inserted into the SspI site of the HT plasmid (an optimized pET28 vector) to express a protein with an N-terminal His₆ tag. EcYbiY was inserted into the NdeI site of the pACYC-MBP vector to express a protein with an N-terminal His₆ tag and maltose-binding protein (MBP).

### Expression and Purification of LpYbiW, LpYbiY, and LpFsaA

HT-LpYbiW, pACYC-MBP-LpYbiY, and HT-LpFsaA plasmids were transformed into *E. coli* BL21(DE3) cells for expression of the corresponding proteins. LB agar plates containing 50 µg/mL kanamycin were used to screen for LpYbiW and LpFsaA, while LB agar plates containing 25 µg/mL chloramphenicol were used to screen for LpYbiY positive clones. Cells were cultured overnight in 4 mL of LB medium, then transferred to fresh LB medium (typically 1 L medium in a 2.6 L flask) and grown in a shaking incubator at 37°C, 220 rpm. When OD₆₀₀ reached approximately 0.8, the temperature was reduced to 18°C, and isopropyl β-D-1-thiogalactopyranoside (IPTG) was added to a final concentration of 0.3 mM to induce production of the target protein. After 16-20 hours, cells were collected by centrifugation (8000 ×g, 10 minutes at 4°C). The harvested cells were resuspended in 40 mL of lysis buffer (50 mM Tris/HCl, pH 8.0, 100 mM KCl, 1 mM phenylmethylsulfonyl fluoride (PMSF), 0.2 mg/mL lysozyme, 0.03% Triton X-100, and 0.02 mg/mL DNase I) and frozen in a refrigerator at -80°C.

The frozen cells were thawed and incubated at room temperature (RT, 25°C) for 20 minutes, during which cell lysis occurred. 5 mM β-mercaptoethanol (BME) was added, and nucleic acids were precipitated by adding 1% streptomycin sulfate. Cell debris was removed by centrifugation at 10,000×g for 10 minutes at 4°C. For LpYbiW and LpFsaA, the supernatant was filtered through a 0.22 µm filter and loaded onto a 5 mL TALON Co²⁺ column (Takara Bio USA, Inc.) pre-equilibrated with buffer A (20 mM Tris/HCl, pH 7.5, 200 mM KCl, and 5 mM BME). The column was washed with 10 times column volumes of buffer A to remove contaminating proteins, and the protein was eluted with 5 times column volumes of buffer A containing 150 mM imidazole. The eluted protein (about 20 mL) was dialyzed against 2 L of buffer A for 3 hours at 4°C, concentrated, aliquoted, frozen in liquid nitrogen, and stored at -80°C. For LpYbiY, the supernatant was filtered and loaded onto a column packed with 10 mL of amylose resin (New England Biolabs, Massachusetts, U.S.A.). The column was washed with 10 times column volumes of buffer A to remove contaminating proteins, and the target protein was eluted with buffer A containing 10 mM maltose.

Purified proteins were detected by SDS-PAGE using commercial gels (SurePAGE, Bis-Tris, 4-20%). Protein concentrations were calculated by measuring absorbance at 280 nm using an ultra-micro UV-visible spectrophotometer (Hangzhou Miou Instruments Co., Ltd.) [LpYbiW (*ε*₂₈₀ = 104,630 M⁻¹ cm⁻¹), MBP-LpYbiY (*ε*₂₈₀ = 100,730 M⁻¹ cm⁻¹), LpFsaA (*ε*₂₈₀ = 20,400 M⁻¹ cm⁻¹)].

### Expression and purification of EcYbiW and EcYbiY for biochemical characterization

The protein expression and purification methods and procedures, SDS-PAGE detection method, and concentration determination method were the same as those described above for LpYbiW and LpYbiY. [EcYbiW (ε₂₈₀ = 99,700 M⁻¹ cm⁻¹), MBP-EcYbiY (ε₂₈₀ = 97,750 M⁻¹ cm⁻¹)].

### Reconstitution and characterization of the LpYbiY prosthetic [Fe-S] cluster

Sequence alignment indicated that LpYbiY contains only one [4Fe-4S] cluster in the radical SAM domain, with a theoretical maximum of 4 Fe and 4 S per monomer. The LpYbiY protein solution was degassed with argon to remove oxygen and then transferred into a glove box. To the degassed protein solution, final concentrations of 100 mM Tris/HCl, (pH 7.5), 10 mM DTT, 4 equivalents of ferrous ammonium sulfate and sodium sulfide were added, and a resulting mixture was incubated overnight in a metal bath at 4°C (Dry Bath H2O3-100C; Coyote Bioscience, Beijing, China). Then, 4 equivalents of EDTA solution were added, followed by repeated concentration using centrifugal filter units (1.5 mL Ym-30 Amicon; Millipore) and buffer exchange by dilution with a buffer (20 mM Tris-HCl, pH 7.5, and 100 mM KCl).

Fe contents of unreconstituted and reconstituted LpYbiY were determined using ferrozine [3-(2-pyridyl)-5,6-diphenyl-1,2,4-triazine-p,p'-disulfonic acid monosodium salt]. An AAS iron standard was used to establish a standard curve for Fe in a range of 0-600 µM. The sulfur contents of unreconstituted and reconstituted LpYbiY were determined by measuring the absorbance of methylene blue formed by reaction with N,N-dimethyl-p-phenylenediamine dihydrochloride (DPD). A standard curve for S in the range of 0-600 µM was established using Na₂S.

UV-Vis absorption spectra of LpYbiY in a range of 200-800 nm were measured using a Nanophotometer NP80 Mobile (Germany). Unreconstituted and reconstituted LpYbiY were diluted to 10 µM with a buffer containing 20 mM Tris/HCl, (pH 7.5), 100 mM KCl, and transferred to a septum-sealed anaerobic cuvette for measurement. To the reconstituted LpYbiY solution, 10 equivalents of titanium(III) citrate were added and incubated for 10 minutes to measure its reduced form.

### LC-MS analysis of S-adenosylmethionine (SAM) cleavage catalyzed by LpYbiY

A reaction system (500 µL) containing 20 mM Tris/HCl, (pH 7.5), 100 mM KCl, 200 µM titanium(III) citrate, 20 µM reconstituted LpYbiY, and 1 mM SAM was incubated overnight at room temperature in a glove box. Titanium(III) citrate was not added to the negative control group. The reaction was quenched by adding formic acid (5% v/v final concentration), and a resulting mixture was incubated in a boiling water bath for 1 minute to denature the protein. Precipitated protein was removed by centrifugation at 14,000×g for 15 minutes. The supernatant was filtered through a 0.22 µm PES membrane and subjected to LC-MS analysis. The method was as follows: 20 µL of sample was loaded onto an Agilent 6420 Triple Quadrupole LC/MS instrument (Agilent Technologies) equipped with a C18 reverse-phase column. UV absorption was detected at 257 nm. The mobile phase system consisted of water (A) and acetonitrile (B). Linear gradient elution was performed from 0 to 16% B over 30 minutes at a flow rate of 0.5 mL/min. Commercial 5'-dA was used as a standard, and the formation of the SAM cleavage product catalyzed by LpYbiY was verified by mass spectrometry.

### Detection of LpYbiW radical generation by electron paramagnetic resonance (EPR) spectroscopy

Continuous-wave X-band EPR spectroscopy was used to characterize the LpYbiW glycyl radical. A reaction system (200 µL) containing 20 mM Tris/HCl, (pH 7.5), 100 mM KCl, 100 µM titanium(III) citrate, 1 mM SAM, 80 µM reconstituted LpYbiY, and 40 µM LpYbiW was incubated for 15 minutes at room temperature in a glove box. 10% glycerol was added, and a resulting mixture was loaded into an EPR tube (Wilmad Lab Glass, 734-LPV-7) with an outer diameter of 4 mm and a length of 8 inches. The tube was sealed with a rubber stopper, removed from the glove box, and frozen using liquid nitrogen before EPR analysis. The experimental spectrum of the glycyl radical was modeled using Bruker Xepr spin fitting to obtain g-values, hyperfine coupling constants, and line widths. Double integration of the simulated spectrum was used to measure the spin concentration. EPR spectra were obtained by accumulating 30 scans under the following conditions: temperature, 90 K; center field, 3370.00 G; sweep width, 200 G; microwave power, 10 µW; microwave frequency, 9.43 MHz; modulation amplitude, 0.5 mT; modulation frequency, 100 kHz; time constant, 20.48 ms; conversion time, 25 ms; sweep time, 20 seconds; receiver gain, 43 dB.

### LC-MS analysis for LpYbiW activity determination

LpYbiW was activated as described for the EPR experiment above, except that glycerol was not added. A 200 µL reaction mixture containing 10 µM activated LpYbiW, 0.1 mM titanium(III) citrate, and 10 mM 1,5-AG-6P was incubated at room temperature for 1 hour in a glove box. In negative controls, 1,5-AG-6P, SAM, or activated LpYbiW was omitted. 200 µL of acetonitrile was added to the reaction system to precipitate protein, the precipitate was removed by centrifugation, and the supernatant was filtered through a 0.22 µm PES membrane for LC-MS analysis.

LC-MS analysis was performed on an Agilent 6420 Triple Quadrupole LC/MS instrument (Agilent Technologies). The drying gas was maintained at a temperature of 300°C, with a flow rate of 9 L/min and a nebulizer pressure of 15 psi. LC-MS analysis was performed using a ZIC-HILIC column (5 µm, 150 × 4.6 mm; Merck). HPLC conditions were as follows: mobile phase A was 90% 20 mM ammonium acetate and 10% acetonitrile, mobile phase B was acetonitrile; gradient elution from 90% B to 70% B over 10 minutes, and from 70% B to 50% B over 20 minutes. The flow rate was set to 0.5 mL/min. The mass spectrometer was operated in ESI negative ion mode.

### LC-MS analysis for EcYbiW activity determination

The LC-MS analysis experiment for EcYbiW activity determination was performed using the same method as that for LpYbiW activity determination.

### LC-MS analysis of LpFsaA coupled with LpYbiW activity

A reaction mixture (200 µL) containing 10 µM activated LpYbiW, 0.1 mM titanium(III) citrate, and 10 mM 1,5-AG-6P was incubated for 1 hour at room temperature in a glove box, the LpFsaA coupled LpYbiW enzyme activity assay was performed. 10 µM LpFsaA was added to the above reaction system, and after an additional 1 hour of reaction in the glove box, 50 µL of the reaction sample was mixed with 550 µL of 0.73 M sodium acetate (pH 5.0), then mixed with 400 µL of freshly prepared 2,4-dinitrophenylhydrazine (DNPH) solution (20 mg DNPH dissolved in 50 mL methanol), followed by incubation at 50°C for 1 hour. A resulting mixture was centrifuged at 13,000×g for 5 minutes and filtered before LC-MS analysis. Product standards were prepared by treating 3-phosphoglyceraldehyde (5 mM, Sigma-Aldrich) and hydroxyacetone (2 mM, TCI) with sodium acetate (pH 5.0) and freshly prepared DNPH for 1 hour in the same manner.

LC-MS analysis was performed using 20 µL of sample on an Agilent ZORBAX SB-C18 reverse-phase column (4.6 × 250 mm) in ESI negative ion mode. The solvent system consisted of solvent C (deionized ultrapure water containing 0.1% formic acid) and D (chromatographic acetonitrile containing 0.1% formic acid). Liquid phase analysis was performed according to the following program: elution from 15% to 100% D over 15 minutes at a flow rate of 1.0 mL/min, and the detection wavelength was set to 360 nm.

### LC-MS analysis of LpFsaA activity

The activity of LpFsaA catalyzing the aldol condensation reaction of hydroxyacetone and 3-phosphoglyceraldehyde was also determined. A 200 µL reaction system containing 50 mM Tris/HCl, (pH 8.0), 100 mM KCl, 5 mM 3-phosphoglyceraldehyde, 10 mM hydroxyacetone, and 10 µM LpFsaA was incubated at room temperature for 1 hour. In negative controls, 3-phosphoglyceraldehyde, hydroxyacetone, or LpFsaA was omitted. LC-MS analysis was performed using the same sample preparation method and elution conditions as described for the LpYbiW activity determination, using a ZIC-HILIC column.

### Determination of Michaelis-Menten Kinetic Parameters of LpYbiW

The Michaelis-Menten kinetic parameters of LpYbiW were determined by coupling with LpFsaA and EcGldA. The reaction rate of LpYbiW was monitored by measuring NADH consumption at A₃₄₀ₙₘ. The absorbance change of the reaction system at 340 nm was measured every 5 seconds using the cuvette mode of an ultra-micro UV-visible spectrophotometer (Nanophotometer) in a glove box (1 cm light path). These reaction systems consisted of 100 nM activated LpYbiW, 20 mM Tris/HCl, (pH 7.5), 100 mM KCl, 1,5-anhydroglucitol-6-phosphate with varying concentrations, 5 µM LpFsaA, 5 µM EcGldA, and 0.4 mM NADH.

### Construction of expression plasmid for EcYbiW for crystallographic studies

The *E. coli* gene encoding YbiW (EcYbiW, Uniprot accession No.: P75793) was amplified by PCR using a primer pair 5F/5R and inserted into the NdeI site of the pACYC vector. Site-directed mutagenesis of plasmid pACYC-EcYbiW was then performed using a primer pair 6F/6R to obtain plasmid pACYC-EcYbiW (E114A, E115A, and K117A, indicating that amino acids at positions 114, 115, and 117 were mutated from glutamic acid (E) and lysine (K) to alanine (A), respectively) in order to express a surface entropy reduction mutant of EcYbiW and increase the success rate of protein crystallization screening.

### Expression and purification of EcYbiW for crystallographic studies

The plasmid pACYC-EcYbiW (E114A, E115A, and K117A) was transformed into *E. coli* BL21(DE3) cells, and positive clones were selected using LB agar plates containing 25 µg/mL chloramphenicol. A single colony was cultured overnight in 4 mL of LB medium, then transferred to 1 L of fresh LB medium and grown at 37°C, 220 rpm until OD₆₀₀ reached approximately 0.8. Expression was induced with 0.3 mM IPTG at 18°C for 16 hours. Cells from the 1 L culture were collected by centrifugation (8000×g, 10 minutes) and resuspended in 40 mL of lysis buffer (50 mM Tris/HCl, pH 8.0, 100 mM KCl, 0.03% Triton X-100). Cells were lysed by sonication, and insoluble cell debris was removed by centrifugation at 10,000×g for 10 minutes at 4°C. The supernatant was filtered through a 0.45 µm filter and loaded onto a 10 mL TALON Co²⁺ column, then washed with 10 times column volumes of buffer A (20 mM Tris/HCl, pH 7.5, 200 mM KCl, and 5 mM BME), and eluted with 5 times column volumes of buffer A containing 150 mM imidazole. The eluate was dialyzed against 2 L of buffer B (20 mM Tris/HCl, pH 7.5, 5 mM BME) for 3 hours, then loaded onto a 10 mL DEAE column and eluted with a linear gradient of 0 to 500 mM KCl in buffer B over 30 times column volumes. Fractions containing EcYbiW were collected and concentrated by ultrafiltration to approximately 5 mL. The protein solution was then injected into a Superdex 200 gel filtration column (300 mL) and eluted with buffer C (20 mM Tris/HCl, pH 7.5, 100 mM KCl, 1 mM DTT). The eluate from the gel filtration column was re-concentrated to 13.3 mg/mL for crystallographic studies.

### Crystallization, data collection, and structure determination of EcYbiW

Initial screening for EcYbiW crystals was performed using the sitting-drop vapor diffusion method in a 96-well format with an automated liquid handling robot system (Gryphon, Art Robbins). Screening was carried out at 291 K using various crystal screening kits from Hampton Research and Molecular Dimensions. After further optimization using the hanging-drop vapor diffusion method in 24-well plates, crystals suitable for single-crystal X-ray diffraction studies were obtained. The optimal conditions for producing block-like crystals of EcYbiW were 0.2 M NaCl, 0.1 M Tris, pH 8.0, 25% PEG3350 plus 10 mM 1,5-AG-6P, with 6.7 mg/mL EcYbiW protein solution. Crystals were cryoprotected using a crystallization solution containing 15% glycerol and flash-cooled in liquid nitrogen. Diffraction data were collected and processed at beamline BL10U2 of the Shanghai Synchrotron Radiation Facility (SSRF) to a resolution of 2.65 Å. A crystal structure model created with the website PHYRE2 was used for molecular replacement in the PHENIX software. The structure was manually built according to the electron density using Coot software, further refined in PHENIX, and then deposited in the RCSB Protein Data Bank (accession code: 8ID7). The appendix table contains data collection and final refinement statistics (Table 1). All structure Fig.s were generated using UCSF Chimera (Fig. 4A and Fig. 4B).

### Identification of the active sitecenter of 1,5-anhydroglucitol-6-phosphate isomerase

The obtained PDB data of the EcYbiW complex with 1,5-AG-6P (with a resolution of 2.65 Å) were displayed using UCSF Chimera software. Focusing on the substrate 1,5-AG-6P, amino acid residues within 10 Å of the substrate center were selected for display. Based on the hydrogen bond distances between the substrate and amino acid residues, key amino acid residues in the active center of 1,5-anhydroglucitol-6-phosphate isomerase that bind to the substrate were identified. In combination with the catalytic mechanism of glycine radical 1,2-lyases described in the literature, the key amino acid residues of the active center of 1,5-anhydroglucitol-6-phosphate isomerase were determined.

### Identification of isozymes of 1,5-anhydroglucitol-6-phosphate isomerase

To identify isozymes of 1,5-anhydroglucitol-6-phosphate isomerase in the glycine radical enzyme (GRE) family, a sequence similarity network (SSN) of 25,347 unique sequences from InterPro family IPR004184 was constructed using the web-based Enzyme Function Initiative-Enzyme Similarity Tool (EFI-EST). The alignment score threshold was set to 250, the minimum sequence length was set to 650, and an 80% similarity representative node network (RepNode network) was displayed using Cytoscape v3.519. Under these settings, previously characterized GREs with different catalytic activities were separated into distinct clusters.

Sequences with 80% or higher similarity were represented by a single node, and sequences were folded to reduce the total number of nodes, making the less complex network easier to load in Cytoscape. For the cluster formed by YbiW, one representative sequence was selected from each node based on different nodes. Using the crystal structure of the complex formed by EcYbiW and the substrate, amino acid residues interacting with the substrate were used as conserved sites for multiple sequence alignment. Sequences that simultaneously contained these conserved sites were retained. Further analysis of neighboring genes of these sequences was performed, and sequences whose gene cluster contained an aldolase were retained. Finally, representative sequences were selected.

### Results, Analysis, and Discussion

During bioinformatics analysis of glycine radical enzyme (GRE) sequences in the UniProt database, the inventors of the present disclosure noted an uncharacterized GRE (YbiW) present in a gene cluster involved in sugar metabolism. In addition to YbiW and its activase (YbiY), this gene cluster also contains an aldolase (FsaA). This aldolase catalyzes the formation of corresponding 2-keto-hexose-6-phosphates from hydroxyacetone, 1,3-dihydroxyacetone, and glyceraldehyde-3-phosphate. Accordingly, the inventors of the present disclosure speculated that this GRE is involved in sugar metabolism and that its substrate might be a hexose phosphate. Based on molecular docking results, the inventors of the present disclosure hypothesized that the substrate of YbiW is 1,5-anhydroglucitol-6-phosphate (1,5-AG-6P) and proposed a novel radical-dependent glycolytic pathway. In this pathway, 1,5-AG-6P is catalyzed by YbiW to undergo C-O bond cleavage, generating 1-deoxyfructose-6-phosphate. This product is further cleaved by the aldolase FsaA to produce hydroxyacetone and glyceraldehyde-3-phosphate (Fig. 1A).

To prove the inventors' hypothesis, the inventors selected the ybiW, ybiY, and fsaA genes from *Lactiplantibacillus plantarum*, subsequently heterologously expressed them in *E. coli* BL21(DE3) cells, and characterized the activity of these proteins.

The inventors reconstituted the [Fe-S]²⁺ cluster of the activase LpYbiY and characterized it. Anaerobic reconstitution of the [4Fe-4S] cluster resulted in 1.54 ± 0.12 Fe and 2.01 ± 0.08 S per monomer (a radical SAM domain, theoretical maximum of 4Fe and 4S for a [4Fe-4S] cluster) and a typical UV-visible spectrum for a [4Fe-4S] protein, with a molar extinction coefficient ε₄₁₀ₙₘ of 8.85 mM⁻¹ cm⁻¹ (Fig. 7B). Since the ε₄₁₀ₙₘ for each [4Fe-4S] cluster is about 15 mM⁻¹ cm⁻¹, the inventors estimated that each monomer contains approximately 0.59 [4Fe-4S], which is generally consistent with the measured Fe and S contents (Fig. 7A). LC-MS results showed that, like other radical SAM enzymes, LpYbiY catalyzed the cleavage of SAM in the presence of the reducing agent titanium(III) citrate to form 5'-deoxyadenosine (Fig. 7C to Fig. 7D). EPR spectroscopy showed that incubation of LpYbiW, LpYbiY, SAM, and Ti(III) generated a radical, which was quantified as 0.017 Gly•/dimer (Fig. 2A).

To determine the activity of LpYbiW, activated LpYbiW was incubated with 1,5-AG-6P and analyzed by LC-MS (Fig. 2B to Fig. 2D). A new peak with m/z (-) = 243.0 at t_{R} = 18.40 min was observed in the complete reaction mixture. In the activity assay of LpFsaA catalyzing the aldol condensation reaction of hydroxyacetone and glyceraldehyde-3-phosphate, a new peak (m/z (-) = 243.0, t_{R} = 18.40 min) was also generated in the complete reaction mixture (Fig. 9A, Fig. 9C). The Michaelis-Menten kinetic parameters of LpYbiW were also determined (Fig. 10, k_{cat} = 43.38 ± 2.96 s⁻¹/LpYbiW, K_{M} = 36.52 ± 6.66 mM). By coupling LpFsaA with LpYbiW for the reaction, LC-MS analysis detected the production of the corresponding products, hydroxyacetone and glyceraldehyde-3-phosphate (Fig. 2E to Fig. 2H). These data indicated that LpYbiW catalyzes the C-O bond cleavage of 1,5-AG-6P to generate 1-deoxyfructose-6-phosphate (1-deoxy-F6P).

According to LC-MS analysis results, a new peak with m/z (-) = 243.0 at t_{R} = 18.40 min (Fig. 18A) was generated in the complete reaction mixture. The mass spectrum corresponding to this new peak was shown in Fig. 18B, and the mass spectrum corresponding to the substrate was shown in Fig. 18C. The LC-MS results indicated that EcYbiW possesses 1,5-anhydroglucitol-6-phosphate isomerase activity.

To further investigate the catalytic mechanism of YbiW, the inventors determined the crystal structure of the EcYbiW complex with 1,5-AG-6P (with a resolution of 2.65 Å). YbiW contains one monomer per asymmetric unit. Each monomer exhibits the typical β/α barrel fold common to other GREs (Fig. 4A), as well as a radical-like conformation with Gly• and Cys• loops. From this crystal structure, the inventors could see that the sulfur atom of the Cys• residue Cys441 is adjacent to the hydrogen atom at the C2 position of the substrate, with a distance of 3.4 Å, which is consistent with a catalytic mechanism involving "abstraction of the C2 hydrogen atom by Cys•". The 2-OH group of substrate 1,5-AG-6P forms a hydrogen bond with Glu443; the phosphate group coordinates with His165, His334, and Arg453 of YbiW; and the 1-O atom forms a hydrogen bond with His334, and the 3-OH forms a hydrogen bond with Ser662 (Fig. 4C). Glu443 is involved in the deprotonation of the substrate 2-OH, which is consistent with the role of a base in the catalytic mechanism of GRE 1,2-elimination enzymes.

Based on the crystal structure of YbiW, the inventors proposed the following catalytic mechanism for YbiW. Similar to other GRE 1,2-elimination enzymes, Cys441• abstracts the H atom from the C2 position of the substrate 1,5-AG-6P, generating a substrate radical at the C2 position. Then, Glu443 deprotonates the 2-OH of the substrate, and the substrate radical translocates to the C1 position, followed by C-O bond cleavage to form a product radical. The product radical then abstracts the H atom that Cys441 abstracted from the substrate, thereby generating the product 1-deoxy-F6P and regenerating Cys441• (Fig. 4E).

Based on the crystal structure of the complex formed by EcYbiW and the substrate 1,5-anhydroglucitol-6-phosphate, and referring to the hydrogen bond distances formed between the substrate and amino acid residues, the key amino acid residues in the active center of 1,5-anhydroglucitol-6-phosphate isomerase that bind to the substrate were identified. In combination with the catalytic mechanism of glycine radical 1,2-lyases described in the literature, the key amino acid residues of the active center of 1,5-anhydroglucitol-6-phosphate isomerase were determined. In EcYbiW (SEQ ID NO:1), these amino acid residues are H165, H282, S283, H334, C441, E443, R453, T455, L562, S662, I664, and G786.

According to SSN (sequence similarity network) data analysis, the YbiW cluster showed a total of 207 nodes and 2,333 sequences. The inventors selected one representative sequence from each node. Using the crystal structure of EcYbiW (Uniprot accession No.: P75793), the amino acid residues interacting with the substrate (with reference to SEQ ID NO:1: H165, H282, S283, H334, C441, E443, R453, T455, L562, S662, I664, and G786) were used as conserved sites for multiple sequence alignment. Sequences that simultaneously contained these conserved sites were retained, resulting in the screening of 173 isozyme sequences. Further analysis of the neighboring genes of these 173 sequences was performed, and sequences whose gene cluster contained the aldolase FsaA were retained. Finally, a total of 95 representative sequences were listed (Fig. 14 and Fig. 15). Table 2 shows the accession numbers, strain sources, and amino acid sequence numbers of the 95 1,5-anhydroglucitol-6-phosphate isomerase isozymes found in the Uniprot database.

### Example 2: Identification, characterization, and testing of the PflD-related pathway

### Experimental Materials

Tryptone and yeast extract used to prepare LB medium were purchased from Oxoid Limited (Hampshire, UK). Ultrapure deionized water from a Millipore Direct-Q system was used. TALON resin was purchased from Clontech Laboratories Inc. (California, USA). 1,5-Anhydromannitol (1,5-AM) and 1,5-anhydromannitol-6-phosphate (1,5-AM-6P) were chemically synthesized by Tianjin Heowns Biochemical Technology Co., Ltd. Direct-Q oligonucleotide primers were synthesized by Beijing Tsingke Biotech Co., Ltd. All protein purification chromatography experiments were performed on ÄKTA pure FPLC system (GE Healthcare, USA). Anaerobic experiments were performed in a Lab2000 glove box (Etelux) purged with N₂ (with an oxygen concentration <5 ppm).

### Gene synthesis and cloning

EcPflD (Uniprot accession No. P32674), EcPflC (Uniprot accession No. P32675), EcFsaB (Uniprot accession No. P32669), and EcGldA (Uniprot accession No. P0A9S5) were amplified from the *E. coli* MG1655 genome using primer pairs 1F/1R, 2F/2R, 3F/3R, and 4F/4R, respectively (Table 3). EcPflD, EcFsaB, and EcGldA were inserted into the SspI site of the HT plasmid (an optimized pET28 vector) to express proteins with an N-terminal His₆ tag. EcPflC was inserted into the SspI site of the HMT vector to express a protein with an N-terminal His₆ tag and maltose-binding protein.

For biochemical characterization purposes, gene fragments of SdPflD (NCBI accession No. OCX05109.1) and SdPflC (NCBI accession No. OCX05103.1) were synthesized by Beijing Tsingke Biotech Co., Ltd. SdPflD was inserted into the SspI site of the HT plasmid (an optimized pET28 vector) to express a protein with an N-terminal His₆ tag. SdPflC was inserted into the NdeI site of the pACYC-MBP vector to express a protein with an N-terminal His₆ tag and MBP.

### Expression and purification of EcPflD, EcPflC, EcFsaB, and EcGldA

The EcPflD, EcPflC, EcFsaB, and EcGldA were plasmidized and transformed into *E. coli* BL21(DE3) cells for expression of corresponding proteins. LB agar plates containing 50 µg/mL kanamycin were used to select positive clones. Cells were cultured overnight in 4 mL of LB medium, then transferred to fresh LB medium (typically 1 L medium in a 2.6 L flask) and grown at 37°C in a shaking incubator at 220 rpm. When the OD₆₀₀ reached approximately 0.8, the temperature was lowered to 18°C, and isopropyl β-D-1-thiogalactopyranoside (IPTG) was added to a final concentration of 0.3 mM to induce production of the target protein. After 16-20 hours, cells were harvested by centrifugation (8000×g, 10 min, 4°C). The collected cells were resuspended in 40 mL of lysis buffer (50 mM Tris/HCl, pH 8.0, 100 mM KCl, 1 mM phenylmethylsulfonyl fluoride (PMSF), 0.2 mg/mL lysozyme, 0.03% Triton X-100, and 0.02 mg/mL DNase I) and frozen in a refrigerator at -80°C.

The frozen cells were thawed and incubated at room temperature (RT, 25°C) for 20 minutes, during which cell lysis occurred. 5 mM β-Mercaptoethanol (BME) was added, and nucleic acids were precipitated with 1% streptomycin sulfate. Cell debris was removed by centrifugation at 10,000×g for 10 minutes at 4°C. For EcPflD, EcFsaB, and EcGldA, the supernatant was filtered through a 0.22 µm filter and loaded onto a 5 mL TALON Co²⁺ column (Takara Bio USA, Inc.) pre-equilibrated with Buffer A (20 mM Tris/HCl, pH 7.5, 200 mM KCl, and 5 mM BME). After washing with 10 times column volumes of Buffer A to remove contaminating proteins, the target proteins were eluted with 5 times column volumes of Buffer A containing 150 mM imidazole. The eluted protein (about 20 mL) was dialyzed against 2 L of Buffer A for 3 hours at 4°C, concentrated, aliquoted, frozen in liquid nitrogen, and stored at -80°C. For EcPflC, the supernatant was filtered and loaded onto a column packed with 10 mL of amylose (New England Biolabs, Massachusetts, U.S.A.). After washing with 10 times column volumes of Buffer A to remove contaminating proteins, the target protein was eluted with Buffer A containing 10 mM maltose.

Purified proteins were detected by SDS-PAGE using commercial gels (SurePAGE, Bis-Tris, 4-20%). Protein concentrations were calculated by measuring absorbance at 280 nm using an ultra-micro UV-Vis spectrophotometer (Hangzhou Miu Instruments Co., Ltd.). [EcPflD (ε₂₈₀ = 74,720 M⁻¹cm⁻¹), MBP-EcPflC (ε₂₈₀ = 88,810 M⁻¹cm⁻¹), EcFsaB (ε₂₈₀ = 18,450 M⁻¹cm⁻¹)], EcGldA (ε₂₈₀ = 32,890 M⁻¹cm⁻¹)].

### Expression and purification of SdPflD and SdPflC for biochemical characterization

Protein expression and purification methods and procedures, SDS-PAGE detection methods, and concentration determination methods were the same as those for LpYbiW and LpYbiY described above. [SdPflD (ε₂₈₀ = 86,070 M⁻¹cm⁻¹), MBP-SdPflC (ε₂₈₀ = 107,260 M⁻¹cm⁻¹)].

### Reconstitution and characterization of the EcPflC prosthetic [Fe-S] cluster

Sequence alignment indicated that EcPflC contains only one [4Fe-4S] cluster in the radical SAM domain, with a theoretical maximum of 4Fe and 4S per monomer. The EcPflC protein solution was degassed with argon to remove oxygy, then transferred into a glove box. To the degassed protein solution, Tris/HCl with a final concentration of 100 mM, (pH 7.5), 10 mM DTT, and 4 equivalents of ferrous ammonium sulfate and sodium sulfide were added, and a resulting mixture was incubated overnight at 4°C in a metal bath (Dry Bath H2O3-100C; Coyote Bioscience, Beijing, China). Subsequently, 4 equivalents of EDTA solution were added, a resulting mixture was repeated concentration using centrifugal filter units (1.5 mL YM-30 Amicon; Millipore) and dilution with a buffer (20 mM Tris/HCl, pH 7.5, and 100 mM KCl) to exchange the buffer.

Fe contents of unconstituted and reconstituted EcPflC were quantified using ferrozine [3-(2-pyridyl)-5,6-diphenyl-1,2,4-triazine-p,p'-disulfonic acid monosodium salt]. A standard curve for Fe was established using AAS iron standards in a range of 0-600 µM. Sulfur contents of unconstituted and reconstituted EcPflC were determined by measuring the absorbance of methylene blue formed from the reaction with N,N-dimethyl-p-phenylenediamine dihydrochloride (DPD). A standard curve for sulfur was established using Na₂S in a range of 0-600 µM.

UV-Vis absorption spectra of EcPflC in a range of 200-800 nm were measured using a NanoPhotometer NP80 Mobile (Germany). Unconstituted and reconstituted EcPflC were diluted to 10 µM with a buffer containing 20 mM Tris-HCl, (pH 7.5), and 100 mM KCl, and transferred to a septum-sealed anaerobic cuvette for measurement. To a reconstituted EcPflC solution, 10 equivalents of titanium(III) citrate were added and a resulting mixure was incubated for 10 minutes to determine its reduced form.

### LC-MS analysis of SAM cleavage catalyzed by EcPflC

A reaction system (500 µL) containing 20 mM Tris/HCl, (pH 7.5), 100 mM KCl, 200 µM titanium(III) citrate, 20 µM reconstituted EcPflC, and 1 mM SAM was incubated overnight at room temperature in a glove box. The negative control lacked titanium(III) citrate. Formic acid (with a final concentration of 5% v/v) was added to quench the reaction, and a resulting mixture was incubated in a boiling water bath for 1 minute to denature the protein. Precipitated protein was removed by centrifugation at 14,000×g for 15 minutes. The supernatant was filtered using a 0.22 µm PES membrane and subjected to LC-MS analysis. The method was as follows: 20 µL of sample was injected onto an Agilent 6420 Triple Quadrupole LC/MS instrument (Agilent Technologies) equipped with a C18 reversed-phase column. UV absorbance was monitored at 257 nm. The mobile phase consisted of water (A) and acetonitrile (B), with a linear gradient elution from 0-16% B over 30 minutes at a flow rate of 0.5 mL/min. Commercial 5'-dA was used as a standard, and the formation of SAM cleavage product catalyzed by EcPflC was verified by mass spectrometry.

### Generation of the EcPflD radical detected by electron paramagnetic resonance (EPR) spectroscopy

The EcPflD glycine radical was characterized using continuous-wave X-band electron paramagnetic resonance (EPR) spectroscopy. A reaction system (200 µL) containing 20 mM Tris/HCl, (pH 7.5), 100 mM KCl, 100 µM titanium(III) citrate, 1 mM SAM, 80 µM reconstituted EcPflC, and 40 µM EcPflD was incubated for 15 minutes at room temperature in a glove box. Glycerol (10%) was added, and a resulting mixture was transferred to an EPR tube with an outer diameter of 4 mm and a length of 8 inches (Wilmad Lab Glass, 734-LPV-7). The tube was sealed with a rubber stopper, removed from the glove box, and frozen in liquid nitrogen prior to EPR analysis. Experimental spectra of the glycyl radical were modeled using Bruker Xepr spin fitting to obtain g-values, hyperfine coupling constants, and linewidths. Double integration of the simulated spectra was used to measure the spin concentration. EPR spectra were obtained by accumulating 30 scans under the following conditions: temperature, 90 K; center field, 3370.00 G; sweep width, 200 G; microwave power, 10 µW; microwave frequency, 9.43 MHz; modulation amplitude, 0.5 mT; modulation frequency, 100 kHz; time constant, 20.48 ms; conversion time, 25 ms; sweep time, 20 s; receiver gain, 43 dB.

### LC-MS analysis for EcPflD activity assay

A 200 µL reaction mixture containing 10 µM activated EcPflD, 0.1 mM titanium(III) citrate, and 10 mM 1,5-AM-6P was incubated for 1 hour at room temperature in a glove box. Negative controls omitted 1,5-AM-6P, SAM, or activated EcPflD. Protein was precipitated by adding 200 µL of acetonitrile, a resulting precipitate was removed by centrifugation, and the supernatant was filtered through a 0.22 µm PES membrane for LC-MS analysis.

LC-MS analysis was performed on an Agilent 6420 Triple Quadrupole LC/MS instrument (Agilent Technologies). The drying gas was maintained at a temperature of 300°C, with a flow rate of 9 L/min, and the nebulizer pressure was 15 psi. LC-MS analysis was performed using a ZIC-HILIC column (5 mm, 200 Å, 150×4.6 mm; Merck). HPLC conditions were as follows: mobile phase A = 90% 20 mM ammonium acetate + 10% acetonitrile, mobile phase B = acetonitrile; gradient elution from 90% B to 70% B over 10 minutes, then from 70% B to 50% B over 20 minutes. Flow rate was set to 0.5 mL/min. The mass spectrometer was operated in ESI negative ion mode.

### LC-MS analysis for SdPflD activity assay

The LC-MS analysis method for species SdPflD of *Streptococcus dysgalactiae subsp.Equisimilis* activity assay was the same as that for EcPflD.

### LC-MS analysis for EcFsaB-coupled EcPflD activity

A reaction mixture (200 µL) containing 10 µM activated EcPflD, 0.1 mM titanium(III) citrate, and 10 mM 1,5-AM-6P was incubated for 1 hour at room temperature in a glove box, enzymatic activity of EcFsaB-coupled EcPflD was measured. 10 µM EcFsaB was added to the above reaction mixture, and a resulting mixture was further incubated for another 1 hour in the glove box. Then, 50 µL of the reaction sample was mixed with 550 µL of 0.73 M sodium acetate (pH 5.0) and then with 400 µL of freshly prepared 2,4-dinitrophenylhydrazine (DNPH) solution (20 mg dissolved in 50 mL methanol), a resulting mixture was further incubated at 50°C for 1 hour. a resulting mixture was centrifuged at 13,000×g for 5 minutes and filtered before LC-MS analysis. 3-Phosphoglyceraldehyde (5 mM, Sigma-Aldrich) and hydroxyacetone (2 mM, TCI) standards were used as positive controls, which were similarly treated by sodium acetate (pH 5.0) and freshly prepared DNPH solution for 1 hour.

LC-MS analysis was performed using an Agilent ZORBAX SB-C18 reversed-phase column (4.6×250 mm) with 20 µL samples in ESI negative ion mode. The solvent system consisted of solvent C (deionized ultrapure water containing 0.1% formic acid) and D (chromatographic acetonitrile containing 0.1% formic acid). The LC analysis was performed with the following program: elution from 15% D to 100% D over 15 minutes at a flow rate of 1.0 mL/min, and the detection wavelength was set to 360 nm.

### Spectrophotometric determination of EcPflD, EcFsaB, and EcGldA coupling

A reaction mixture containing 20 mM Tris/HCl, (pH 7.5), 100 mM KCl, 10 mM 1,5-AM-6P, 40 nM activated EcPflD, 5 µM EcFsaB, 5 µM EcGldA, and 0.4 mM NADH was incubated in a 1 cm Eppendorf cuvette in a glove box at room temperature. Absorbance at 340 nm was monitored every 5 seconds using a cuvette mode of an ultra-micro UV-Vis spectrophotometer (Hangzhou Miu Instruments Co., Ltd.) inside the glove box. Negative controls were prepared by omitting 1,5-AM-6P, SAM, or EcPflD.

### LC-MS analysis for EcFsaB activity

EcFsaB activity for catalyzing the aldol condensation of hydroxyacetone and 3-phosphoglyceraldehyde was also assayed. A 200 µL reaction system containing 50 mM Tris/HCl, (pH 8.0), 100 mM KCl, 5 mM 3-phosphoglyceraldehyde, 10 mM hydroxyacetone, and 10 µM EcFsaB was incubated for 1 hour at room temperature. Negative controls omitted 3-phosphoglyceraldehyde, hydroxyacetone, or EcFsaB. LC-MS analysis was performed using the same sample preparation method and elution conditions as described for EcPflD activity assay, using a ZIC-HILIC column.

### Determination of Michaelis-Menten kinetic parameters of EcPflD

The reaction system containing 20 mM Tris/HCl, (pH 7.5), 100 mM KCl, 40 nM activated EcPflD and 1,5-AM-6P with various substrate concentrations, 2 µM EcFsaB, 2 µM EcGldA, and 0.4 mM NADH was measured using a cuvette mode of an ultra-micro UV-Vis spectrophotometer (Hangzhou Miu Instruments Co., Ltd.) in a 1 cm Eppendorf cuvette inside a glove box. The decrease in A₃₄₀ₙₘ was monitored at 5-second intervals.

### Construction of SdPflD expression plasmid for crystallography studies

An *E. coli* codon-optimized DNA fragment encoding PflD from *Streptococcus dysgalactiae subsp. equisimilis* (SdPflD, NCBI accession No. OCX05109.1) was synthesized by Beijing Tsingke Biotech Co., Ltd. and inserted into the SspI site of the HT vector.

### Expression and purification of SdPflD for crystallography studies

### Spectrophotometric determination of EcPflD, EcFsaB, and EcGldA coupling

For crystallographic studies of SdPflD, *E. coli* BL21(DE3) cells carrying the plasmid HT-SdPflD were cultured in 2 L of LB medium containing 50 µg/mL kanamycin. Cells were harvested and lysed using the same method as for EcYbiW. The supernatant of the cell lysate was purified using a 10 mL TALON Co²⁺ column. The eluate was pre-mixed with recombinant His₆-tagged TEV protease (with a molar ratio of TEV protease to SdPflD of approximately 1:10) and dialyzed overnight against 2 L of buffer A (20 mM Tris/HCl, pH 7.5, 200 mM KCl, and 5 mM BME) containing 5 mM BME. The dialyzed sample was loaded onto a 10 mL TALON Co²⁺ column to retain the TEV protease and His₆ tag. The flow-through was collected and dialyzed against 2 L of buffer B (20 mM Tris/HCl, pH 7.5, 5 mM BME), and then loaded onto a 10 mL DEAE column as described above. The main peak containing SdPflD was concentrated and purified using a Superdex 200 gel filtration column. The sample from the gel filtration column was concentrated to a final concentration of 9.9 mg/mL.

### Crystallization, data collection, and structure determination of SdPflD

Initial screening for SdPflD crystals was performed using the sitting-drop vapor diffusion method in a 96-well format with an automated liquid handling robot system (Gryphon, Art Robbins). Screening was carried out at 291 K using various crystal screening kits from Hampton Research and Molecular Dimensions. After further optimization using the hanging-drop vapor diffusion method in 24-well plates, crystals suitable for single-crystal X-ray diffraction studies were obtained. The optimal conditions for producing block-like crystals of SdPflD were 5.0 mg/mL SdPflD protein solution, 1.2 M sodium citrate, 0.1 M sodium HEPES, pH 8.0, and 10 mM 1,5-AM-6P. The block-like crystals were cryoprotected with 10% glycerol. Diffraction data were collected and processed at beamline BL10U2 of the Shanghai Synchrotron Radiation Facility (SSRF) to a resolution of 2.34 Å. A crystal structure model created with the website PHYRE2 was used for molecular replacement in the PHENIX software. The structure was manually built according to the electron density using Coot software, further refined in PHENIX, and then deposited in the RCSB Protein Data Bank (accession code: 8ID0). The appendix table contains data collection and final refinement statistics (Table 1). All structure drawings were generated using UCSF Chimera (Fig. 4B and Fig. 4D).

### Identification of the active center of 1,5-anhydromannitol-6-phosphate isomerase

The obtained PDB data of the SdPflD complex with 1,5-AM-6P (with a resolution of 2.34 Å) were displayed using UCSF Chimera software. Focusing on the substrate 1,5-AM-6P, amino acid residues within 10 Å of the substrate center were selected for display. Based on the hydrogen bond distances between the substrate and amino acid residues, key amino acid residues in the active center of 1,5-anhydromannitol-6-phosphate isomerase that bind to the substrate were identified. In combination with the catalytic mechanism of glycine radical 1,2-lyases described in the literature, the key amino acid residues of the active center of 1,5-anhydromannitol-6-phosphate isomerase were determined.

### Identification of isozymes of 1,5-anhydromannitol-6-phosphate isomerase

To identify isozymes of 1,5-anhydromannitol-6-phosphate isomerase in the glycine radical enzyme (GRE) family, a sequence similarity network (SSN) of 25,347 unique sequences from InterPro family IPR004184 was constructed using the web-based Enzyme Function Initiative Enzyme Similarity Tool (EFI-EST). The alignment score threshold was set to 250, the minimum sequence length was set to 650, and an 80% similarity representative node network (RepNode network) was displayed using Cytoscape v3.519. Under these settings, previously characterized GREs with different catalytic activities were separated into distinct clusters.

Sequences with 80% or higher similarity were represented by a single node, and sequences were folded to reduce the total number of nodes, making the less complex network easier to load in Cytoscape. For the cluster formed by PflD, one representative sequence was selected from each node based on different nodes. Using the crystal structure of the complex formed by SdPflD and the substrate, amino acid residues interacting with the substrate were used as conserved sites for multiple sequence alignment. Sequences that simultaneously contained these conserved sites were retained. Further analysis of neighboring genes of these sequences was performed, and sequences whose gene cluster contained an aldolase were retained. Finally, representative sequences were selected.

### Results, analysis, and discussion

A gene cluster involved in sugar metabolism is present in the *E. coli* MG1655 genome. Through bioinformatics analysis of this gene cluster, the inventors noted an uncharacterized GRE (PflD) involved therein. In addition to PflD and the S-adenosylmethionine radical enzyme family glycine radical enzyme activase (PflC), this gene cluster also contains a 1-deoxyfructose-6-phosphate aldolase (FsaB), a hydroxyacetone reductase (GldA), and a complete phosphoenolpyruvate-dependent phosphotransferase system (PTS), which includes PtsA, FrwB, FrwC, and FrwD. The function of the PTS is to transfer a phosphate group from phosphoenolpyruvate (PEP) to sugar coming in cells via a series of sequential steps involving different PTS components. The PTS consists of cytoplasmic components lacking sugar specificity and membrane-associated enzymes that are specific for up to a few sugars. This PTS transport complex may be involved in the uptake of hexoses and the phosphorylation of substrates. The aldolase FsaB may catalyze the formation of corresponding 2-keto-hexose-6-phosphates from hydroxyacetone, 1,3-dihydroxyacetone, and glyceraldehyde-3-phosphate. GldA catalyzes the dehydrogenation of glycerol or propylene glycol to generate 1,3-dihydroxyacetone or hydroxyacetone. Accordingly, the inventors speculated that this GRE is involved in sugar metabolism and that its substrate might be a hexose phosphate. Based on molecular docking results, the inventors hypothesized that the substrate of PflD is 1,5-anhydromannitol-6-phosphate (1,5-AM-6P) and proposed a novel radical-dependent glycolytic pathway. In this pathway, 1,5-AM-6P is catalyzed by PflD to undergo C-O bond cleavage, generating 1-deoxy-F6P. This product is further cleaved by the aldolase FsaB to produce hydroxyacetone and glyceraldehyde-3-phosphate. Hydroxyacetone is reduced by the hydroxyacetone reductase GldA to generate 1,2-propanediol (Fig. 1B).

To prove the inventors' hypothesis, the inventors selected the pflD, pflC, fsaB, and gldA genes from *E. coli* MG1655, subsequently heterologously expressed them in *E. coli* BL21(DE3) cells, and characterized the activity of these proteins.

The inventors reconstituted the [Fe-S]²⁺ cluster of the activase EcPflC and characterized it. Anaerobic reconstitution of the [4Fe-4S] cluster resulted in 2.15 ± 0.12 Fe and 2.30 ± 0.07 S per monomer (a radical SAM domain, theoretical maximum of 4Fe and 4S for a [4Fe-4S] cluster) and a typical UV-visible spectrum for a [4Fe-4S] protein, with a molar extinction coefficient ε₄₁₀ₙₘ of 9.45 mM⁻¹ cm⁻¹. Since the ε₄₁₀ₙₘ for each [4Fe-4S] cluster is about 15 mM⁻¹ cm⁻¹, the inventors estimated that each monomer contains approximately 0.63 [4Fe-4S], which is generally consistent with the measured Fe and S contents (Fig. 8A and Fig. 8B). LC-MS results showed that, like other radical SAM enzymes, EcPflC catalyzed the cleavage of SAM in the presence of the reducing agent titanium(III) citrate to form 5'-deoxyadenosine (Fig. 8C and Fig. 8D). EPR spectroscopy showed that incubation of EcPflD, EcPflC, SAM, and Ti(III) generated a radical, which was quantified as 0.060 Gly•/dimer (Fig. 3A).

To determine the activity of EcPflD, activated EcPflD was incubated with 1,5-AM-6P and analyzed by LC-MS. A new peak with m/z (-) = 243.0 at t_{R} = 18.40 min was observed in the complete reaction mixture (Fig. 3B to Fig. 3D). In the activity assay of EcFsaB catalyzing the aldol condensation of hydroxyacetone and glyceraldehyde-3-phosphate, a new peak (m/z (-) = 243.0, t_{R} = 18.40 min) was also generated in the complete reaction mixture (Fig. 9B to Fig. 9D). The Michaelis-Menten kinetic parameters of EcPflD were also determined (Fig. 11, k_{cat} = 33.47 ± 0.79 s⁻¹/EcPflD, K_{M} = 1.33 ± 0.12 mM). By coupling EcPflD with EcFsaB for the reaction, LC-MS analysis detected the production of the corresponding products, hydroxyacetone and glyceraldehyde-3-phosphate (Fig. 3E to Fig. 3H). Using the coupled spectrophotometric assay of EcPflD, EcFsaB, and EcGldA, a decrease in A₃₄₀ₙₘ was observed only in the complete reaction mixture, indicating that substrate 1,5-AM-6P undergoes C-O bond cleavage catalyzed by EcPflD to generate 1-deoxy-F6P, which is then cleaved by EcFsaB to produce hydroxyacetone and glyceraldehyde-3-phosphate; hydroxyacetone is reduced by EcGldA to generate 1,2-propanediol (Fig. 3I).

The pflD gene cluster in *Streptococcus dysgalactiae subsp. equisimilis* was shown in Fig. 19A. According to LC-MS analysis results, a new peak with m/z (-) = 243.0 at t_{R} = 18.40 min (Fig. 19B) was generated in the complete reaction mixture. The mass spectrum corresponding to this new peak was shown in Fig. 19C, and the mass spectrum corresponding to the substrate was shown in Fig. 19D. The LC-MS results indicated that SdPflD possesses 1,5-anhydromannitol-6-phosphate isomerase activity.

To further investigate the catalytic mechanism of PflD, the inventors determined the crystal structure of the SdPflD complex with 1,5-AM-6P (with a resolution of 2.34 Å). PflD contains one monomer per asymmetric unit. Each monomer exhibits the typical β/α barrel fold common to other GREs (Fig. 4B), as well as a radical-like conformation with Gly• and Cys• loops. From this crystal structure, it can be seen that the sulfur atom of the Cys• residue Cys431 is adjacent to the hydrogen atom at the C2 position of the substrate, with a distance of 3.74 Å, which is consistent with a catalytic mechanism involving "abstraction of the C2 hydrogen atom by Cys•". The 2-OH group of substrate 1,5-AM-6P forms a hydrogen bond with Glu433; the phosphate group coordinates with Gln162, Ser277, and Arg323 of PflD; and the 1-O atom forms hydrogen bonds with His169 and Glu445, and the 3-OH forms a hydrogen bond with Ser278 (Fig. 4D). Glu433 is involved in the deprotonation of the substrate 2-OH, consistent with the role of a base in the catalytic mechanism of GRE 1,2-elimination enzymes.

Based on the crystal structure of PflD and its similarity to other GRE 1,2-elimination enzymes, the inventors proposed the following catalytic mechanism for PflD. Cys431• abstracts the H atom from the C2 position of the substrate 1,5-AM-6P, generating a substrate radical at the C2 position. Then, Glu433 deprotonates the 2-OH of the substrate, and the substrate radical translocates to the C1 position, followed by C-O bond cleavage to form a product radical. The product radical then abstracts the H atom that Cys431 abstracted from the substrate, thereby generating the product 1-deoxy-F6P and regenerating Cys431• (Fig. 4F).

Based on the crystal structure of the complex formed by SdPflD and the substrate 1,5-anhydromannitol-6-phosphate, and referring to the hydrogen bond distances formed between the substrate and amino acid residues, the key amino acid residues in the active center of 1,5-anhydromannitol-6-phosphate isomerase that bind to the substrate were identified. In combination with the catalytic mechanism of glycine radical 1,2-lyases described in the literature, the key amino acid residues of the active center of 1,5-anhydromannitol-6-phosphate isomerase were determined. In SdPflD, these amino acid residues are Q162, H169, S277, S278, R323, F331, P335, C431, E433, D445, Y628, V630, and G752.

According to the SSN sequence analysis, the PflD cluster showed a total of 89 nodes and 953 sequences. the inventors selected one representative sequence from each node. Using the crystal structure of SdPflD (NCBI accession number OCX05109.1), the amino acid residues interacting with the substrate (Q162, H169, S277, S278, R323, F331, P335, C431, E433, D445, Y628, V630, and G752) were used as conserved sites for multiple sequence alignment. Sequences that simultaneously contained these conserved sites were retained, resulting in the screening of 77 isozyme sequences. Further analysis of the neighboring genes of these 77 sequences was performed, and sequences whose gene cluster contained the aldolase FsaB were retained. Finally, a total of 47 representative sequences were listed (Fig. 14 and Fig. 16).

### Example 3: Experiments on E. coli utilizing 1,5-anhydroglucitol and 1,5-anhydromannitol

### Gene knockout in E. coli MG1655

The CRISPR-assisted homologous recombination strategy was used to knock out ybiW and pflD in *E. coli* MG1655, respectively. The inventors designed primer pairs 7F/7R and 8F/8R to amplify the upstream and downstream sequences of ybiW, 500 bp at the 5' end and 504 bp at the 3' end, respectively, to generate fragments 1 and 2. Fragments 1 and 2 were assembled using PCR with primers 7F and 8R to generate fragment 3. Using pRed_Cas9_recA plasmid as template, fragments 4 and 5 were obtained by PCR amplification with primer pairs 9F/9R and 10F/10R. Fragments 3, 4, and 5 were Gibson assembled to generate the recombinant pRed_Cas9_recA_ΔybiW plasmid, which contains the guide RNA sequence "GCCTGCCAGAAAGTCTGCGG" (SEQ ID NO:187) designed to introduce Cas9-catalyzed cleavage near the homologous recombination site to increase the rate of desired homologous recombination.

The construction of the recombinant plasmid pRed_Cas9_recA_ΔpflD was similar to that of pRed_Cas9_recA_ΔybiW. Primer pairs 11F/11R and 12F/12R were used to amplify the upstream (500 bp) and downstream (505 bp) sequences of pflD to generate fragments 6 and 7. Similarly, fragment 8 was generated using fragments 6 and 7 as templates and 11F and 12R as primers. Fragments 9 and 10 were amplified by PCR using pRed_Cas9_recA as template with primer pairs 9F/13R and 13F/10R. pRed_Cas9_recA_ΔpflD containing the guide RNA sequence "AAATACCAGAACCCGCGCGG" (SEQ ID NO:188) was constructed using Gibson assembly with fragments 8, 9, and 10. DNA sequencing with primers 14F, 14R, and 15F confirmed the recombinant plasmids pRed_Cas9_recA_ΔybiW and pRed_Cas9_recA_ΔpflD.

To obtain *E. coli* MG1655_ΔybiW and E. coli MG1655_ΔpflD strains, pRed_Cas9_recA_ΔybiW and pRed_Cas9_recA_ΔpflD were transformed into competent cells of *E. coli* MG1655 strain by electroporation, respectively. Positive transformants were selected on LB agar plates containing 50 µg/mL kanamycin in a 30°C incubator. Single colonies were cultured in 5 mL LB medium containing 50 mg/L kanamycin and 2 g/L arabinose at 30°C for 24 hours. Cells were harvested and plated on LB agar plates containing 50 g/L kanamycin and 2 g/L arabinose at 30°C. Single colonies were picked and streaked on LB agar plates and cultured at 37°C to eliminate the recombinant plasmid. Colony PCR verification was performed using primers 16F/16R for ΔybiW and 17F/17R for ΔpflD, theoretically amplifying DNA fragments of approximately 1500 bp. Agarose gel electrophoresis showed successful genomic knockout, which was further confirmed by DNA sequencing using the same primer pairs.

### Anaerobic growth of E. coli strains using 1,5-AG/1,5-AM as the sole carbon source

Single colonies of *E. coli* MG1655 WT, ΔybiW, and ΔpflD strains freshly grown on LB agar plates were inoculated into 5 mL LB medium and cultured in a shaking incubator at 37°C for 4 hours. Cells from 100 µL of culture were pelleted by centrifugation, transferred to anaerobic bottles containing 5 mL anaerobic LB medium, and cultured at 37°C for 6 hours. Cells from 100 µL of culture were collected by centrifugation, transferred to anaerobic bottles containing 5 mL anaerobic M9 medium, and cultured at 37°C for 3 days. 1 mL of cells were harvested, washed three times with glucose-free anaerobic M9 medium, and resuspended in this medium. 100 µL of the cell suspension was transferred to anaerobic bottles, each containing 5 mL of glucose-free M9 medium or the same medium supplemented with 20 mg of sugar (glucose, 1,5-AG, or 1,5-AM). The anaerobic bottles were then placed in an incubator at 37°C for 7 days before photography, and cells were collected for SDS-PAGE gel analysis.

### SDS/PAGE and mass spectrometry for protein identification

Cells were harvested by centrifugation, lysed by boiling in Laemmli loading buffer, and analyzed on 10% SDS/PAGE gels. Prominent protein bands induced in cells cultured with glucose, 1,5-anhydroglucitol, or 1,5-anhydromannitol were excised manually. After in-gel digestion and extraction, the peptide mixture was analyzed by a Fusion Lumos mass spectrometer coupled to an Easy nLC 1200 system (Thermo Fisher Scientific). MS/MS spectra from each LC-MS/MS run were searched against the E. coli protein database (release April 1, 2021) containing 15,862 sequence entries from UniProt using an in-house Proteome Discoverer (version 2.2) search algorithm. Protein identification was performed based on Sequest HT.

### GC analysis of fermentation broth

Wild-type *E. coli* MG1655 was cultured at 37°C for 7 days in different carbon sources, and cells were removed by centrifugation. 200 µL of fermentation broth was aspirated, 800 µL of chromatographic grade absolute ethanol was added, mixed by vortexing, and centrifuged to remove insoluble precipitate. The resulting solution was filtered through a 0.45 µm organic filter membrane, and the filtrate was analyzed by gas chromatography. (R)-1,2-propanediol commercial standard was dissolved in chromatographic grade absolute ethanol.

Gas chromatography (GC) analysis was performed using an Agilent 6820 G1176A gas chromatograph (Agilent Technologies). The GC column used for analysis was an AT^{™}-Aquawax-DA (Alltech) gas chromatography column (30 m × 0.53 mm, 1.0 µm). GC conditions were as follows: carrier gas was high-purity nitrogen, column flow rate was constant at 1.0 mL/min; injector temperature 230°C, detector temperature 240°C; hydrogen flow rate 20 mL/min; air flow rate 200 mL/min. Temperature program was used: initial column temperature 60°C, hold for 2 minutes, ramp to 80°C at 20°C/min, hold for 3 minutes, ramp to 160°C at 20°C/min, hold for 2 minutes; then ramp to 220°C at 15°C/min, hold for 10 minutes.

### Results, analysis, and discussion

Using M9 medium with 1,5-AG as the sole carbon source, *E. coli* MG1655_wild-type and *E. coli* MG1655_ΔybiW strains were cultured anaerobically; glucose was used as the sole carbon source for positive control, and no carbon source was added for negative control. The *E. coli* MG1655_wild-type strain grew when using glucose or 1,5-AG as the carbon source, respectively; whereas the *E. coli* MG1655_ΔybiW strain could only grow using glucose as the sole carbon source (Fig. 5A). Further SDS-PAGE analysis results showed that when the *E. coli* MG1655_wild-type strain used 1,5-AG as the sole carbon source, the corresponding proteins in the YbiW-dependent pathway were induced. Combined with protein mass spectrometry analysis, the induced bands were identified respectively: two about 95 kDa bands contained PtsA and YbiW; an about 42 kDa band was identified as GldA; an about 27 kDa band contained FsaB and FsaA (Fig. 5B).

Similarly, using M9 medium with 1,5-AM as the sole carbon source, *E. coli* MG1655_wild-type and *E. coli* MG1655_ΔpflD strains were cultured anaerobically; glucose was used as the sole carbon source for positive control, and no carbon source was added for negative control. The *E. coli* MG1655_wild-type strain grew when using glucose or 1,5-AM as the carbon source, respectively; whereas the *E. coli* MG1655_ΔpflD strain could only grow using glucose as the sole carbon source (Fig. 5C). Further SDS-PAGE analysis results showed that when the *E. coli* MG1655_wild-type strain used 1,5-AM as the sole carbon source, the corresponding proteins in the PflD-dependent pathway were induced. Combined with protein mass spectrometry analysis, the induced bands were identified respectively: an about 95 kDa band contained PtsA; an about 90 kDa band contained PflD; an about 42 kDa band was identified as GldA; an about 27 kDa band contained FsaB (Fig. 5D). GC analysis results showed that when 1,5-AG or 1,5-AM was used as the sole carbon source, *E. coli* MG1655 produced 1,2-propanediol under anaerobic conditions, with a retention time of 8.85 min (Fig. 13).

The *E. coli* MG1655 genome contains the ybiW and pflD gene clusters (Fig. 5E). Combined with the growth results of strains on different carbon sources, SDS-PAGE analysis, and protein mass spectrometry analysis results, the inventors believe that 1,5-AG can induce the expression of the YbiW and PflD-related gene clusters in *E. coli* MG1655. 1,5-AG is transported into the cell using the PTS system in the PflD gene cluster, inducing high expression of FsaB and GldA in the PflD gene cluster, while also inducing high expression of YbiW and FsaA in the YbiW gene cluster. 1,5-AG is transported into the cell by the PTS system, and the C-6 position of the substrate is phosphorylated to generate 1,5-AG-6P; under the catalysis of YbiW, C-O bond cleavage occurs to generate 1-deoxy-F6P; this product is cleaved by FsaB and FsaA to produce hydroxyacetone and glyceraldehyde-3-phosphate; hydroxyacetone is reduced by EcGldA to generate 1,2-propanediol.

In contrast, 1,5-AM can only induce high expression of PflD-related pathway proteins. The substrate is transported into the cell by the PTS system in the gene cluster, and the C-6 position is phosphorylated to generate 1,5-AM-6P. Under the catalysis of PflD, C-O bond cleavage occurs to generate 1-deoxy-F6P; this product is cleaved by FsaB to produce hydroxyacetone and glyceraldehyde-3-phosphate; hydroxyacetone is reduced by GldA to generate 1,2-propanediol (Fig. 5F).

**Table 1. Data collection and refinement statistics for EcYbiW and SdPflD crystals**

| | **EcYbiW** | **SdPflD** |
|---|---|---|
| Data collection | | |
| Space group | F 2 2 2 | P 62 |

| **Cell size (Å)** | | |
|---|---|---|
| a, b, c (Å) | 116.48Å 207.17Å 208.05Å | 183.91Å 183.91Å 60.02Å |
| α, β, γ (°) | 90.00, 90.00, 90.00 | 90.00, 90.00, 120.00 |
| Resolution (Å) | 46.48 - 2.65 (2.745 - 2.65) | 53.09 - 2.34 (2.424 - 2.34) |
| Rmerge | 0.19 | 0.20 |
| I/σ (I) | 1.55 | 5.34 |
| Completeness (%) | 97.44 (92.74) | 98.79(96.34) |

| **Refinement** | | |
|---|---|---|
| Resolution (Å) | 46.48 - 2.65 (2.745 - 2.65) | 53.09 - 2.34 (2.424 - 2.34) |
| Reflection number | 35659 (3322) | 48712 (4715) |
| Rwork/Rfree | 0.223/0.284 | 0.157/0.193 |

| Atomic number | | |
|---|---|---|
| Protein | 6264 | 6111 |
| Ligand/Ion | 15 | 15 |
| Water | 165 | 564 |

| **Ramachandran plot (%)** | | |
|---|---|---|
| Optimal | 89.21 | 96.62 |
| Additionally permitted | 9.18 | 3.12 |

| **R.M.S. Deviation** | | |
|---|---|---|
| RMSD Length (Å) | 0.009 | 0.007 |
| RMSD Angle (°) | 1.28 | 1.18 |

| | | |
|---|---|---|
| Note: The statistics shown in the parentheses represent the highest resolution enclosure information. | | |

**Table 2. Accession numbers, strain sources, and SEQ ID NOs for 1,5-anhydroglucitol-6-phosphate isomerase isozymes**

| **Accession number** | **Strain source** | **SEQ ID NO:** |
|---|---|---|
| A0A807DR53 | *Lactiplantibacillus plantarum* | 2 |
| A0A0R2FVZ1 | *Lactobacillus selangorensis* | 3 |
| A0A0A6S4M4 | *Streptococcus uberis* | 4 |
| A0A0K1F3T2 | *Olsenella* sp. oral taxon 807 | 5 |
| A0A134AK54 | *Leptotrichia wadei* | 6 |
| A0A134A1S1 | *Senella* sp. DNF00959 | 7 |
| A0A0E2UQR7 | *Streptococcus parauberis* | 8 |
| A0A173RRH3 | *Anaerostipes hadrus* | 9 |
| A0A1E8VV95 | *Senella* sp. HMSC062G07 | 10 |
| A0A1I3HGA4 | *Selenomonas ruminantium* | 11 |
| A0A285HLM7 | *Orenia metallireducens* | 12 |
| A0A2M9H9C8 | *Bifidobacterium primatium* | 13 |
| C9N0T4 | *Leptotrichia hofstadii*F0254 | 14 |
| A0A0R2AF79 | *Ligilactobacillus agilis*DSM 20509 | 15 |
| U2TM27 | *Senella profusa*F0195 | 16 |
| A0A0A6PW80 | *Clostridium butyricum* | 17 |
| A0A1C0A9L3 | *Orenia metallireducens* | 18 |
| A0A0B8PMP8 | *Vibrio ishigakensis* | 19 |
| A0A425WQY7 | *Coriobacteriaceae* | 20 |
| F2N714 | *Coriobacterium glomerans* strain ATCC 49209/DSM 20642/JCM 10262/PW2 | 21 |
| M2NEK5 | *Eggertshia catenaformis*OT 569 | 22 |
| A0A0A7FYZ2 | *Clostridium baratiistr. Sullivan* | 23 |
| A0A143X8V0 | *Clostridiales* bacteria CHKCI006 | 24 |
| A0A2N2BBI5 | *Firmicutes* bacteria HGW-Firmicutes-5 | 25 |
| A0A2T0BJ14 | *Clostridium vincintii* | 26 |
| A0A380JDF6 | *Streptococcus downei* MFe28 | 27 |
| A0A847CI73 | *Erysipelotrichaceae* | 28 |
| A0A8J6Z417 | Quinella sp.3Q1 | 29 |
| D1AFQ7 | *Sebaldella termitidis* strain ATCC 33386/NCTC 11300 | 30 |
| A0A062WZ81 | *Ligilactobacillus animalis* | 31 |
| A0A0R1V613 | *Liquorilactobacillus satsumensis* DSM 16230 | 32 |
| A0A0M0A869 | *Clostridium botulinum* | 33 |
| A0A0V8QAB4 | *Acetivibrio ethanolgignens* | 34 |
| A0A2N3J357 | *Aeromonas sobria* | 35 |
| A0A1I2MD83 | *Clostridium cadaveris* | 36 |
| A0A1L8MKJ9 | *Streptococcus bovimastitidis* | 37 |
| A0A4R1NC63 | *Sodalis ligni* | 38 |
| A0A5R9CBU2 | Lactococcus raffinolactis | 39 |
| C2ELS4 | *Lactobacillus ultunensis* DSM 16047 | 40 |
| A0A3P1W4E8 | *Leptotrichia sp.* OH3620 | 41 |
| A0A498R759 | *Lucifera butyrica* | 42 |
| A0A4V6Q2Z2 | *Fonticella tunisiensis* | 43 |
| A7MME9 | *Cronobacter sakazakii* strain ATCC BAA-894 | 44 |
| A0A0R1Q920 | *Liquorilactobacillus uvarum* DSM 19971 | 45 |
| A0A1Y4F189 | *Anaeromassilibacillus sp.* An250 | 46 |
| A0A498D0T9 | *Anaerotruncus sp.* 22A2-44 | 47 |
| A0A510JEA5 | *Leptotrichia hofstadii* | 48 |
| A0A0A2VZ96 | *Beauveria bassiana* D1-5 | 49 |
| A0A447V187 | *Cedecea lapagei* | 50 |
| B2ISH7 | *Streptococcus pneumoniae* strain CGSP14 | 51 |
| A0A075SG00 | *Streptococcus suis* 6407 | 52 |
| A0A0R2GM47 | *Lactiplantibacillus plantarum* | 53 |
| A0A1Q8F4Y7 | *Aeromonas veronii* | 54 |
| A0A290PWH6 | *Lactococcus raffinolactis* | 55 |
| A0A447Y009 | *Escherichia coli* | 56 |
| A0A0B7GIL0 | *Streptococcus sanguinis* | 57 |
| A0A1L7RNK7 | *Actinomyces succiniciruminis* | 58 |
| A0A376TJI4 | *Bacillus coli* | 59 |
| A0A3R9YDI0 | *Vagococcus humatus* | 60 |
| A0A064C019 | *Streptococcus pneumoniae* | 61 |
| A0A0P7FGJ3 | *Vibrio alginolyticus* | 62 |
| A0A0R2KGV0 | *Ligilactobacillus acidipiscis* | 63 |
| A0A1T4P4Y5 | *Pilibacter termitis* | 64 |
| A0A1E3KU28 | *Lactiplantibacillus plantarum* | 65 |
| A0A349BXL4 | *Lachnospiraceae* | 66 |
| A0A3T0ZV83 | *Aeromonas hydrophila* | 67 |
| A0A1L8WPN6 | *Enterococcus ratti* | 68 |
| A0A0R2H5D7 | *Kandleria vitulina* | 69 |
| A0A0H3J4P2 | *Clostridium pasteurianum* DSM 525 | 70 |
| A0A0T6U3W3 | *Aeromonas allosaccharophila* | 71 |
| K8CC45 | *Enterobacter sakazakii* | 72 |
| A0A1W6B1J9 | *Pantoea alhagi* | 73 |
| B6FW14 | *Peptacetobacter hiranonis* strain DSM 13275/JCM 10541/KCTC 15199/TO-931 | 74 |
| A0A034T1M9 | *Edwardsiella piscicida* | 75 |
| A0A089PXZ6 | *Cedecea neteri* | 76 |
| A0A0A3AQL5 | *Chelonobacter oris* | 77 |
| A0A0D0QVG1 | *Aeromonas sp.* L_1B5_3 | 78 |
| A0A0F4VRP2 | *Clostridium sp.* IBUN125C | 79 |
| A0A1M5EXF5 | *Vibrio gazogenes* DSM 21264 | 80 |
| A0A2Z5Y0I5 | *Melissococcus plutonius* | 81 |
| A0A5C7QZI1 | *Tolumonas sp.* | 82 |
| A0A3D0GQ55 | *Erysipelotrichaceae* | 83 |
| A0A0R1F6B2 | *Loigolactobacillus coryniformissub sp. Coryniformis* KCTC 3167 | 84 |
| A0A4P9VTG8 | *Zooshikella ganghwensis* | 85 |
| A0A829T2C0 | *Vibrio sp.* | 86 |
| A0A2T3N2G3 | *Photobacterium lipolyticum* | 87 |
| A0A8S7XWX0 | *Bacillus coli* | 88 |
| X0PS46 | *Agrilactobacillus composti* DSM 18527 | 89 |
| A0A061Q2Y5 | *Vibrio sp.* JCM 19052 | 90 |
| A0A174H662 | *Clostridium symbiosum* | 91 |
| F3Y874 | *Melissococcus plutonius* strain ATCC 35311/CIP 104052/LMG 20360/NCIMB 702443 | 92 |
| A0A1S1BRH2 | *Aerococcus sp.* HMSC035B07 | 93 |
| A0A1Y4BCL8 | *Senella sp.* An293 | 94 |
| A0A7Z0VH52 | -- | 95 |
| A0A0I9W1Z4 | -- | 96 |

**Table 3. Primers for plasmid construction.**

| **SEQ ID NO:** | **Name** | **Target** | **Sequence (5' to 3')** | **Application** |
|---|---|---|---|---|
| 154 | 1F | genome DNA* | cctgtacttccaatccatgacgaatcgtatctc | Construction of HT-EcPflD |
| 155 | 1R | genome DNA* | cgttatccacttccttacagctgatgcgtctg | Construction of HT-EcPflD |
| 156 | 2F | genome DNA* | cctgtacttccaatccatgacatcatccgcc | Construction of HMT-EcPflC |
| 157 | 2R | genome DNA* | ccgttatccacttccttaacctcccacggtaac | Construction of HMT-EcPflC |
| 158 | 3F | genome DNA* | cctgtacttccaatccatggaactgtatctgg | Construction of HT-EcFsaB |
| 159 | 3R | genome DNA* | ccgttatccacttccaatttagagatgagtagtgcc | Construction of HT-EcFsaB |
| 160 | 4F | genome DNA* | cctgtacttccaatccatggaccgcattattc | Construction of HT-EcGldA |
| 161 | 4R | genome DNA* | ccgttatccacttccaatttattcccactctgc | Construction of HT-EcGldA |
| 162 | 5F | genome DNA* | ccagagcggatcaggaatgaccacactgaa | Construction of pACYC-EcYbiW |
| 163 | 5R | genome DNA* | gagaatctgcccatatgttacagcatatgttc | Construction of pACYC-EcYbiW |
| 164 | 6F | pACYC-EcYbiW | ggctttgcggtgagcgcagcgaacgcacgcgtctgcac atg | Construction of pACYC-EcYbiW(E1 14A, E115A and K117A) |
| 165 | 6R | pACYC-EcYbiW | cgctcaccgcaaagccagcacccgggacg | Construction of pACYC-EcYbiW(E1 14A, E115A and K117A) |

| | | | | |
|---|---|---|---|---|
| Note: * represents the genome of *E coli* MG165. | | | | |

**Table 4. Accession numbers, strain sources, and SEQ ID NOs of 1,5-anhydromannitol-6-phosphate isomerase isozymes**

| **Accession number** | **Strain source** | **SEQ ID NO:** |
|---|---|---|
| P32674 | *Bacillus coli* | 98 |
| A0A7C9H654 | *Firmicutes* | 99 |
| A0A510J954 | *Pseudoleptotrichia goodfellowii* | 100 |
| D4F6M7 | *Edwardsiella tarda* | 101 |
| D4E0C4 | *Serratia odorifera* DSM 4582 | 102 |
| A0A2V4E297 | *Gilliamella apicola* | 103 |
| A0A084T2T3 | *Vibrio* ER1A | 104 |
| A0A0R2FPV6 | *Lactobacillus selangorensis* | 105 |
| A0A0N8NM72 | *Caloranaerobacter sp.* | 106 |
| A0A1T4K0K6 | *Pilibacter termitis* | 107 |
| A0A096BHK0 | *Caloranaerobacter azorensis* H53214 | 108 |
| A0A725B0Z8 | *Salmonella enteritidis* | 109 |
| A0A0B8Q7B9 | *Vibrio* JCM 19236 | 110 |
| A0A037YM28 | *Bacillus coli* | 111 |
| C4LFY8 | *Tolumonas auensis* strain DSM 9187/TA4 | 112 |
| A0A6N7XPT4 | *Senella porci* | 113 |
| A0A4S2F5T8 | *Mycobacteriaceae* | 114 |
| A0A4R7KVA0 | *Fonticella tunisiensis* | 115 |
| A0A437UWZ5 | *Coriobacterialesbacteria* OH1046 | 116 |
| A0A3R9P0U8 | *Bacillus sp*. HMF5848 | 117 |
| A0A380TM32 | *Actinobacillus rossii* | 118 |
| C2ELS8 | *Lactobacillus ultunensis* DSM 16047 | 119 |
| A0A2N2B0Z5 | *Firmicutes bacteria* HGW-Firmicutes-7 | 120 |
| A0A1J0GF99 | *Clostridium estertheticum Esterification subspecies* | 121 |
| A0A1D8GPW2 | *Geosporobacter ferrireducens* | 122 |
| A0A0B8PE23 | *Vibrio ishigakensis* | 123 |
| A0A239SQG5 | *Streptococcus merionis* | 124 |
| A0A823B0Q1 | *Shigella boydii* | 125 |
| X0PU04 | *AgriLactobacillus composti* DSM 18527 | 126 |
| A0A0R2FH98 | *Lactobacillus selangorensis* | 127 |
| A0A430AQ99 | *Vagococcus elongatus* | 128 |
| A0A1W1WZ75 | *Clostridium acidisoli* DSM 12555 | 129 |
| A0A376L3P4 | *Bacillus coli* | 130 |
| A0A1T4WMT8 | *Caloramator quimbayensis* | 131 |
| A0A11H9K1 | *Clostridium uliginosum* | 132 |
| A0A143I1W1 | *Enterobacter asburiae* | 133 |
| N2BYX9 | *Atopobium minutum* | 134 |
| A0A4S2EVE2 | *Coriobacteriaceae* | 135 |
| A0A7S7M803 | *Thermophilibacter immobilis* | 136 |
| A0A2T3N2G1 | *Lactobacillus solubilis* | 137 |
| A0A381GG45 | *Citrobacter amalonaticus* | 138 |
| A0A0A0F8F5 | *Bacillus coli* G3 | 139 |
| A0A0J0GCD9 | *Escherichia albertii* | 140 |
| A0A0H3J2B1 | *Pasteurella pestis bacillus* | 141 |
| D1AJA5 | *Termite Saccharomonospora* strain ATCC 33386/NCTC 11300 | 142 |
| A0A8S0W6S0 | *Acididesulfobacillus acetoxydans* | 143 |
| A0A7W2G1V0 | *clostridium* | 144 |

**Table 5. Primers for construction of E. coli ΔybiW and ΔpflD strains**

| SEQ ID NO: | **name** | **Target** | **Sequence (5' to 3')** | **Application** |
|---|---|---|---|---|
| 166 | 7F | genome DNA† | ccagtagaaacagacgtctaggggcattttcgctgacata | Construction of pRed_Cas9_recA_ΔvbiW |
| 167 | 7R | genome DNA† | ccttacgaggataacaacctctcctcggcccggcgg | Construction of pRed_Cas9_recA_ΔybiW |
| 168 | 8F | genome DNA† | ggtgttatcctcgtaaggtcgcggttaac | Construction of pRed_Cas9_recA_ΔybiW |
| 169 | 8R | genome DNA† | glgagaatccaagcttctagccttlatgcagccggaaatg | Construction of pRed_Cas9_recA_ΔybiW |
| 170 | 9F | pRed_Cas9_recA | ctagaagcttggattctaccaataaaaaacgc | Construction of pRed_Cas9_recA_ΔybiW and pRed_Cas9_recA_ΔpflD |
| 171 | 9R | pRed_Cas9_recA | cccgcagactttctggcaggccgctagatctgactccataac | Construction of pRed_Cas9_recA_ΔybiW |
| 172 | 10F | pRed_Cas9_recA | gcggctgcc gagaaagtctgcgggtttagagctagaaatagc | Construction of pRed_Cas9_recA_ΔybiW |
| 172 | 10F | pRed_Cas9_recA | gcggctgcc gagaaagtctgcgggtttagagctagaaatagc | Construction of pRed_Cas9_recA_ΔybiW |
| 173 | 10R | pRed_Cas9_recA | ctagacgtctgtttctactggtattggcacaaaacctg | Construction of pRed_Cas9_recA_ΔybiW and pRed_Cas9_recA_ΔpflD |
| 174 | 11F | genome DNA† | ccagtagaaacagacgtctagcggagcagtggtgcggctg | Construction of pRed_Cas9_recA_ΔpflD |
| 175 | 11R | genome DNA† | gtccgcgcgatgatgcatgaggaacctcgtgggaagc | Construction of pRed_Cas9_recA_ΔpflD |
| 176 | 12F | genome DNA† | atgacatcatccgccggacagcgcatcagctg | Construction of pRed_Cas9_recA_ΔpflD |
| 177 | 12R | genome DNA† | gagaatccaagcttctagcaatttcgccagcggtaa | Construction of pRed_Cas9_recA_ΔpflD |
| 178 | 13F | pRed_Cas9_recA | gcgaaataccagaacccgcgcgggttttagagctagaaatagc aa | Construction of pRed_Cas9_recA_ΔpflD |
| 179 | 13R | pRed_Cas9_recA | ccgcgcgggttctggtattccgctagatctgactccca | Construction of pRed_Cas9_recA_ΔpflD |
| 180 | 14F | pRed_Cas9_recA_Δy biW and pRed_Cas9_recA_Δp flD | gctgtatgcacaaagcatc | Sequencing |
| 181 | 14R | pRed_Cas9_recA_Δy biW and pRed_Cas9_recA_Δp flD | cgagagctcgctggacctcctg | Sequencing |
| 182 | 15F | pRed_Cas9_recA_Δy biW and pRed_Cas9_recA_Δp flD | gctgctgagcaacccgaactc | Sequencing |
| 183 | 16F | genome DNA# | ccgcgaaataccgtttgccttatgtagacc | Sequencing |
| 184 | 16R | genome DNA# | ggtgtcagaacccggaaagcc | Sequencing |
| 185 | 17F | genome DNA* | gcggcactgtgatgggctgtg | Sequencing |
| 186 | 17R | genome DNA* | gccacatcagctgacgacggcgc | Sequencing |

| | | | | |
|---|---|---|---|---|
| Note: † represents the genome of *E coli* MG1655WT; # represents the genome of ΔybiW; * represents the genome of ΔPflD. | | | | |

**Table 6. Primers for plasmid construction**

| **SEQ ID NO:** | **Name** | **Target** | **Sequence (5' to 3')** | **Application** |
|---|---|---|---|---|
| 189 | 18F | genome DNA* | cctgtacttcccaatccatgaccacactgaaactg | Construction of HT-EcYbiW |
| 190 | 18R | genome DNA* | gatccgttatccacttccttacagcatatgttcag | Construction of HT-EcYbiW |
| 191 | 19F | genome DNA* | cctgtattttcagggcgcccatatgccaacgacatcacaac | Construction of pACYC-MBP-EcYbiY |
| 192 | 19R | genome DNA* | ccaattgagatctgcccatatgttaccacccattcacccg | Construction of pACYC-MBP-EcYbiY |

| | | | | |
|---|---|---|---|---|
| Note: * represents the genome of *E coli* MG165 | | | | |

The exemplary examples of the various embodiments of the present disclosure are described above. However, without departing from the spirit and scope of the present disclosure, those skilled in the art may modify or improve the exemplary examples described herein. Variations or equivalent solutions resulting therefrom also fall within the scope of the present disclosure.

## Claims

1. A polypeptide comprising an amino acid sequence shown in SEQ ID NO: 1 or 97, or a functional variant thereof, wherein the functional variant possesses 1,5-anhydroalditol-6-phosphate isomerase activity.

2. The polypeptide of claim 1, wherein the polypeptide comprises the amino acid sequence shown in SEQ ID NO: 1 or a functional variant thereof, wherein the functional variant possesses 1,5-anhydroglucitol-6-phosphate isomerase activity;
preferably, the polypeptide has an active site in its three-dimensional conformation defined such that, with reference to SEQ ID NO: 1, the active site includes amino acid residues H165, H282, S283, H334, C441, E443, R453, T455, L562, S662, I664, and G786, which are spatially close to one another.

3. The polypeptide of claim 2, wherein the functional variant is a natural isozyme of the amino acid sequence shown in SEQ ID NO: 1;
preferably, the natural isozyme is derived from: *Lactiplantibacillus plantarum*, *Escherichia coli*, *Lactobacillus selangorensis*, *Streptococcus uberis*, *Olsenella sp.*, *Leptotrichia wadei*, *Streptococcus parauberis*, *Anaerostipes hadrus*, *Senella sp.*, Selenomonas ruminantium, *Orenia metallireducens*, *Bifidobacterium primatium*, *Leptotrichia hofstadii*, *Ligilactobacillus agilis*, *Senella profusa*, *Clostridium butyricum*, *Vibrio ishigakensis*, *Coriobacteriaceae*, *Coriobacterium glomerans*, *Eggerthia cateniformis*, *Clostridium baratii str. Sullivan*, *Clostridiales, Firmicutes, Clostridium vincentii, Streptococcus downei, Erysipelotrichaceae, Quinella sp., Sebaldella termitidis, Ligilactobacillus animalis, Liquorilactobacillus satsumensis, Clostridium botulinum, Acetivibrio ethanolgignens, Aeromonas sobria, Clostridium cadaveris, Streptococcus bovimastitidis, Sodalis ligni, Lactococcus raffinolactis, Lactobacillus ultunensis, Leptotrichia sp., Lucifera butyrica, Fonticella tunisiensis, Cronobacter sakazakii, Liquorilactobacillus uvarum, Anaeromassilibacillus sp., Anaerotruncus sp., Beauveria bassiana, Cedecea lapagei, Streptococcus pneumoniae, Streptococcus suis, Aeromonas veronii, Lactococcus raffinolactis, Streptococcus sanguinis, Actinomyces succiniciruminis, Vagococcus humatus, Vibrio alginolyticus, Ligilactobacillus acidipiscis, Pilibacter termitis, Lachnospiraceae, Aeromonas hydrophila, Enterococcus ratti, Kandleria vitulina, Clostridium pasteurianum, Aeromonas allosaccharophila, Pantoea alhagi, Peptacetobacter hiranonis, Edwardsiella piscicida, Cedecea neteri, Chelonobacter oris, Aeromonas sp., Clostridium sp., Vibrio gazogenes, Melissococcus plutonius, Tolumonas sp., Loigolactobacillus coryniformis, Zooshikella ganghwensis, Vibrio sp., Photobacterium lipolyticum, Agrilactobacillus composti, Clostridium symbiosum*, or *Aerococcus sp.*;
more preferably, the natural isozyme comprises an amino acid sequence shown in any one of SEQ ID NOs: 2-96.

4. The polypeptide of claim 1, wherein the polypeptide comprises the amino acid sequence shown in SEQ ID NO: 97 or a functional variant thereof, wherein the functional variant possesses 1,5-anhydromannitol-6-phosphate isomerase activity;
preferably, the polypeptide has an active site in its three-dimensional conformation defined such that, with reference to SEQ ID NO: 97, the active site includes amino acid residues Q162, H169, S277, S278, R323, F331, P335, C431, E433, D445, Y628, V630, and G752, which are spatially close to one another.

5. The polypeptide of claim 4, wherein the functional variant is a natural isozyme of the amino acid sequence shown in SEQ ID NO: 97;
preferably, the natural isozyme is derived from: *Escherichia coli, Streptococcus dysgalactiae subsp. equisimilis, Firmicutes, Pseudoleptotrichia goodfellowii, Edwardsiella tarda, Serratia odorifera, Gilliamella apicola, Vibrio sp., Lactobacillus selangorensis, Caloranaerobacter sp., Pilibacter termitis, Caloranaerobacter azorensis, Salmonella enteritidis, Tolumonas auensis, Senella porci, Coriobacteriaceae, Fonticella tunisiensis, Coriobacteriales, Bacillus sp., Actinobacillus rossii, Lactobacillus ultunensis, Clostridium estertheticum, Geosporobacter ferrireducens, Vibrio ishigakensis, Streptococcus merionis, Shigella boydii, Agrilactobacillus composti, Lactobacillus selangorensis, Vagococcus elongatus, Clostridium acidisoli, Caloramator quimbayensis, Clostridium uliginosum, Enterobacter asburiae, Atopobium minutum, Thermophilobacter immobilis, Photobacterium lipolyticum, Citrobacter amalonaticus, Clostridium pasteurianum, Sebaldella termitidis, Acididesulfobacillus acetoxydans,* or *Clostridium sp.*;
more preferably, the natural isozyme comprises an amino acid sequence shown in any one of SEQ ID NOs: 98-144.

6. The polypeptide of any one of claims 1 to 5, wherein an substrate of the polypeptide is 1,5-anhydroalditol-6-phosphate; preferably, the 1,5-anhydroalditol-6-phosphate is 1,5-anhydroglucitol-6-phosphate or 1,5-anhydromannitol-6-phosphate.

7. The polypeptide of any one of claims 1 to 6, wherein the functional variant is generated by one or more amino acid insertions, substitutions, and/or deletions in the amino acid sequence shown in SEQ ID NO: 1 or 97, or a natural isozyme thereof, optionally, the insertions, substitutions, and/or deletions do not occur in the active site.

8. A nucleic acid molecule encoding the polypeptide of any one of claims 1 to 7.

9. An expression cassette comprising the nucleic acid molecule of claim 8.

10. An expression vector comprising the nucleic acid molecule of claim 8 or the expression cassette of claim 9.

11. A host cell comprising the nucleic acid molecule of claim 8, the expression cassette of claim 9, or the expression vector of claim 10.

12. The host cell of claim 11, wherein the host cell expresses: an activating enzyme, an aldolase, a hydroxyacetone reductase, and/or a transport complex;
preferably, the host cell further expresses an S-adenosylmethionine radical enzyme family glycine radical enzyme activating enzyme, a 1-deoxyfructose-6-phosphate aldolase, a hydroxyacetone reductase, and/or a transport complex for the polypeptide of any one of claims 1 to 7.

13. The host cell of claim 12, wherein the transport complex possesses phosphoenolpyruvate-phosphotransferase system transport activity;
preferably, the transport complex is a phosphoenolpyruvate-phosphotransferase system;
more preferably, the transport complex comprises at least one of SEQ ID NOs: 150-153 or a functional variant thereof.

14. The host cell of any one of claims 11 to 13, wherein the host cell is a eukaryotic cell or a prokaryotic cell;
preferably, the eukaryotic cell is a yeast cell; and/or
preferably, the prokaryotic cell is selected from: *Escherichia spp., Klebsiella spp., Streptococcus spp., Lactobacillus spp., Bifidobacterium spp., Bacteroidetes*, and *Firmicutes*;
more preferably, the *Escherichia spp.* i*s Escherichia coli*.

15. A composition comprising the polypeptide of any one of claims 1 to 7;
preferably, the composition further comprises an activating enzyme, an aldolase, and/or a hydroxyacetone reductase; more preferably, the composition further comprises an S-adenosylmethionine radical enzyme family glycine radical enzyme activating enzyme, a 1-deoxyfructose-6-phosphate aldolase, and/or a hydroxyacetone reductase for the polypeptide of any one of claims 1 to 7.

16. The composition of claim 15, for use in catalyzing a conversion of 1,5-anhydroalditol-6-phosphate to 1,2-propanediol;
preferably, the 1,5-anhydroalditol-6-phosphate is 1,5-anhydroglucitol-6-phosphate or 1,5-anhydromannitol-6-phosphate.

17. Use of the polypeptide of any one of claims 1 to 7, the nucleic acid molecule of claim 8, the expression cassette of claim 9, the expression vector of claim 10, or the host cell of any one of claims 11-14 in the preparation of a composition for catalyzing a conversion of 1,5-anhydroalditol-6-phosphate to 1,2-propanediol;
preferably, the 1,5-anhydroalditol-6-phosphate is 1,5-anhydroglucitol-6-phosphate or 1,5-anhydromannitol-6-phosphate.

18. A method for producing 1,2-propanediol, comprising culturing the host cell of any one of claims 11 to 14 in a medium containing 1,5-anhydroglycitol to obtain 1,2-propanediol;
preferably, the 1,5-anhydroglycitol is 1,5-anhydroglucitol or 1,5-anhydromannitol.

19. A method for producing 1,2-propanediol, comprising contacting the composition of claim 15 or 16 with 1,5-anhydroalditol-6-phosphate to obtain 1,2-propanediol;
preferably, the 1,5-anhydroalditol-6-phosphate is 1,5-anhydroglucitol-6-phosphate or 1,5-anhydromannitol-6-phosphate.
